# EUROPEAN PATENT APPLICATION

(11) **EP 3 859 339 A1**
(43) Date of publication of application: **04.08.2021**
(21) Application number: 19865099.6
(22) Date of filing: 26.09.2019
(51) Int. Cl.: G01N 33/68

(54) **MILD-COGNITIVE-IMPAIRMENT EVALUATION METHOD, CALCULATION METHOD, EVALUATION DEVICE, CALCULATION DEVICE, EVALUATION PROGRAM, CALCULATION PROGRAM, RECORDING MEDIUM, EVALUATION SYSTEM, AND TERMINAL DEVICE**

(30) Priority: 26.09.2018 JP 2018180994
(71) Applicant: AJINOMOTO CO., INC., Chuo-ku Tokyo 104-8315 (JP)
(72) Inventor: IKEUCHI, Takeshi, Niigata-shi, Niigata 950-2181 (JP); YANO, Yuki, Kawasaki-shi, Kanagawa 210-8681 (JP); KONDO, Hiroko, Kawasaki-shi, Kanagawa 210-8681 (JP); SATO, Wataru, Kawasaki-shi, Kanagawa 210-8681 (JP); ARASHIDA, Naoko, Kawasaki-shi, Kanagawa 210-8681 (JP); UENO,Satoko, Kawasaki-shi, Kanagawa 210-8681 (JP); NISHIMOTO, Rumi, Kawasaki-shi, Kanagawa 210-8681 (JP); HARADA, Masashi, Kawasaki-shi, Kanagawa 210-8681 (JP)
(74) Representative: Mewburn Ellis LLP
(86) International application number: PCT/JP2019/038057
(87) International publication number: WO 2020/067386

(57) **Abstract**

Provided is the evaluating method and the like, which can provide reliable information that may be helpful in knowing a state of mild cognitive impairment. In the present embodiment, a state of mild cognitive impairment for a subject to be evaluated is evaluated using at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of the subject.

## Description

### TECHNICAL FIELD

The present invention relates to an evaluating method, a calculating method, an evaluating apparatus, a calculating apparatus, an evaluating program, a calculating program, a recording medium, an evaluating system, and a terminal apparatus for mild cognitive impairment (hereinafter, referred to as MCI).

### BACKGROUND ART

MCI means a condition considered as a previous stage or a borderline case of various types of dementia that shows severer problems in cognitive function than those of a person of the same age or a normally aging person, but does not affect daily living, being not diagnosed as dementia. For diagnosis of MCI, at present, the major two diagnostic criteria have been offered and widely accepted (Nonpatent Literatures 1 and 2). Background diseases for MCI include various diseases, in addition to Alzheimer's Disease (AD), such as Lewy body dementia (DLB), frontotemporal lobar degeneration (FTLD), vascular dementia (VaD), and depression in the elderly. In some of the primary causes, early intervention may reduce the risk of developing dementia. It is therefore desired to provide simple screening methods that can distinguish MCI from cognitively healthy people to increase the chance for more subjects to have a medical checkup in earlier stages at specialized institutions, to increase the chance for more subjects to get a treatment opportunity in earlier stages, and to contribute to avoiding increase in the number of patients of dementia.

Unfortunately, when the neuropsychological test called "mini mental state examination (MMSE)", the revised Hasegawa dementia scale "HDS-R", and the Alzheimer's disease assessment scale-cognitive (ADAS-cog) that are spread as screening test methods for dementia are applied to screening for MCI, the accuracy of detecting MCI is decreased relative to the accuracy of detecting dementia. Moreover, the neuropsychological test takes a long time for examination and thus is low throughput and involves a skilled examiner. It is desired to provide a new and simple test technique as an MCI screening test method to support a conventional neuropsychological test.

As a technique for determining MCI based on a blood test, a technique for measuring the blood concentration of peptide fragments and determining MCI using the measured blood concentration as an index is known (Nonpatent Literature 3). AD determination and MCI determination techniques by quantitatively analyzing amino acids and amino acid related metabolites in blood are also known (Patent Document 1).

### PRIOR ART REFERENCES

### PATENT DOCUMENT

Patent Document 1: WO 2018/008764

### NON-PATENT LITERATURE

Nonpatent Literature 1: Petersen, et al., Arch Neurol (1999) 56(6):760.; Mild cognitive impairment: clinical characterization and outcome.
Nonpatent Literature 2: Winblad, et al., J. Intern. Med. (2004) 256(3): 240-6.; Mild cognitive impairment--beyond controversies, towards a consensus: report of the International Working Group on Mild Cognitive Impairment.
Nonpatent Literature 3: Nakamura, et al., Nature. (2018) 554 (7691): 249-254.; High performance plasma amyloid-β biomarkers for Alzheimer's disease.

### DISCLOSURE OF INVENTION

### PROBLEM TO BE SOLVED BY THE INVENTION

However, there is a problem in that techniques for accurately determining MCI based on the blood concentration of metabolites including amino acids and amino acid related metabolites as indices need to be further improved in accuracy to satisfy the standards clinically required when the techniques are put into practical use.

The present invention has been made in view of the above descriptions, and an object of the present invention is to provide an evaluating method, a calculating method, an evaluating apparatus, a calculating apparatus, an evaluating program, a calculating program, a recording medium, an evaluating system, and a terminal apparatus, which can provide reliable information that may be helpful in knowing a state of MCI.

### MEANS FOR SOLVING PROBLEM

To solve the problem and achieve the object, an evaluating method according to one aspect of the present invention includes an evaluating step of evaluating a state of MCI for a subject to be evaluated, using (i) at least one value of concentration values of 22 kinds of amino acids (a-ABA, Ala, Arg, Asn, Cit, Glu, Gln, Gly, His, Ile, Leu, Lys, Met, Orn, Phe, Pro, Ser, Thr, Trp, Tyr, Val, and Taurine) and 30 kinds of amino acid related metabolites (1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Put, Sar, SDMA, Spd, Spm, and Thioproline) and test values of 7 kinds of blood biochemical test items (Alb, Folate, WBC, RBC, Hb, Ht, and PLT) in blood of the subject, or (ii) a value of a formula including an explanatory variable to be substituted with the at least one value, calculated using the at least one value and the formula.

In the present description, various amino acids , various amino acid related metabolites, and various blood biochemical test items are mainly written in abbreviations, the formal names of these are as follows.

| (Abbreviation) | (Formal name) |
|---|---|
| α-ABA | α-Aminobutyric acid |
| Ala | Alanine |
| Arg | Arginine |
| Asn | Asparagine |
| Cit | Citrulline |
| Gln | Glutamine |
| Glu | Glutamic acid |
| Gly | Glycine |
| His | Histidine |
| Ile | Isoleucine |
| Leu | Leucine |
| Lys | Lysine |
| Met | Methionine |
| Orn | Ornithine |
| Phe | Phenylalanine |
| Pro | Proline |
| Ser | Serine |
| Taurine | Taurine |
| Thr | Threonine |
| Trp | Tryptophan |
| Tyr | Tyrosine |
| Val | Valine |
| BCAA | Total value of Valine, Leucine, and Isoleucine |
| EAA | Total value of His, Ile, Leu, Lys, Met, Phe, Thr, Tyr, and Val |
| 1-MeHis | N (pi) -Methyl-L-histidine |
| 3-MeHis | N (tau) -Methyl-L-histidine |
| aAAA | Aminoadipic acid |
| aAiBA | 2-Aminoisobutyric acid |
| ADMA | Asymmetric dimethylarginine |
| Allyl Cys | Allyl cysteine |
| bABA | 3-Aminobutanoic acid |
| bAiBA | L-3-Aminoisobutyric acid |
| Cystathionine | L-Cystathionine |
| Cysteic acid | Cysteic acid |
| EtGly | Ethylglycine |
| GABA | 4-Aminobutyric Acid |
| hArg | L-Homoarginine |
| Hypotaurine | Hypotaurine |
| Hypro | 4-Hydroxy-L-proline |
| Kyn | L-Kynurenine |
| Kyn/Trp | L-Kynurenine/Tryptophan |
| Kyn/BCAA | L-Kynurenine/BCAA |
| Pyrazole-1-Ala | 2-amino-3-(1H-pyrazol-1-yl)propanoic acid |
| MCSO | S-Methyl-L-cysteine sulfoxide |
| MCSO1 | S-Methyl-L-cysteine sulfoxide 1st peak |
| MCSO2 | S-Methyl-L-cysteine sulfoxide 2nd peak |
| MeCys | Methyl cysteine |
| MCSO2/MeCys | S-Methyl-L-cysteine sulfoxide 2nd peak/Methyl cysteine |
| N6-AcLys | Ne-Acetyl-L-lysine |
| N8-AcSpd | N8-Acetylspermidine |
| Opthalmic acid | Opthalmic acid |
| PEA | Phosphoethanolamine |
| Pipecolic acid | Pipecolic acid |
| Put | Putrescine |
| Sar | Sarcosine |
| SDMA | Symmetric dimethylarginine |
| Spd | Spermidine |
| Spm | Spermine |
| Thioproline | Thioproline |
| Alb | Albumin |
| Folate | Folate |
| WBC | White blood cell counts |
| RBC | Red blood cell counts |
| Hb | Hemoglobin |
| Ht | Hematocrit |
| PLT | Platelet counts |

In the evaluating method according to one aspect of the present invention, the evaluating step is executed by a control unit of an information processing apparatus.

A calculating method according to one aspect of the present invention includes a calculating step of using (i) at least one value of concentration values of the 22 kinds of amino acids and the 30 kinds of amino acid related metabolites and test values of the 7 kinds of blood biochemical test items in blood of a subject to be evaluated, and (ii) a formula for evaluating a state of MCI, including an explanatory variable to be substituted with the at least one value, to calculate a value of the formula.

In the calculating method according to one aspect of the present invention, the calculating step is executed by a control unit of an information processing apparatus.

An evaluating apparatus according to one aspect of the present invention is an evaluating apparatus including a control unit. The control unit includes an evaluating unit that evaluates a state of MCI for a subject to be evaluated, using (i) at least one value of concentration values of the 22 kinds of amino acids and the 30 kinds of amino acid related metabolites and test values of the 7 kinds of blood biochemical test items in blood of the subject, or (ii) a value of a formula including an explanatory variable to be substituted with the at least one value, calculated using the at least one value and the formula.

The evaluating apparatus according to one aspect of the present invention is communicatively connected via a network to a terminal apparatus that provides blood data on the at least one value, or the value of the formula. The control unit further includes: a data-receiving unit that receives the blood data of the subject or the value of the formula transmitted from the terminal apparatus; and a result-sending unit that transmits an evaluation result obtained by the evaluating unit to the terminal apparatus. The evaluating unit uses the at least one value included in the blood data or the value of the formula received by the data-receiving unit.

A calculating apparatus according to one aspect of the present invention is a calculating apparatus including a control unit. The control unit includes a calculating unit that uses (i) at least one value of concentration values of the 22 kinds of amino acids and the 30 kinds of amino acid related metabolites and test values of the 7 kinds of blood biochemical test items in blood of a subject to be evaluated, and (ii) a formula for evaluating a state of MCI, including an explanatory variable to be substituted with the at least one value, to calculate a value of the formula.

An evaluating program according to one aspect of the present invention is an evaluating program for causing a control unit of an information processing apparatus to execute: an evaluating step of evaluating a state of MCI for a subject to be evaluated, using (i) at least one value of concentration values of the 22 kinds of amino acids and the 30 kinds of amino acid related metabolites and test values of the 7 kinds of blood biochemical test items in blood of the subject, or (ii) a value of a formula including an explanatory variable to be substituted with the at least one value, calculated using the at least one value and the formula.

A calculating program according to one aspect of the present invention is a calculating program for causing a control unit of an information processing apparatus to execute: a calculating step of using (i) at least one value of concentration values of the 22 kinds of amino acids and the 30 kinds of amino acid related metabolites and test values of the 7 kinds of blood biochemical test items in blood of a subject to be evaluated, and (ii) a formula for evaluating a state of MCI, including an explanatory variable to be substituted with the at least one value, to calculate a value of the formula.

A recording medium according to one aspect of the present invention is a computer readable recording medium storing the evaluating program or the calculating program. Specifically, the recording medium according to one aspect of the present disclosure is a non-transitory computer readable recording medium including programmed instructions for causing an information processing apparatus to execute the evaluating method or the calculating method.

An evaluating system according to one aspect of the present invention is an evaluating system including: an evaluating apparatus including a control unit; and a terminal apparatus including a control unit to provide (i) blood data on at least one value of concentration values of the 22 kinds of amino acids and the 30 kinds of amino acid related metabolites and test values of the 7 kinds of blood biochemical test items in blood of a subject to be evaluated, or (ii) a value of a formula including an explanatory variable to be substituted with the at least one value, calculated using the at least one value and the formula. The evaluating apparatus and the terminal apparatus are communicatively connected to each other via a network. The control unit of the terminal apparatus includes: a data-sending unit that transmits the blood data of the subject, or the value of the formula, to the evaluating apparatus; and a result-receiving unit that receives an evaluation result on a state of MCI for the subject, transmitted from the evaluating apparatus. The control unit of the evaluating apparatus includes: a data-receiving unit that receives the blood data of the subject or the value of the formula transmitted from the terminal apparatus; an evaluating unit that evaluates a state of MCI for the subject, using the at least one value included in the blood data of the subject or the value of the formula received by the data-receiving unit; and a result-sending unit that transmits the evaluation result obtained by the evaluating unit to the terminal apparatus.

A terminal apparatus according to one aspect of the present invention is a terminal apparatus including a control unit. The control unit includes a result-obtaining unit that obtains an evaluation result on a state of MCI for a subject to be evaluated. The evaluation result is a result of evaluating a state of MCI for the subject, using (i) at least one value of concentration values of the 22 kinds of amino acids and the 30 kinds of amino acid related metabolites and test values of the 7 kinds of blood biochemical test items in blood of the subject, or (ii) a value of a formula including an explanatory variable to be substituted with the at least one value, calculated using the at least one value and the formula.

The terminal apparatus according to one aspect of the present invention is communicatively connected via a network to an evaluating apparatus that evaluates a state of MCI for the subject. The control unit includes a data-sending unit that transmits blood data on at least one value of concentration values of the 22 kinds of amino acids and the 30 kinds of amino acid related metabolites and test values of the 7 kinds of blood biochemical test items in blood of the subject, or the value of the formula, to the evaluating apparatus. The result-obtaining unit receives the evaluation result transmitted from the evaluating apparatus.

### EFFECT OF THE INVENTION

According to the present invention, reliable information that may be helpful in knowing a state of MCI can be provided.

### BRIEF DESCRIPTION OF DRAWINGS

FIG. 1 is a principle configurational diagram showing a basic principle of a first embodiment;
FIG. 2 is a principle configurational diagram showing a basic principle of a second embodiment;
FIG. 3 is a diagram showing an example of an entire configuration of a present system;
FIG. 4 is a diagram showing another example of an entire configuration of the present system;
FIG. 5 is a block diagram showing an example of a configuration of an evaluating apparatus 100 in the present system;
FIG. 6 is a chart showing an example of information stored in a blood data file 106a;
FIG. 7 is a chart showing an example of information stored in an index state information file 106b;
FIG. 8 is a chart showing an example of information stored in a designated index state information file 106c;
FIG. 9 is a chart showing an example of information stored in a formula file 106d1;
FIG. 10 is a chart showing an example of information stored in an evaluation result file 106e;
FIG. 11 is a block diagram showing a configuration of an evaluating part 102d;
FIG. 12 is a block diagram showing an example of a configuration of a client apparatus 200 in the present system;
FIG. 13 is a block diagram showing an example of a configuration of a database apparatus 400 in the present system;
FIG. 14 is a diagram of a chromatogram obtained by a liquid chromatograph mass spectrometer;
FIG. 15 is a diagram of a chromatogram obtained by the liquid chromatograph mass spectrometer;
FIG. 16 is a table of explanatory variables included in a logistic regression expression and ROC_AUC;
FIG. 17 is a table of explanatory variables included in a logistic regression expression and ROC_AUC;
FIG. 18 is a table of the frequencies of occurrence of explanatory variables in a logistic regression expression;
FIG. 19 is a table of the frequencies of occurrence of combinations of two kinds of explanatory variables in the logistic regression expression;
FIG. 20 is a table of explanatory variables included in a logistic regression expression and ROC_AUC;
FIG. 21 is a table of explanatory variables included in a logistic regression expression and ROC_AUC;
FIG. 22 is a table of the frequencies of occurrence of explanatory variables in a logistic regression expression;
FIG. 23 is a table of the frequencies of occurrence of combinations of two kinds of explanatory variables in the logistic regression expression;
FIG. 24 is a table of explanatory variables included in a logistic regression expression and ROC_AUC;
FIG. 25 is a table of explanatory variables included in a logistic regression expression and ROC_AUC;
FIG. 26 is a table of the frequencies of occurrence of explanatory variables in a logistic regression expression;
FIG. 27 is a table of the frequencies of occurrence of combinations of two kinds of explanatory variables in the logistic regression expression;
FIG. 28 is a table of explanatory variables included in a logistic regression expression and ROC_AUC;
FIG. 29 is a table of explanatory variables included in a logistic regression expression and ROC_AUC;
FIG. 30 is a table of explanatory variables included in a logistic regression expression and ROC_AUC;
FIG. 31 is a table of explanatory variables included in a logistic regression expression and ROC_AUC;
FIG. 32 is a table of explanatory variables included in a logistic regression expression and ROC_AUC;
FIG. 33 is a table of explanatory variables included in a logistic regression expression and ROC_AUC;
FIG. 34 is a table of explanatory variables included in a logistic regression expression and ROC_AUC;
FIG. 35 is a table of explanatory variables included in a logistic regression expression and ROC_AUC;
FIG. 36 is a table of explanatory variables included in a logistic regression expression and ROC_AUC; and
FIG. 37 is a table of explanatory variables included in a logistic regression expression and ROC_AUC.

### BEST MODE(S) FOR CARRYING OUT THE INVENTION

Hereinafter, an embodiment (first embodiment) of the evaluating method and the calculating method according to the present invention and an embodiment (second embodiment) of the evaluating apparatus, the calculating apparatus, the evaluating method, the calculating method, the evaluating program, the calculating program, the recording medium, the evaluating system, and the terminal apparatus according to the present invention are described in detail with reference to the drawings. The present invention is not limited to these embodiments.

### First Embodiment

### 1-1. Outline of first embodiment

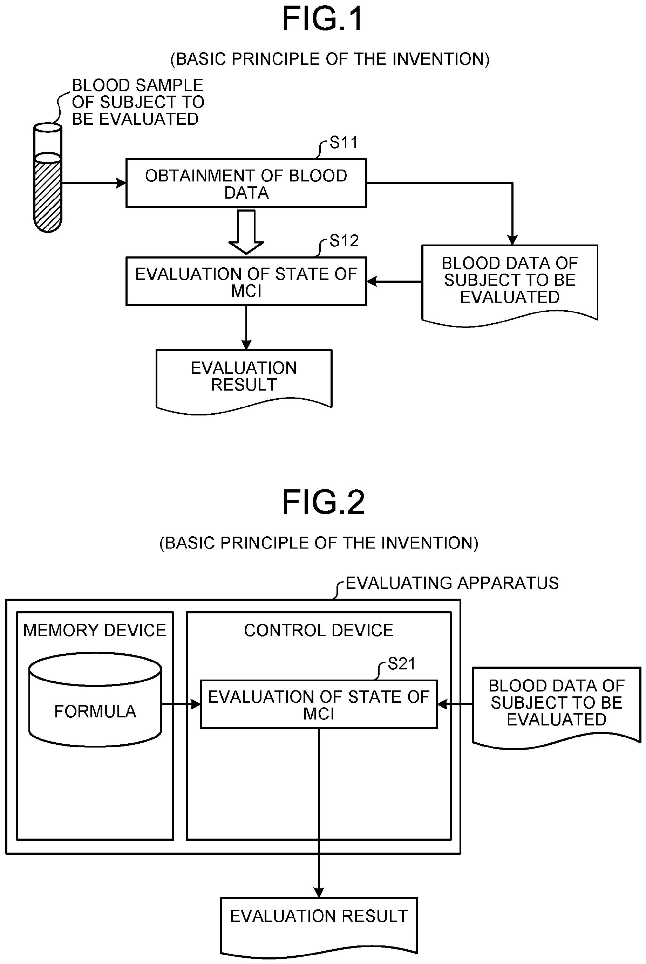

Here, an outline of the first embodiment will be described with reference to FIG. 1. FIG. 1 is a principle configurational diagram showing a basic principle of the first embodiment.

First, blood data on at least one value of concentration values of the 22 kinds of amino acids and the 30 kinds of amino acid related metabolites and test values of the 7 kinds of blood biochemical test items in the blood (including, for example, plasma, serum, or whole blood) extracted from a subject to be evaluated (for example, an individual such as animal or human) is obtained (Step S11).

At Step S11, for example, the blood data measured by a clinical labolatory or the like that measures concentrations or test values may be obtained. For example, the following measuring method of (A), (B), or (C) may be used to measure the concentration from the blood extracted from the subject to obtain the blood data. In the present description, the unit of the concentration may be, for example, molar concentration, weight concentration, enzyme activity, or one obtained by addition, subtraction, multiplication, and division of any constant with these concentrations. For example, a measuring method such as the modified bromocresol purple (BCP) method, chemiluminescent enzyme immuno assay (CLEIA), flow cytometry using semiconductor laser, the sheath flow DC detection method, the SLS-hemoglobin detection method, or the RBC pulse height detection method may be used to measure the test value from the blood extracted from the subject to obtain the blood data.
(A) Plasma is separated from blood by centrifuging the collected blood sample. All plasma samples are frozen and stored at -80°C until the concentration is measured. At the time of measuring the concentration, after acetonitrile is added to deproteinize the plasma samples, impurities such as phospholipid are removed by solid phase extraction as needed, pre-column derivatization is then performed using a labeling reagent (3-aminopyridyl-N-hydroxysuccinimidyl carbamate), and the concentration is analyzed by liquid chromatograph mass spectrometry (including tandem mass spectrometry) (see International Publication WO 2003/069328 and International Publication WO 2005/116629) .
(B) Plasma is separated from blood by centrifuging the collected blood sample. All plasma samples are frozen and stored at -80°C until the concentration is measured. At the time of measuring the concentration, sulfosalicylic acid is added to deproteinize the plasma samples, and the concentration is analyzed by an amino acid analyzer based on post-column derivatization using a ninhydrin reagent.
(C) Blood cell separation is performed on the collected blood sample by using a membrane, micro-electro-mechanical System (MEMS) technology, or the principle of centrifugation, whereby plasma or serum is separated from the blood. A plasma or serum sample the concentration of which is not measured immediately after obtaining the plasma or the serum is frozen and stored at -80°C until the concentration is measured. At the time of measuring the concentration, a molecule that reacts with or binds to a target substance in blood, such as an enzyme or an aptamer, is used to perform quantitative analysis and the like on an increasing or decreasing substance or a spectroscopic value by substrate recognition, whereby the concentration is analyzed.

The state of MCI for the subject is evaluated using the at least one value included in the blood data obtained at Step S11 (Step S12). Before Step S12 is executed, data such as defective and outliers may be removed from the blood data obtained at Step S11. In the present description, evaluation of a state of MCI is, for example, to examine the present condition of MCI.

According to the first embodiment described above, the blood data of the subject is obtained at Step S11, and at Step S12, the state of MCI for the subject is evaluated using the at least one value included in the blood data of the subject obtained at Step S11 (in other words, information for evaluating the state of MCI for the subject or reliable information that may be helpful in knowing the state of MCI for the subject is obtained). Thus, information for evaluating the state of MCI for the subject or reliable information that may be helpful in knowing the state of MCI for the subject can be provided.

It may be decided that at least one value of concentration values of the 22 kinds of amino acids and the 30 kinds of amino acid related metabolites and test values of the 7 kinds of blood biochemical test items reflects the state of MCI for the subject. The concentration value or the test value may be converted, for example, by the methods listed below, and it may be decided that the converted value reflects the state of MCI for the subject. In other words, the concentration value or the test value, or the converted value may be treated per se as an evaluation result on the state of MCI for the subject.

The concentration value may be converted such that a possible range of the concentration value falls within a predetermined range (for example, the range from 0.0 to 1.0, the range from 0.0 to 10.0, the range from 0.0 to 100.0, or the range from -10.0 to 10.0), for example, by addition, subtraction, multiplication, and division of any given value with the concentration value, by conversion of the concentration value by a predetermined conversion method (for example, exponential transformation, logarithm transformation, angular transformation, square root transformation, probit transformation, reciprocal transformation, Box-Cox transformation, or power transformation), or by performing a combination of these computations on the concentration value (the same applies to the test value). For example, a value of an exponential function with the concentration value as an exponent and Napier constant as the base may be further calculated (specifically, a value of p/(1-p) where a natural logarithm ln(p/(1-p)) is equal to the concentration value when the probability p that the state of MCI has a predetermined state (for example, a state of being MCI with a high probability exceeding a criteria) is defined), and a value (specifically, a value of the probability p) may be further calculated by dividing the calculated value of the exponential function by the sum of 1 and the value of the exponential function (the same applies to the test value).

The concentration value may be converted such that the converted value is a particular value when a particular condition is met. For example, the concentration value may be converted such that the converted value is 5.0 when the specificity is 80% and the converted value is 8.0 when the specificity is 95% (the same applies to the test value).

For each amino acid and each amino acid related metabolite, after normally distributing the concentration distribution, the concentration value may be standardized with a mean of 50 and a standard deviation of 10 (the same applies to the test value).

These conversions may be performed by gender or age (the same applies to the test value).

The concentration value in the present description may be the concentration value itself or may be the converted value of the concentration value (the same applies to the test value).

Positional information about a position of a predetermined mark on a predetermined scale visually presented on a display device such as a monitor or a physical medium such as paper may be generated using at least one value of concentration values of the 22 kinds of amino acids and the 30 kinds of amino acid related metabolites and test values of the 7 kinds of blood biochemical test items or, if the at least one value is converted, the converted value, and it may be decided that the generated positional information reflects the state of MCI for the subject. The predetermined scale is for evaluating the state of MCI and is, for example, a graduated scale at least marked with graduations corresponding to the upper limit value and the lower limit value in "a possible range of the concentration value or the test value, or the converted value", or "part of the range". The predetermined mark corresponds to the concentration value or the test value, or the converted value and is, for example, a circle sign or a star sign.

If at least one value of concentration values of the 22 kinds of amino acids and the 30 kinds of amino acid related metabolites and test values of the 7 kinds of blood biochemical test items is lower than a predetermined value (e.g., mean ±1SD, 2SD, 3SD, N quantile, N percentile, or a cutoff value the clinical significance of which is recognized) or is equal to or lower than the predetermined value, or the at least one value is equal to or higher than the predetermined value or is higher than the predetermined value, the state of MCI for the subject may be evaluated. In this case, instead of the concentration value itself, a concentration standard score (a value obtained by normally distributing the concentration distribution by gender and then standardizing the concentration value with a mean of 50 and a standard deviation of 10 for each amino acid and each amino acid related metabolite) may be used (the same applies to the test value). For example, if the concentration standard score is lower than the mean -2SD (when the concentration standard score<30) or if the concentration standard score is higher than the mean +2SD (when the concentration standard score>70), the state of MCI for the subject may be evaluated (the same applies to the test value).

The state of MCI for the subject may be evaluated by using at least one value of concentration values of the 22 kinds of amino acids and the 30 kinds of amino acid related metabolites and test values of the 7 kinds of blood biochemical test items and a formula including an explanatory variable to be substituted with the at least one value, to calculate a value of the formula.

It may be decided that the calculated value of the formula reflects the state of MCI for the subject. The value of the formula may be converted, for example, by the methods listed below, and it may be decided that the converted value reflects the state of MCI for the subject. In other words, the value of the formula, or the converted value may be treated per se as an evaluation result on the state of MCI for the subject.

The value of the formula may be converted such that a possible range of the value of the formula falls within a predetermined range (for example, the range from 0.0 to 1.0, the range from 0.0 to 10.0, the range from 0.0 to 100.0, or the range from -10.0 to 10.0), for example, by addition, subtraction, multiplication, and division of any given value with the value of the formula, by conversion of the value of the formula by a predetermined conversion method (for example, exponential transformation, logarithm transformation, angular transformation, square root transformation, probit transformation, reciprocal transformation, Box-Cox transformation, or power transformation), or by performing a combination of these computations on the value of the formula. For example, a value of an exponential function with the value of the formula as an exponent and Napier constant as the base may be further calculated (specifically, a value of p/(1-p) where a natural logarithm ln(p/(1-p)) is equal to the value of the formula when the probability p that the state of MCI has a predetermined state (for example, a state of being MCI with a high probability exceeding a criteria) is defined), and a value (specifically, a value of the probability p) may be further calculated by dividing the value of the exponential function by the sum of 1 and the value of the exponential function.

The value of the formula may be converted such that the converted value is a particular value when a particular condition is met. For example, the value of the formula may be converted such that the converted value is 5.0 when the specificity is 80% and the converted value is 8.0 when the specificity is 95%.

The value of the formula may be standardized with a mean of 50 and a standard deviation of 10.

These conversions may be performed by gender or age.

The value of the formula in the present description may be the value of the formula itself or may be the converted value of the value of the formula.

Positional information about a position of a predetermined mark on a predetermined scale visually presented on a display device such as a monitor or a physical medium such as paper may be generated using the value of the formula or, if the value of the formula is converted, the converted value, and it may be decided that the generated positional information reflects the state of MCI for the subject. The predetermined scale is for evaluating the state of MCI and is, for example, a graduated scale at least marked with graduations corresponding to the upper limit value and the lower limit value in "a possible range of the value of the formula, or the converted value", or "part of the range". The predetermined mark corresponds to the value of the formula or the converted value and is, for example, a circle sign or a star sign.

The degree of the possibility that the subject has MCI may be qualitatively evaluated. Specifically, the subject may be classified into any one of a plurality of categories defined at least considering the degree of the possibility of having MCI, using "at least one value of concentration values of the 22 kinds of amino acids and the 30 kinds of amino acid related metabolites and test values of the 7 kinds of blood biochemical test items, and one or more preset thresholds", or "at least one value of concentration values of the 22 kinds of amino acids and the 30 kinds of amino acid related metabolites and test values of the 7 kinds of blood biochemical test items, a formula including an explanatory variable to be substituted with the at least one value, and one or more preset thresholds". The categories may include (i) a category to which a subject with a high degree of the possibility of having MCI (for example, a subject assumed to have MCI) belongs, (ii) a category to which a subject with a low degree of the possibility of having MCI (for example, a subject assumed not to have MCI) belongs, and (iii) a category to which a subject with an intermediate degree of the possibility of having MCI belongs. The categories may include (i) the category to which a subject with a high degree of the possibility of having MCI belongs, and (ii) the category to which a subject with a low degree of the possibility of having MCI belongs. The concentration value or the test value, or the value of the formula may be converted by the predetermined method, and the subject may be classified into any one of the categories using the converted value.

As for the formula used for the evaluation, the form of the formula is not specifically designated, however, for example, may be the following forms.
- linear model such as multiple regression equation, linear discriminant, principal component analysis, and canonical discriminant analysis that are based on the least-squares method;
- generalized linear model such as logistic regression and Cox regression that are based on the maximum likelihood method;
- generalized linear mixed model considering random effects due to individual differences, facility differences, and other factors in addition to the generalized linear model
- expression generated by cluster analysis such as the K-means method and hierarchical cluster analysis;
- expression generated on the basis of the Bayesian statistics such as the Markov chain Monte Carlo (MCMC), the Bayesian network, and the hierarchical Bayesian method;
- expression generated by class classification such as support vector machine and decision tree;
- expression generated by a method that does not belong to the above-cited categories such as fractional expression; and
- expression represented as, for example, the summation of expressions of different forms

The formula used for the evaluation may be prepared by a method described in WO 2004/052191 that is an international application filed by the present applicant or by a method described in WO 2006/098192 that is an international application filed by the present applicant. Any formulae obtained by these methods can be preferably used in the evaluation of the state of MCI, regardless of the units of concentrations of amino acids, concentrations of amino acid related metabolites, or test values of blood biochemical test items in the blood data as input data.

In the multiple regression equation, the multiple logistic regression equation, and the canonical discriminant function, a coefficient and a constant term are added to each explanatory variable, and the coefficient and the constant term may be preferably real numbers, more preferably values in the range of 99% confidence interval for the coefficient and the constant term obtained from data for the various kinds of classifications described above, more preferably values in the range of 95% confidence interval for the coefficient and the constant term obtained from data for the various kinds of classifications described above. The value of each coefficient and the confidence interval thereof may be those multiplied by a real number, and the value of the constant term and the confidence interval thereof may be those having an arbitrary actual constant added or subtracted or those multiplied or divided by an arbitrary actual constant. When an expression such as the logistic regression, the linear discriminant, and the multiple regression equation is used for the evaluation, a linear transformation of the expression (addition of a constant and multiplication by a constant) and a monotonic increasing (decreasing) transformation (for example, a logit transformation) of the expression do not alter evaluation performance and thus evaluation performance after transformation is equivalent to that before transformation. Therefore, the expression includes an expression that is subjected to the linear transformation and the monotonic increasing (decreasing) transformation.

In the fractional expression, the numerator of the fractional expression is expressed by the sum of the explanatory variables A, B, C etc. and the denominator of the fractional expression is expressed by the sum of the explanatory variables a, b, c etc. The fractional expression also includes the sum of the fractional expressions α, β, γ etc. (for example, α+β) having such constitution. The fractional expression also includes divided fractional expressions. The explanatory variables used in the numerator or denominator may have suitable coefficients respectively. The explanatory variables used in the numerator or denominator may appear repeatedly. Each fractional expression may have a suitable coefficient. A value of a coefficient for each explanatory variable and a value for a constant term may be any real numbers. In a fractional expression and the one in which explanatory variables in the numerator and explanatory variables in the denominator in the fractional expression are switched with each other, the positive and negative signs are generally reversed in correlation with objective explanatory variables, but because their correlation is maintained, the evaluation performance can be assumed to be equivalent.
The fractional expression therefore also includes the one in which explanatory variables in the numerator and explanatory variables in the denominator in the fractional expression are switched with each other.

When the state of MCI is evaluated, a value related to other biological information (for example, values listed below) may further be used in addition to at least one value of concentration values of the 22 kinds of amino acids and the 30 kinds of amino acid related metabolites and test values of the 7 kinds of blood biochemical test items. The formula used for the evaluation may additionally include one or more explanatory variables to be substituted with a value related to the other biological information (for example, values listed below) in addition to the explanatory variable to be substituted with at least one value of concentration values of the 22 kinds of amino acids and the 30 kinds of amino acid related metabolites and test values of the 7 kinds of blood biochemical test items.
1. Concentration values of metabolites in blood other than amino acids and amino acid related metabolites (e.g., carbohydrates and lipids), proteins, peptides, minerals, hormones, or the like.
2. Blood test values such as total protein, triglyceride (neutral fat), HbA1c, glycoalbumin, insulin resistance index, total cholesterol, LDL cholesterol, HDL cholesterol, amylase, total bilirubin, creatinine, estimated glomerular filtration rate (eGFR), uric acid, GOT (AST), GPT (ALT), GGTP (γ-GTP), glucose (glucose level), CRP (C-reactive protein), MCV, MCH, or MCHC.
3. Values obtained from image information such as ultrasonic echo, X ray, CT (Computed Tomography), MRI (Magnetic Resonance Imaging), or endoscope image.
4. Values of biological indices such as age, height, weight, BMI, abdominal girth, systolic blood pressure, diastolic blood pressure, gender, smoking information, dietary information, drinking information, exercise information, stress information, sleeping information, family medical history information, or disease history information (for example, diabetes).
5. Values obtained from gene information, for example, number of allele of risk genes for Alzheimer's disease (for example, APOPEε4)

### Second Embodiment

### 2-1. Outline of the second embodiment

Here, outlines of the second embodiment will be described in detail with reference to FIG. 2. FIG. 2 is a principle configurational diagram showing a basic principle of the second embodiment. In the description of the present second embodiment, description duplicating that of the first embodiment is sometimes omitted. In particular, herein, when a state of MCI is evaluated, a case of using a value of the formula or the converted value thereof is described as one example. However, for example, at least one value of concentration values of the 22 kinds of amino acids and the 30 kinds of amino acid related metabolites and test values of the 7 kinds of blood biochemical test items or the converted value thereof (for example, a concentration standard score) may be used.

A control device evaluates the state of MCI for the subject by using (i) at least one value of concentration values of the 22 kinds of amino acids and the 30 kinds of amino acid related metabolites and test values of the 7 kinds of blood biochemical test items in blood included in the previously obtained blood data of the subject (for example, an individual such as animal or human) on the at least one value and (ii) a formula previously stored in a memory device, including an explanatory variable to be substituted with the at least one value, to calculate a value of the formula (step S21). Thus, reliable information that may be helpful in knowing the state of MCI can be provided.

The formula used at step S21 may be generated based on the formula-preparing processing (step 1 to step 4) described below. Here, the summary of the formula-preparing processing is described. The processing described below is merely one example, and the method of preparing the formula is not limited thereto.

First, the control device prepares a candidate formula (e.g., y=a₁x₁+a₂x₂+...+aₙxₙ, y: index data, xᵢ: blood data, aᵢ: constant, i=1,2,...,n) based on a predetermined formula-preparing method from index state information (data such as defective and outliers may be removed from the index state information in advance) previously stored in the memory device, containing the blood data and index data on an index representing a state of MCI (step 1).

In step 1, a plurality of the candidate formulae may be prepared from the index state information by using a plurality of the different formula-preparing methods (including those for multivariate analysis such as the principal component analysis, the discriminant analysis, the support vector machine, the multiple regression analysis, the Cox regression analysis, the logistic regression analysis, the K-means method, the cluster analysis, and the decision tree). Specifically, a plurality of groups of the candidate formulae may be prepared simultaneously and concurrently by using a plurality of different algorithms with the index state information which is multivariate data composed of the blood data and the index data obtained by analyzing blood obtained from a large number of healthy groups and MCI groups. For example, the two different candidate formulae may be formed by performing the discriminant analysis and the logistic regression analysis simultaneously with the different algorithms. Alternatively, the candidate formula may be formed by converting the index state information with the candidate formula prepared by performing the principal component analysis and then performing the discriminant analysis of the converted index state information. In this way, it is possible to finally prepare the most suitable formula for the evaluation.

The candidate formula prepared by the principal component analysis is a linear expression including each explanatory variable maximizing the variance of all blood data. The candidate formula prepared by the discriminant analysis is a high-powered expression (including exponential and logarithmic expressions) including each explanatory variable minimizing the ratio of the sum of the variances in respective groups to the variance of all blood data. The candidate formula prepared by using the support vector machine is a high-powered expression (including kernel function) including each explanatory variable maximizing the boundary between groups. The candidate formula prepared by using the multiple regression analysis is a high-powered expression including each explanatory variable minimizing the sum of the distances from all blood data. The candidate formula prepared by using the Cox regression analysis is a linear model including a logarithmic hazard ratio, and is a linear expression including each explanatory variable with a coefficient thereof maximizing the likelihood of the linear model. The candidate formula prepared by using the logistic regression analysis is a linear model expressing logarithmic odds of probability, and a linear expression including each explanatory variable maximizing the likelihood of the probability. The K-means method is a method of searching k pieces of neighboring blood data in various groups, designating the group containing the greatest number of the neighboring points as its data-belonging group, and selecting the explanatory variable that makes the group to which input blood data belong agree well with the designated group. The cluster analysis is a method of clustering (grouping) the points closest in entire blood data. The decision tree is a method of ordering explanatory variables and predicting the group of blood data from the pattern possibly held by the higher-ordered explanatory variable.

Returning to the description of the formula-preparing processing, the control device verifies (mutually verifies) the candidate formula prepared in step 1 based on a particular verifying method (step 2). The verification of the candidate formula is performed on each other to each candidate formula prepared in step 1. In step 2, at least one of discrimination rate, sensitivity, specificity, information criterion, ROC_AUC (area under the curve in a receiver operating characteristic curve), and the like of the candidate formula may be verified by at least one of bootstrap method, holdout method, N-fold method, leave-one-out method, and the like. In this way, it is possible to prepare the candidate formula higher in predictability or reliability, by taking the index state information and the evaluation condition into consideration.

The discrimination rate is a rate in which a subject to be evaluated whose true state is negative (for example, the subject who does not have MCI) is correctly evaluated as being negative by the evaluation method according to the present embodiment and a subject to be evaluated whose true state is positive (for example, the subject who has MCI) is correctly evaluated as being positive by the evaluation method according to the present embodiment. The sensitivity is a rate in which a subject to be evaluated whose true state is positive is correctly evaluated as being positive by the evaluation method according to the present embodiment. The specificity is a rate in which a subject to be evaluated whose true state is negative is correctly evaluated as being negative by the evaluation method according to the present embodiment. The Akaike information criterion is a criterion representing how observation data agrees with a statistical model, for example, in the regression analysis, and it is determined that the model in which the value defined by "-2×(maximum log-likelihood of statistical model)+2×(the number of free parameters of statistical model)" is smallest is the best. ROC_AUC is defined as the area under the receiver operating characteristics curve (ROC) created by plotting (x, y)=(1-specificity, sensitivity) on two-dimensional coordinates. The value of ROC_AUC is 1 in perfect discrimination, and the closer this value is to 1, the higher the discriminative characteristic. The predictability is the average of discrimination rates, sensitivities, or specificities obtained by repeating the validation of the candidate formula. The robustness refers to the variance of discrimination rates, sensitivities, or specificities obtained by repeating the validation of the candidate formula.

Returning to the description of the formula-preparing processing, the control device selects a combination of the blood data contained in the index state information used in preparing the candidate formula, by selecting an explanatory variable of the candidate formula based on a predetermined explanatory variable-selecting method (step 3). In step 3, the selection of the explanatory variable may be performed on each candidate formula prepared in step 1. In this way, it is possible to select the explanatory variable of the candidate formula properly. The step 1 is executed once again by using the index state information including the blood data selected in step 3. In step 3, the explanatory variable of the candidate formula may be selected based on at least one of stepwise method, best path method, local search method, and genetic algorithm from the verification result obtained in step 2. The best path method is a method of selecting an explanatory variable by optimizing an evaluation index of the candidate formula while eliminating the explanatory variables contained in the candidate formula one by one.

Returning to the description of the formula-preparing processing, the control device prepares the formula used for the evaluation by repeatedly performing steps 1, 2 and 3, and based on the verification results thus accumulated, selecting the candidate formula used for the evaluation from the candidate formulae (step 4). In the selection of the candidate formula, there are cases where the optimum formula is selected from the candidate formulae prepared in the same formula-preparing method or the optimum formula is selected from all candidate formulae.

As described above, in the formula-preparing processing, the processing for the preparation of the candidate formulae, the verification of the candidate formulae, and the selection of the explanatory variables in the candidate formulae are performed based on the index state information in a series of operations in a systematized manner, whereby the formula most appropriate for evaluating a state of MCI can be prepared. In other words, in the formula-preparing processing, at least one value of concentration values of the 22 kinds of amino acids and the 30 kinds of amino acid related metabolites and test values of the 7 kinds of blood biochemical test items is used in multivariate statistical analysis, and for selecting the optimum and robust combination of the explanatory variables, the explanatory variable-selecting method is combined with cross-validation to extract the formula having high evaluation performance.

### 2-2. System configuration

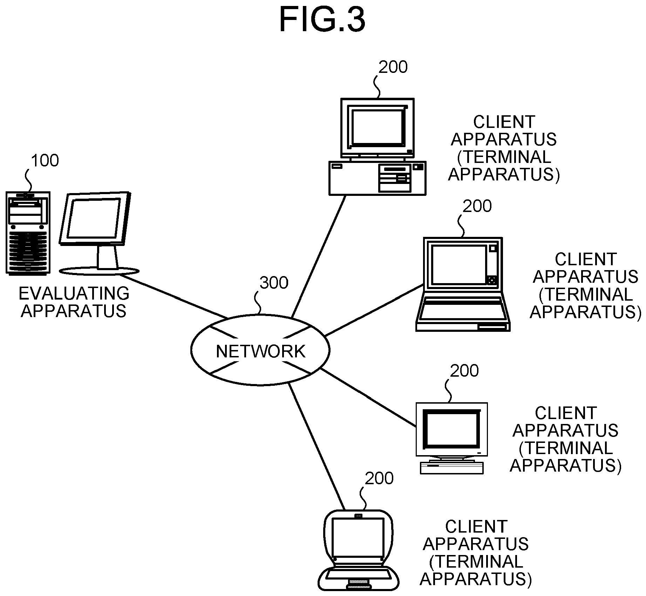

Hereinafter, the configuration of the evaluating system according to the second embodiment (hereinafter referred to sometimes as the present system) will be described with reference to FIGS. 3 to 14. This system is merely one example, and the present invention is not limited thereto. In particular, herein, when a state of MCI is evaluated, a case of using a value of the formula or the converted value thereof is described as one example. However, for example, at least one value of concentration values of the 22 kinds of amino acids and the 30 kinds of amino acid related metabolites and test values of the 7 kinds of blood biochemical test items or the converted value thereof (for example, a concentration standard score) may be used.

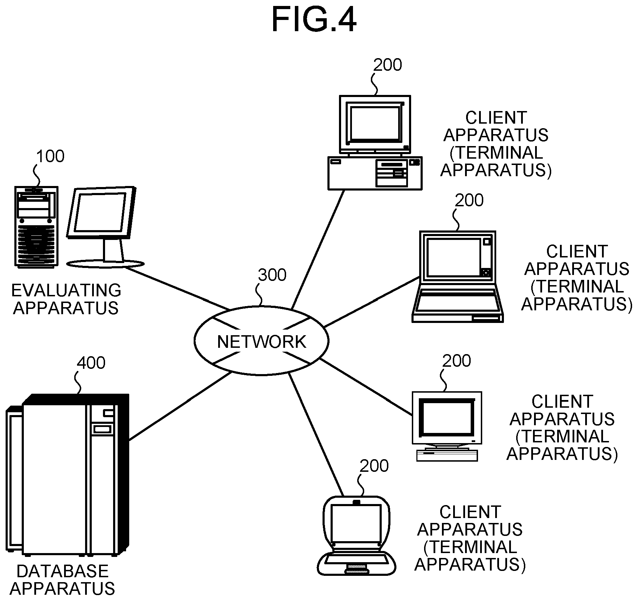

First, an entire configuration of the present system will be described with reference to FIGS. 3 and 4. FIG. 3 is a diagram showing an example of the entire configuration of the present system. FIG. 4 is a diagram showing another example of the entire configuration of the present system. As shown in FIG. 3, the present system is constituted in which the evaluating apparatus 100 that evaluates the state of MCI for the individual as the subject and the client apparatus 200 (corresponding to the terminal apparatus of the present invention) that provides the blood data of the individual on the at least one value of concentration values of the 22 kinds of amino acids and the 30 kinds of amino acid related metabolites and test values of the 7 kinds of blood biochemical test items in blood, are communicatively connected to each other via a network 300.

In the present system, the client apparatus 200 that provides data for use in evaluation and the client apparatus 200 that receives the evaluation result may be different. In the present system as shown in FIG. 4, in addition to the evaluating apparatus 100 and the client apparatus 200, the database apparatus 400 storing, for example, the index state information used in preparing the formula in the evaluating apparatus 100 and the formula used for the evaluation, may be communicatively connected via the network 300. In this configuration, for example, information that may be helpful in knowing the state of MCI is provided via the network 300 from the evaluating apparatus 100 to the client apparatuses 200 and the database apparatus 400, or from the client apparatuses 200 and the database apparatus 400 to the evaluating apparatus 100. The information that may be helpful in knowing the state of MCI is, for example, information on the measured value of a particular item as to the state of MCI of organisms including human. The information that may be helpful in knowing the state of MCI is generated in the evaluating apparatus 100, the client apparatus 200, or other apparatuses (e.g., various measuring apparatuses) and stored mainly in the database apparatus 400.

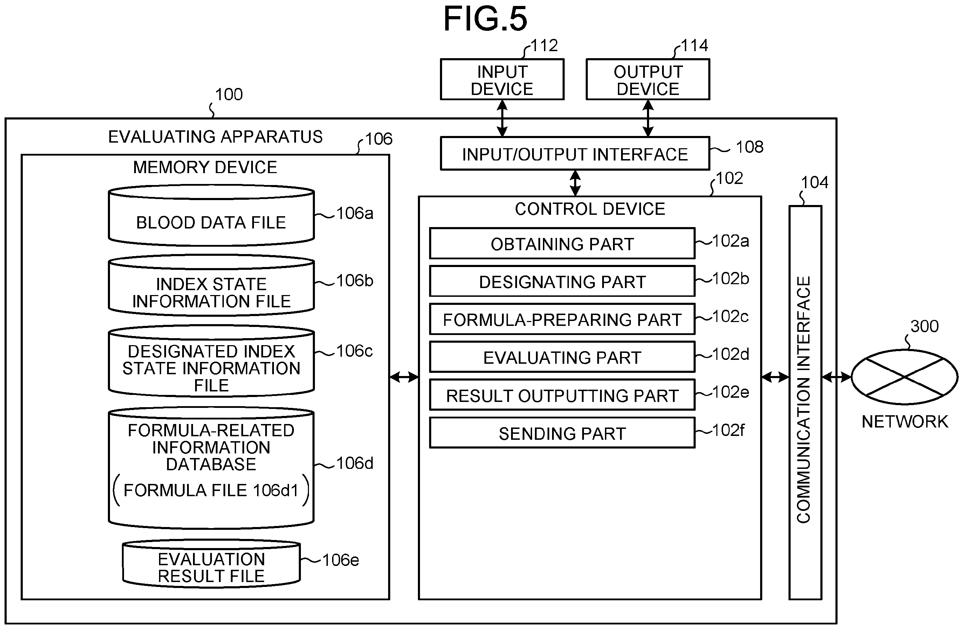

Now, the configuration of the evaluating apparatus 100 in the present system will be described with reference to FIGS. 5 to 11. FIG. 5 is a block diagram showing an example of the configuration of the evaluating apparatus 100 in the present system, showing conceptually only the region relevant to the present invention.

The evaluating apparatus 100 includes: (i) a control device 102, such as CPU (Central Processing Unit), that integrally controls the evaluating apparatus; (ii) a communication interface 104 that connects the evaluating apparatus to the network 300 communicatively via communication apparatuses such as a router and wired or wireless communication lines such as a private line; (iii) a memory device 106 that stores various databases, tables, files and others; and (iv) an input/output interface 108 connected to an input device 112 and an output device 114, and these parts are connected to each other communicatively via any communication channel. The evaluating apparatus 100 may be present together with various analyzers (e.g., an amino acid analyzer) in a same housing. For example, the evaluating apparatus 100 may be a compact analyzing device including components (hardware and software) that calculate (measure) at least one value of concentration values of the 22 kinds of amino acids and the 30 kinds of amino acid related metabolites and test values of the 7 kinds of blood biochemical test items in blood and output (e.g., print or display on a monitor) the calculated value, wherein the compact analyzing device is characterized by further including the evaluating part 102d described later, and using the components to output results obtained by the evaluating part 102d.

The communication interface 104 allows communication between the evaluating apparatus 100 and the network 300 (or a communication apparatus such as a router). Thus, the communication interface 104 has a function to communicate data via a communication line with other terminals.

The input/output interface 108 is connected to the input device 112 and the output device 114. A monitor (including a home television), a speaker, or a printer may be used as the output device 114 (hereinafter, the output device 114 may be described as the monitor 114). A keyboard, a mouse, a microphone, or a monitor functioning as a pointing device together with a mouse may be used as the input device 112.

The memory device 106 is a storage means, and examples thereof include a memory apparatus such as RAM (Random Access Memory) and ROM (Read Only Memory), a fixed disk drive such as a hard disk, a flexible disk, and an optical disk. The memory device 106 stores computer programs giving instructions to the CPU for various processings, together with OS (Operating System). As shown in the figure, the memory device 106 stores the blood data file 106a, the index state information file 106b, the designated index state information file 106c, a formula-related information database 106d, and the evaluation result file 106e.

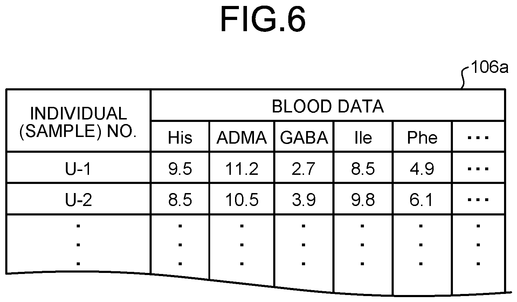

The blood data file 106a stores the blood data on at least one value of concentration values of the 22 kinds of amino acids and the 30 kinds of amino acid related metabolites and test values of the 7 kinds of blood biochemical test items in blood. FIG. 6 is a chart showing an example of information stored in the blood data file 106a. As shown in FIG. 6, the information stored in the blood data file 106a includes an individual number for uniquely identifying the individual (sample) as the subject and the blood data that are correlated to one another. In FIG. 6, the blood data is assumed to be numerical values, i.e., on a continuous scale, but the blood data may be expressed on a nominal scale or an ordinal scale. In the case of the nominal or ordinal scale, any number may be allocated to each state for analysis. The blood data may be combined with a value related to the other biological information (see above).

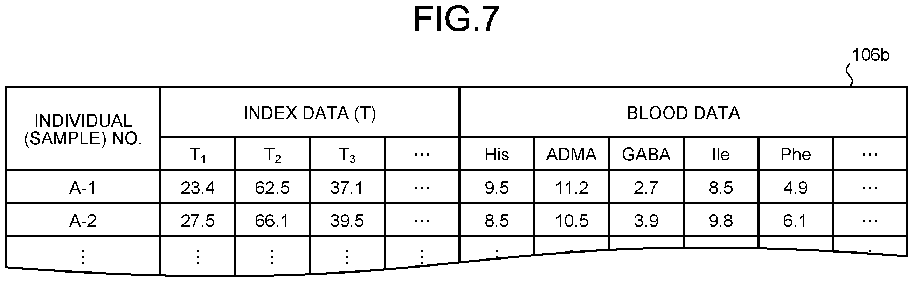

Returning to FIG. 5, the index state information file 106b stores the index state information used in preparing the formula. FIG. 7 is a chart showing an example of information stored in the index state information file 106b. As shown in FIG. 7, the information stored in the index state information file 106b includes the individual number, the index data (T) on the index representing the state of MCI (index T₁, index T₂, index T₃ ...), and the blood data that are correlated to one another. In FIG. 7, the index data and the blood data are assumed to be numerical values, i.e., on a continuous scale, but the index data and the blood data may be expressed on a nominal scale or an ordinal scale. In the case of the nominal or ordinal scale, any number may be allocated to each state for analysis. The index data is, for example, a known index serving as a marker of a state of MCI, and numerical data may be used.

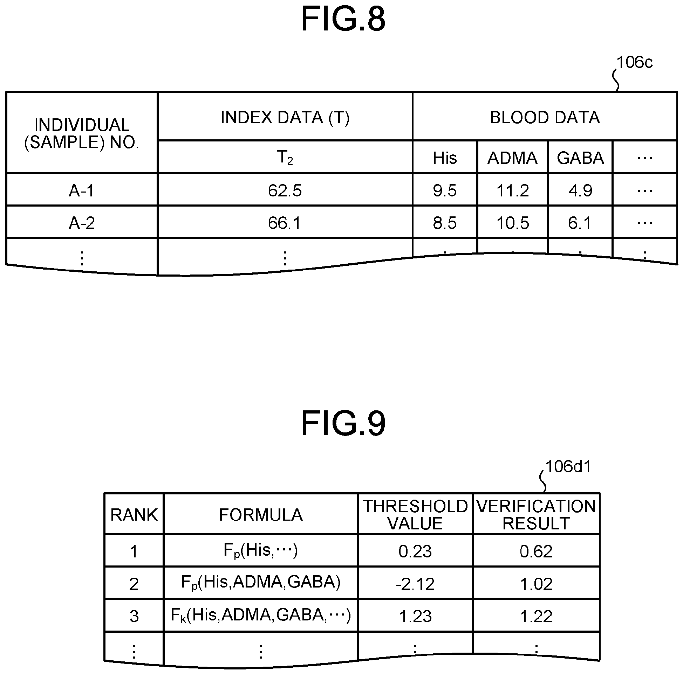

Returning to FIG. 5, the designated index state information file 106c stores the index state information designated in a designating part 102b described below. FIG. 8 is a chart showing an example of information stored in the designated index state information file 106c. As shown in FIG. 8, the information stored in the designated index state information file 106c includes the individual number, the designated index data, and the designated blood data that are correlated to one another.

Returning to FIG. 5, the formula-related information database 106d is composed of the formula file 106d1 storing the formula prepared in a formula-preparing part 102c described below. The formula file 106d1 stores the formulae used for the evaluation. FIG. 9 is a chart showing an example of information stored in the formula file 106d1. As shown in FIG. 9, the information stored in the formula file 106d1 includes a rank, the formula (e.g., Fₚ (His,...), Fₚ (His, ADMA, GABA) , Fₖ (His, ADMA, GABA, ... ) in FIG. 9), a threshold corresponding to each formula-preparing method, and the verification result of each formula (e.g., the value of each formula) that are correlated to one another.

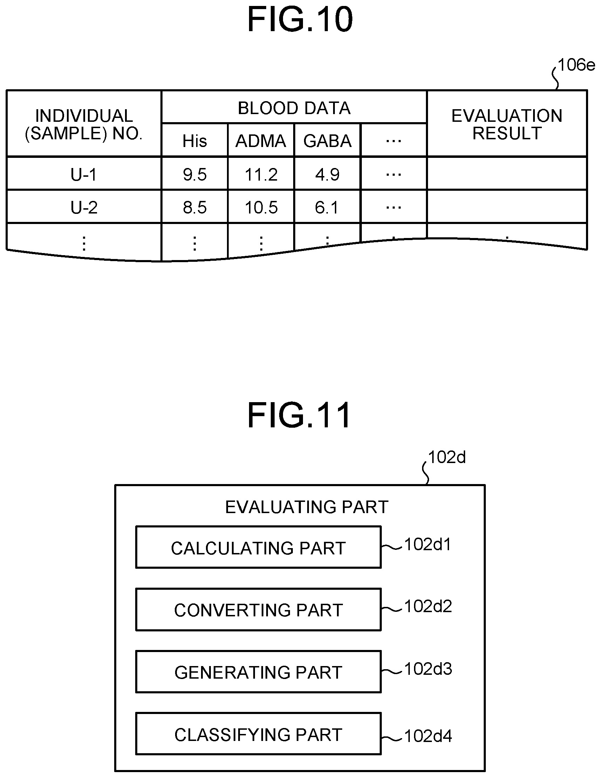

Returning to FIG. 5, the evaluation result file 106e stores the evaluation results obtained in the evaluating part 102d described below. FIG. 10 is a chart showing an example of information stored in the evaluation result file 106e. The information stored in the evaluation result file 106e includes the individual number for uniquely identifying the individual (sample) as the subject, the previously obtained blood data of the individual, and the evaluation result on the state of MCI (for example, the value of the formula calculated by a calculating part 102d1 described below, the converted value of the value of the formula obtained by a converting part 102d2 described below, the positional information generated by a generating part 102d3 described below, or the classification result obtained by a classifying part 102d4 described below), that are correlated to one another.

Returning to FIG. 5, the control device 102 has an internal memory storing, for example, control programs such as OS (Operating System), programs for various processing procedures, and other needed data, and performs various information processings according to these programs. As shown in the figure, the control device 102 includes mainly an obtaining part 102a, the designating part 102b, the formula-preparing part 102c, the evaluating part 102d, a result outputting part 102e, and a sending part 102f. The control device 102 performs data processings such as removal of data including defective, removal of data including many outliers, and removal of explanatory variables for the defective-including data in the index state information transmitted from the database apparatus 400 and in the blood data transmitted from the client apparatus 200.

The obtaining part 102a obtains information (specifically, blood data, index state information, formula, and the like). For example, the obtaining part 102a may receive information (specifically, blood data, index state information, formula, and the like) transmitted from the client apparatus 200 or the database apparatus 400 via the network 300 to obtain information. The obtaining part 102a may receive data for use in evaluation transmitted from a client apparatus 200 different from the client apparatus 200 to which the evaluation result is transmitted. When the evaluating apparatus 100 includes a mechanism (including hardware and software) for reading information recorded on a recording medium, the obtaining part 102a may obtain information by reading information recorded on a recording medium (specifically, blood data, index state information, formula, and the like) through the mechanism. The specifying part 102b specifies target index data and blood data in creating a formula.

The formula-preparing part 102c generates the formula based on the index state information obtained in the obtaining part 102a or the index state information designated in the designating part 102b. If the formulae are stored previously in a predetermined region of the memory device 106, the formula-preparing part 102c may generate the formula by selecting the desired formula out of the memory device 106. Alternatively, the formula-preparing part 102c may generate the formula by selecting and downloading the desired formula from another computer apparatus (e.g., the database apparatus 400) in which the formulae are previously stored.

The evaluating part 102d evaluates the state of MCI for the individual by using the previously obtained formula (for example, the formula prepared by the formula-preparing part 102c or the formula obtained by the obtaining part 102a) and the at least one value included in the blood data of the individual obtained by the obtaining part 102a to calculate the value of the formula. The evaluating part 102d may evaluate the state of MCI for the individual using the at least one value of concentration values of the 22 kinds of amino acids and the 30 kinds of amino acid related metabolites and test values of the 7 kinds of blood biochemical test items or the converted value of the at least one value (for example, the concentration standard score).

Hereinafter, a configuration of the evaluating part 102d will be described with reference to FIG. 11. FIG. 11 is a block diagram showing the configuration of the evaluating part 102d, and only a part in the configuration related to the present invention is shown conceptually.
The evaluating part 102d includes the calculating part 102d1, the converting part 102d2, the generating part 102d3, and the classifying part 102d4, additionally.

The calculating part 102d1 uses (i) at least one value of concentration values of the 22 kinds of amino acids and the 30 kinds of amino acid related metabolites and test values of the 7 kinds of blood biochemical test items, and (ii) the formula including the explanatory variable to be substituted with the at least one value, to calculate the value of the formula. The evaluating part 102d may store the value of the formula calculated by the calculating part 102d1 as the evaluation result in a predetermined region of the evaluation result file 106e.

The converting part 102d2 converts the value of the formula calculated by the calculating part 102d1, for example, by the conversion method described above. The evaluating part 102d may store the converted value by the converting part 102d2 as the evaluation result in a predetermined region of the evaluation result file 106e. The converting part 102d2 may convert the at least one value included in the blood data, for example, by the conversion method described above.

The generating part 102d3 generates the positional information about the position of the predetermined mark on the predetermined scale visually presented on the display device such as a monitor or the physical medium such as paper, using the value of the formula calculated by the calculating part 102d1 or the converted value by the converting part 102d2 (the concentration value or the test value, or the converted value of the concentration value or the test value may be used as well). The evaluating part 102d may store the positional information generated by the generating part 102d3 as the evaluation result in a predetermined region of the evaluation result file 106e.

The classifying part 102d4 classifies the individual into any one of the categories defined at least considering the degree of the possibility of having MCI, using the value of the formula calculated by the calculating part 102d1 or the converted value by the converting part 102d2 (the concentration value or the test value, or the converted value of the concentration value or the test value may be used as well).

The result outputting part 102e outputs, into the output device 114, for example, the processing results in each processing part in the control device 102 (including the evaluation results obtained by the evaluating part 102d) .

The sending part 102f transmits the evaluation results to the client apparatus 200 that is a sender of the blood data of the individual, and transmits the formulae prepared in the evaluating apparatus 100 and the evaluation results to the database apparatus 400. The sending part 102f may transmit the evaluation result to a client apparatus 200 different from the client apparatus 200 that from which data for use in evaluation is transmitted.

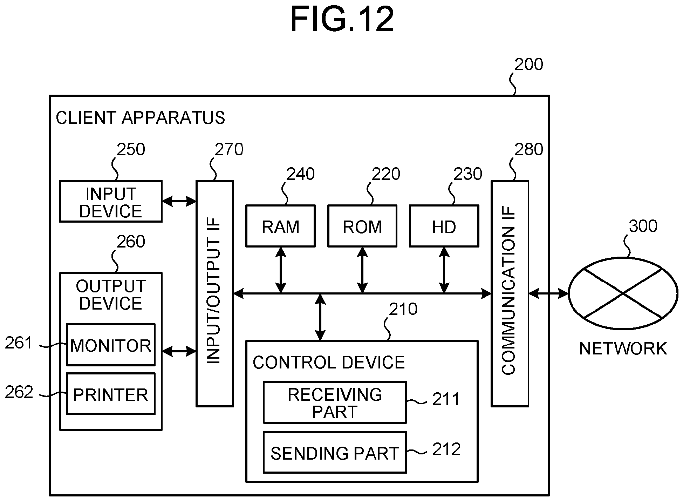

Hereinafter, a configuration of the client apparatus 200 in the present system will be described with reference to FIG. 12. FIG. 12 is a block diagram showing an example of the configuration of the client apparatus 200 in the present system, and only the part in the configuration relevant to the present invention is shown conceptually.

The client apparatus 200 includes a control device 210, ROM 220, HD (Hard Disk) 230, RAM 240, an input device 250, an output device 260, an input/output IF 270, and a communication IF 280 that are connected communicatively to one another through a communication channel. The client apparatus 200 may be realized based on an information processing apparatus (for example, an information processing terminal such as a known personal computer, a workstation, a family computer, Internet TV (Television), PHS (Personal Handyphone System) terminal, a mobile phone terminal, a mobile unit communication terminal, or PDA (Personal Digital Assistants)) connected as needed with peripheral devices such as a printer, a monitor, and an image scanner.

The input device 250 is, for example, a keyboard, a mouse, or a microphone. The monitor 261 described below also functions as a pointing device together with a mouse. The output device 260 is an output means for outputting information received via the communication IF 280, and includes the monitor 261 (including home television) and a printer 262. In addition, the output device 260 may have a speaker or the like additionally. The input/output IF 270 is connected to the input device 250 and the output device 260.

The communication IF 280 connects the client apparatus 200 to the network 300 (or communication apparatus such as a router) communicatively. In other words, the client apparatus 200 is connected to the network 300 via a communication apparatus such as a modem, TA (Terminal Adapter) or a router, and a telephone line, or via a private line. In this way, the client apparatus 200 can access to the evaluating apparatus 100 by using a particular protocol.

The control device 210 has a receiving part 211 and a sending part 212. The receiving part 211 receives various kinds of information such as the evaluation results transmitted from the evaluating apparatus 100, via the communication IF 280. The sending part 212 sends various kinds of information such as the blood data of the individual, via the communication IF 280, to the evaluating apparatus 100.

All or a part of processings of the control device 210 may be performed by CPU and programs read and executed by the CPU. Computer programs for giving instructions to the CPU and executing various processings together with the OS (Operating System) are recorded in the ROM 220 or HD 230. The computer programs, which are executed as they are loaded in the RAM 240, constitute the control device 210 with the CPU. The computer programs may be stored in application program servers connected via any network to the client apparatus 200, and the client apparatus 200 may download all or a part of them as needed. All or any part of processings of the control device 210 may be realized by hardware such as wired-logic.

The control device 210 may include an evaluating part 210a (including a calculating part 210a1, a converting part 210a2, a generating part 210a3, and a classifying part 210a4) having the same functions as the functions of the evaluating part 102d in the evaluating apparatus 100. When the control device 210 includes the evaluating part 210a, the evaluating part 210a may convert the value of the formula (the concentration value or the test value may be used as well) in the converting part 210a2, generate the positional information corresponding to the value of the formula or the converted value (the concentration value or the test value, or the converted value of the concentration value or the test value may be used as well) in the generating part 210a3, and classify the individual into any one of the categories using the value of the formula or the converted value (the concentration value or the test value, or the converted value of the concentration value or the test value may be used as well) in the classifying part 210a4, in accordance with information included in the evaluation results transmitted from the evaluating apparatus 100.

Hereinafter, the network 300 in the present system will be described with reference to FIGS. 3 and 4. The network 300 has a function to connect the evaluating apparatus 100, the client apparatuses 200, and the database apparatus 400 mutually, communicatively to one another, and is for example the Internet, an intranet, or LAN (Local Area Network (including both wired and wireless)). The network 300 may be VAN (Value Added Network), a personal computer communication network, a public telephone network (including both analog and digital), a leased line network (including both analog and digital), CATV (Community Antenna Television) network, a portable switched network or a portable packet-switched network (including IMT2000 (International Mobile Telecommunication 2000) system, GSM (registered trademark) (Global System for Mobile Communications) system, or PDC (Personal Digital Cellular)/PDC-P system), a wireless calling network, a local wireless network such as Bluetooth (registered trademark), PHS network, a satellite communication network (including CS (Communication Satellite), BS (Broadcasting Satellite), ISDB (Integrated Services Digital Broadcasting), and the like), or the like.

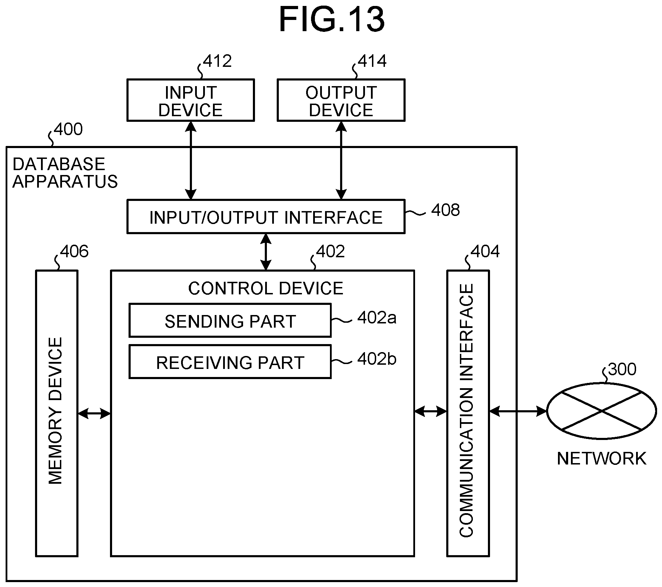

Hereinafter, the configuration of the database apparatus 400 in the present system will be described with reference to FIG. 13. FIG. 13 is a block diagram showing an example of the configuration of the database apparatus 400 in the present system, showing conceptually only the region relevant to the present invention.

The database apparatus 400 has functions to store, for example, the index state information used in preparing the formulae in the evaluating apparatus 100 or the database apparatus, the formulae prepared in the evaluating apparatus 100, and the evaluation results obtained in the evaluating apparatus 100. As shown in FIG. 13, the database apparatus 400 includes; (i) a control device 402, such as CPU, that integrally controls the database apparatus; (ii) a communication interface 404 connecting the database apparatus to the network 300 communicatively via communication apparatuses such as a router and wired or wireless communication circuits such as a private line; (iii) a memory device 406 storing various databases, tables, files (for example, files for Web pages) and others; and (iv) an input/output interface 408 connected to an input device 412 and an output device 414, and these parts are connected communicatively to each other via any communication channel.

The memory device 406 is a storage means, and, examples thereof include a memory apparatus such as RAM or ROM, a fixed disk drive such as a hard disk, a flexible disk, and an optical disk. The memory device 406 stores, for example, various programs used in various processings. The communication interface 404 allows communication between the database apparatus 400 and the network 300 (or a communication apparatus such as a router). Thus, the communication interface 404 has a function to communicate data via a communication line with other terminals. The input/output interface 408 is connected to the input device 412 and the output device 414. A monitor (including a home television), a speaker, or a printer may be used as the output device 414. A keyboard, a mouse, a microphone, or a monitor functioning as a pointing device together with a mouse may be used as the input device 412.

The control device 402 has an internal memory storing, for example, control programs such as OS (Operating System), programs for various processing procedures, and other needed data, and performs various information processings according to these programs. As shown in the figure, the control device 402 includes mainly a sending part 402a and a receiving part 402b. The sending part 402a transmits various kinds of information such as the index state information and the formulae to the evaluating apparatus 100. The receiving part 402b receives various kinds of information such as the formula and the evaluation results, transmitted from the evaluating apparatus 100.

In the present description, the evaluating apparatus 100 executes the obtainment of the blood data, the calculation of the value of the formula, the classification of the individual into the category, and the transmission of the evaluation results, while the client apparatus 200 executes the reception of the evaluation results, described as an example. However, when the client apparatus 200 includes the evaluating unit 210a, the evaluating apparatus 100 only has to execute the calculation of the value of the formula. For example, the conversion of the value of the formula, the generation of the positional information, and the classification of the individual into the category may be appropriately shared between the evaluating apparatus 100 and the client apparatus 200.

For example, when the client apparatus 200 receives the value of the formula from the evaluating apparatus 100, the evaluating unit 210a may convert the value of the formula in the converting unit 210a2, generate the positional information corresponding to the value of the formula or the converted value in the generating unit 210a3, and classify the individual into any one of the categories using the value of the formula or the converted value in the classifying unit 210a4.

When the client apparatus 200 receives the converted value from the evaluating apparatus 100, the evaluating unit 210a may generate the positional information corresponding to the converted value in the generating unit 210a3, and classify the individual into any one of the categories using the converted value in the classifying unit 210a4.

When the client apparatus 200 receives the value of the formula or the converted value and the positional information from the evaluating apparatus 100, the evaluating unit 210a may classify the individual into any one of the categories using the value of the formula or the converted value in the classifying unit 210a4.

### 2-3. Other embodiments

In addition to the second embodiment described above, the evaluating apparatus, the calculating apparatus, the evaluating method, the calculating method, the evaluating program, the calculating program, the recording medium, the evaluating system, and the terminal apparatus according to the present invention can be practiced in various different embodiments within the technological scope of the claims.

Of the processings described in the second embodiment, all or a part of the processings described as automatically performed ones may be manually performed, or all or a part of the processings described as manually performed ones may be also automatically performed by known methods.

In addition, the processing procedures, the control procedures, the specific names, the information including parameters such as registered data of various processings and retrieval conditions, the screen examples, and the database configuration shown in the description and the drawings may be arbitrarily modified unless otherwise specified.

The components of the evaluating apparatus 100 shown in the figures are functionally conceptual and therefore not be physically configured as shown in the figures.

For example, for the operational functions provided in the evaluating apparatus 100, in particular, for the operational functions performed in the control device 102, all or part thereof may be implemented by the CPU (Central Processing Unit) and programs interpreted and executed in the CPU, or may be implemented by wired-logic hardware. The program is recorded in a non-transitory tangible computer-readable recording medium including programmed instructions for making an information processing apparatus execute the evaluating method or the calculating method according to the present invention, and is mechanically read as needed by the evaluating apparatus 100. More specifically, computer programs to give instructions to the CPU in cooperation with the OS (operating system) to perform various processes are recorded in the memory device 106 such as ROM or a HDD (hard disk drive). The computer programs are executed by being loaded to RAM, and form the control unit in cooperation with the CPU.

The computer programs may be stored in an application program server connected to the evaluating apparatus 100 via an arbitrary network, and all or part thereof can be downloaded as necessary.

The evaluating program or the calculating program according to the present invention may be stored in the non-transitory tangible computer-readable recording medium, or can be configured as a program product. The "recording medium" mentioned here includes any "portable physical medium" such as a memory card, a USB (universal serial bus) memory, an SD (secure digital) card, a flexible disk, a magneto-optical disc, ROM, EPROM (erasable programmable read only memory), EEPROM (registered trademark) (electronically erasable and programmable read only memory), CD-ROM (compact disk read only memory), MO (magneto-optical disk), DVD (digital versatile disk), and Blu-ray (registered trademark) Disc.

The "program" mentioned here is a data processing method described in an arbitrary language or description method, and therefore any form such as a source code and a binary code is acceptable. The "program" is not necessarily limited to a program configured as a single unit, and, therefore, includes those dispersively configured as a plurality of modules and libraries and those in which the function of the program is achieved in cooperation with separate programs represented as OS (operating system). Any known configuration and procedures can be used as a specific configuration and reading procedure to read a recording medium by each apparatus shown in the embodiments, an installation procedure after the reading, and the like.

The various databases and the like stored in the memory device 106 is a storage unit such as a memory device such as RAM and ROM, a fixed disk drive such as a hard disk, a flexible disk, or an optical disc. The memory device 106 stores therein various programs, tables, databases, files for Web (World Wide Web) pages, and the like used to perform various processes and to provide Web sites.

The evaluating apparatus 100 may be configured as an information processing apparatus such as known personal computer and work station, or may be configured as the information processing apparatus connected to an arbitrary peripheral device. The evaluating apparatus 100 may be provided by installing software (including the programs and the data, etc.) to cause the information processing apparatus to implement the evaluating method or the calculating method according to the present invention.

Furthermore, a specific configuration of dispersion or integration of the apparatuses is not limited to the shown one. The apparatuses can be configured by functionally or physically dispersing or integrating all or part of the apparatuses in arbitrary units according to various types of additions or the like or according to functional loads. In other words, the embodiments may be implemented in arbitrary combinations thereof or an embodiment may be selectively implemented.

### Example 1

The plasma samples of MCI people aged 65 years old or older with clinical diagnosis of MCI (MCI group: 56 subjects) and elderly people aged 65 years old or older presumably with normal cognitive function (healthy group: 73 subjects) were measured by the amino acid analyzing method (A) to determine the blood concentration of metabolites.

The ability to discriminate between the MCI group and the healthy group was evaluated using data listed below.
- the plasma concentrations (nmol/ml) of 22 kinds of amino acids (aABA, Ala, Arg, Asn, Cit, Glu, Gln, Gly, His, Ile, Leu, Lys, Met, Orn, Phe, Pro, Ser, Thr, Trp, Tyr, Val, and Taurine)
- the plasma concentrations (nmol/ml) of 27 kinds of amino acid related metabolites (1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, N6-AcLys, N8-AcSpd, Opthalmic acid, PEA, Pipecolic acid, Put, Sar, SDMA, Spd, Spm, and Thioproline)
- the peak areas of 4 kinds of amino acid related metabolites (MeCys, Pyrazole-1-Ala, MCSO1, and MCSO2)
- 4 kinds of metabolite ratios (Kyn/Trp, Kyn/BCAA, MCSO2/MeCys, and MCSO1/MCSO2)
- the plasma concentration of BCAA (the total value of plasma concentrations of Val, Leu, and Ile)
- the plasma concentration of EAA (the total value of plasma concentrations of His, Ile, Leu, Lys, Met, Phe, Thr, Trp, and Val)

### MCSO1 and MCSO2 were defined as follows.

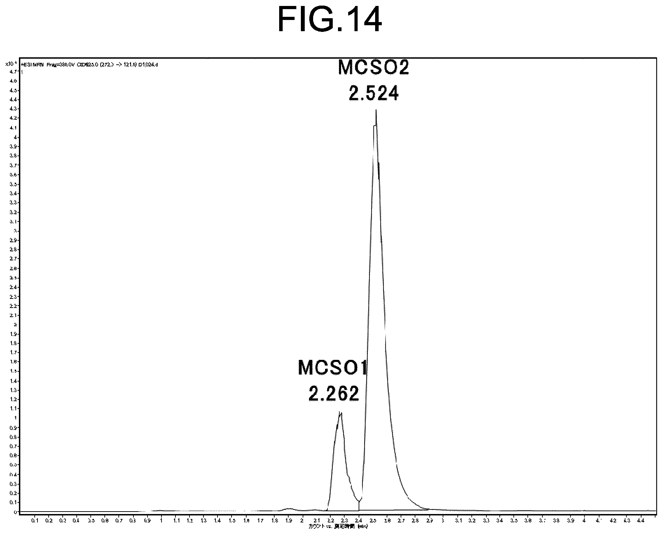

Acetonitrile was added to deproteinize pooled plasma extracted from healthy subjects (32 subjects), pre-column derivatization was then performed using a labeling reagent (3-aminopyridyl-N-hydroxysuccinimidyl carbamate), and analysis was conducted by a liquid chromatograph mass spectrometer (LC-MS/MS) according to the analysis conditions below. Among the peaks detected from the resultant extracted ion chromatogram (FIG. 14), the peak around 2.3 minutes and the peak around 2.5 minutes were determined as MCSO1 and MCSO2, respectively.

### Analysis Conditions

(1) Liquid chromatograph mass spectrometer (LC-MS/MS): HPLC system "1290 Infinity II" (Agilent Technologies), mass analysis system "6495B Triple Quad LC/MS" (Agilent Technologies)
(2) Analytical column: "Inertcil ODS-3 (particle size: 2.0 µm, inner diameter: 2.1 mm, length: 150 mm)" (GL Sciences Inc. )
(3) Column temperature: 50°C
(4) Eluent A: eluent for APDS amionotag Wako (Wako Pure Chemical Industries, Ltd.)
(5) Eluent B: acetonitrile/water (60:40, v/v)
(6) Flow rate: 0.5 mL/min
(7) Gradient condition for mobile phase (time and the proportion of eluent B):
   0.00 minutes to 3.50 minutes: 6%
   3.50 minutes to 5.00 minutes: 6% to 8%
   5.00 minutes to 7.00 minutes: 8% to 20%
   7.00 minutes to 8.50 minutes: 20%
   8.50 minutes to 9.50 minutes: 20% to 25%
   9.50 minutes to 12.00 minutes: 25%
   12.00 minutes to 12.01 minutes: 25% to 35%
   12.01 minutes to 14.00 minutes: 35% to 80%
   14.00 minutes to 14.01 minutes: 80% to 95%
   14.01 minutes to 16.00 minutes: 95%
   16.00 minutes to 16.10 minutes: 95% to 6%
   16.10 minutes to 19.00 minutes: 6%
(8) Ionization: electrospray, positive mode
(9) Scan type: Selected reaction monitoring

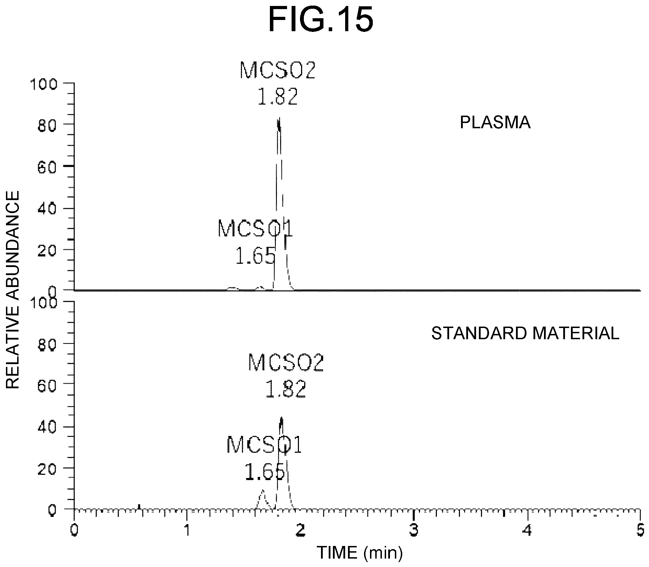

For MCSO1 and MCSO2, structural analysis was separately conducted. For structure determination, acetonitrile was added to deproteinize pooled plasma extracted from healthy subjects (32 subjects), and the sample and a reference material S-methylsysteine sulfoxide (Santa Cruz Biotechnology, Inc., product number sc-204899) were subjected to pre-column derivatization using a labeling reagent (3-aminopyridyl-N-hydroxysuccinimidyl carbamate) and analyzed by a liquid chromatograph mass spectrometer (LC-MS/MS) according to the analysis conditions below. In the resultant extracted ion chromatogram, the retention times of the peaks detected around 1.7 minutes and around 1.8 minutes agreed in the plasma sample and the standard material (FIG. 15). The accurate masses of the plasma and the standard material agreed with the theoretical value (272.0700), and the structure for the peaks was determined as S-methylsysteine sulfoxide. S-methylsysteine sulfoxide is a diastereomer mixture (a mixture of (+)-S-Carboxymethyl-L-cysteine (RS) and (-)-S-Carboxymethyl-L-cysteine (RR)) and therefore two peaks are detected. The RS is more naturally occurring. Analysis Conditions
(1) Liquid chromatograph mass spectrometer (LC-MS/MS): HPLC system "UFLC System" (Shimadzu Corporation), mass analysis system "Q-Exactive TM Plus" (Thermo Fisher Scientific K.K.)
(2) Analytical column: "Inertcil ODS-3 (particle size: 2.0 µm, inner diameter: 2.1 mm, length: 100 mm) (GL Sciences Inc.)
(3) Column temperature: 50°C
(4) Eluent A: eluent for APDS amionotag Wako (Wako Pure Chemical Industries, Ltd.)
(5) Eluent B: acetonitrile/water (60:40, v/v)
(6) Flow rate: 0.5 mL/min
(7) Gradient condition for mobile phase (time and the portion of eluent B):
   0.00 minutes to 0.01 minutes: 5% to 6%
   0.01 minutes to 3.50 minutes: 6%
   3.50 minutes to 5.00 minutes: 6% to 8%
   5.00 minutes to 6.00 minutes: 8% to 20%
   6.00 minutes to 8.50 minutes: 20%
   8.50 minutes to 9.50 minutes: 20% to 24%
   9.50 minutes to 12.00 minutes: 24%
   12.00 minutes to 12.01 minutes: 24% to 35%
   12.01 minutes to 15.00 minutes: 35% to 80%
   15.00 minutes to 15.10 minutes: 80% to 95%
   15.10 minutes to 17.00 minutes: 95%
   17.01 minutes to 19.00 minutes: 5%
(8) Ionization: electrospray, positive mode
(9) Scan type: full-scan mode

For the blood concentrations in the healthy group and the MCI group, in the test with null hypothesis (Mann-Whitney U test) that "both groups have the same average value", the amino acids and the amino acid related metabolites significant (p<0.05) for the healthy group were Taurine, Spd, PEA, MCSO2, Pyrazole-1-Ala, MeCys, MCSO2/MeCys, Put, Lys, His, MCSO1/MCSO2, hArg, Thr, Ala, EAA, Met, Hypotaurine, aABA, and Kyn/BCAA.

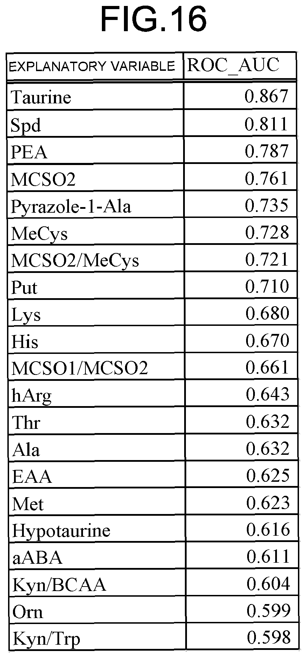

FIG. 16 shows the result (ROC_AUC) of logistic regression using each amino acid and each amino acid related metabolite. The amino acids and the amino acid related metabolites with ROC_AUC equal to or greater than 0.595 were Taurine, Spd, PEA, MCSO2, Pyrazole-1-Ala, MeCys, MCSO2/MeCys, Put, Lys, His, MCSO1/MCSO2, hArg, Thr, Ala, EAA, Met, Hypotaurine, aABA, Kyn/BCAA, Orn, and Kyn/Trp. Taurine, Spd, PEA, MCSO2, MeCys, MCSO2/MeCys, Put, Lys, His, hArg, Thr, Ala, EAA, Met, Hypotaurine, aABA, and Orn decreased in the MCI group, whereas Pyrazole-1-Ala, MCSO1/MCSO2, Kyn/BCAA, and Kyn/Trp increased in the MCI group. The concentrations of these metabolites are presumed to be valuable for evaluating a state of MCI in consideration of a healthy state.

### Example 2

The sample data obtained in Example 1 was used. A multivariate discriminant (multivariate function) for discriminating between the two groups, namely, the MCI group and the healthy group, including explanatory variables to be substituted with the plasma concentrations of metabolites was found.

A logistic regression expression was used as the multivariate discriminant. The plasma concentrations of the 22 kinds of amino acids, the plasma concentrations of the 27 kinds of amino acid related metabolites, the peak areas of the 4 kinds of amino acid related metabolites, and the 4 kinds of metabolite ratios that are listed in Example 1 were searched for a combination of two explanatory variables to be included in the logistic regression expression, and a search for logistic regression expressions having a satisfactory ability to discriminate between the MCI group and the healthy group was conducted.

A list of logistic regression expressions with two explanatory variables, in which ROC_AUC for the MCI group and the healthy group is equal to or greater than 0.700, is provided in [1. Two-Explanatory Variable Expressions] below. These logistic regression expressions are presumed to be valuable in the evaluation because ROC_AUC is high. In [1. Two-Explanatory Variable Expressions] below, the explanatory variables included in the expression and ROC_AUC are listed for each expression (the same applies hereinafter).

### Example 3

The sample data used in Example 1 was used. A multivariate discriminant (multivariate function) for discriminating between the two groups, namely, the MCI group and the healthy group, including explanatory variables to be substituted with the plasma concentrations of metabolites was found.

A logistic regression expression was used as the multivariate discriminant. The plasma concentrations of the 22 kinds of amino acids, the plasma concentrations of the 27 kinds of amino acid related metabolites, the peak areas of the 4 kinds of amino acid related metabolites, and the 4 kinds of metabolite ratios were searched for a combination of three explanatory variables to be included in the logistic regression expression, in the same manner as in Example 2, and a search for logistic regression expressions having a satisfactory ability to discriminate between the MCI group and the healthy group was conducted.

A list of logistic regression expressions with three explanatory variables, in which ROC_AUC for the MCI group and the healthy group is equal to or greater than 0.750, is provided in [2. Three-Explanatory Variable Expressions] below. These logistic regression expressions are presumed to be valuable in the evaluation because ROC_AUC is high.

### Example 4

The sample data used in Example 1 was used. A multivariate discriminant (multivariate function) for discriminating between the two groups, namely, the MCI group and the healthy group, including explanatory variables to be substituted with the plasma concentrations of metabolites and the age was found.

A logistic regression expression was used as the multivariate discriminant. The plasma concentrations of the 22 kinds of amino acids, the plasma concentrations of the 27 kinds of amino acid related metabolites, the peak areas of the 4 kinds of amino acid related metabolites, the 4 kinds of metabolite ratios, and the ages were searched for a combination of four explanatory variables to be included in the logistic regression expression, and a search for logistic regression expressions having a satisfactory ability to discriminate between the MCI group and the healthy group was conducted.

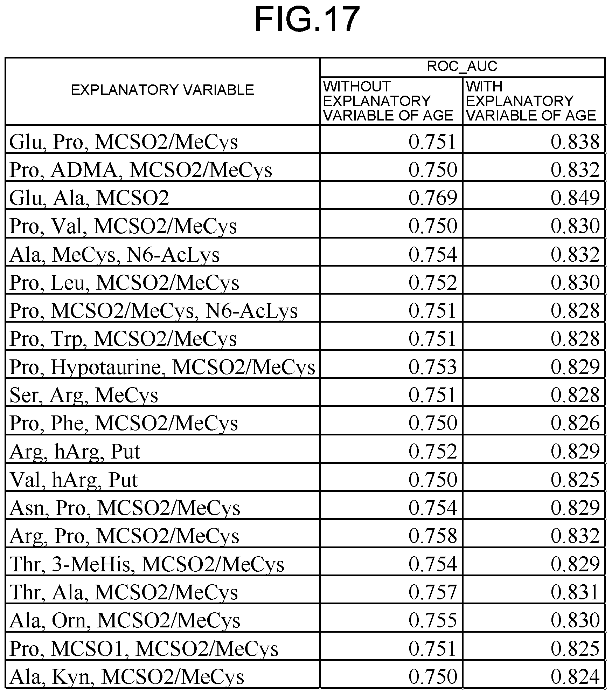

Among the obtained logistic regression expressions, the top 20 expressions with the explanatory variable of age are listed in FIG. 17, from among the expressions in which ROC_AUC for the MCI group and the healthy group was increased by incorporating the age into the explanatory variables.

Among the obtained logistic regression expressions, the combinations in which ROC_AUC was increased by adding the explanatory variable of age to 3485 expressions listed in [2. Three-Explanatory Variable Expressions] below were 3053 expressions. Adding the age to the explanatory variables is presumed to be valuable for the evaluation because adding the age to the explanatory variables improves ROC_AUC in the majority of logistic regression expressions.

### Example 5

The sample data used in Example 1 was used. A multivariate discriminant (multivariate function) for discriminating between the two groups, namely, the MCI group and the healthy group, including explanatory variables to be substituted with the plasma concentrations of metabolites was found.

A logistic regression expression was used as the multivariate discriminant. The plasma concentrations of the 22 kinds of amino acids, the plasma concentrations of the 27 kinds of amino acid related metabolites, the peak areas of the 4 kinds of amino acid related metabolites, and the 4 kinds of metabolite ratios were searched for a combination of six explanatory variables to be included in the logistic regression expression, and a search for logistic regression expressions having a satisfactory ability to discriminate between the MCI group and the healthy group was conducted. To remove combinations with many defectives, combinatorial expressions with six explanatory variables in which the number of data usable for calculation is smaller than 70 in the MCI group and the healthy group in total were removed from the search results.

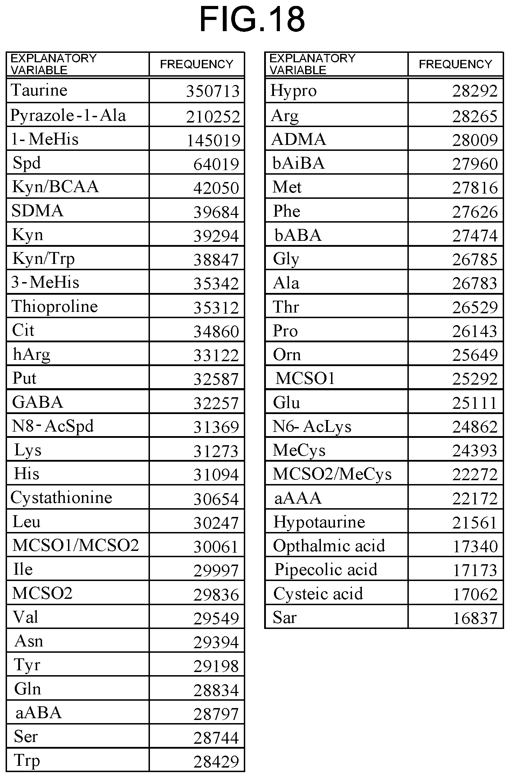

Among the obtained logistic regression expressions, the frequencies of occurrence of amino acid/amino acid related metabolite explanatory variables included in 359,603 expressions with ROC_AUC equal to or greater than 0.900 were calculated and listed in FIG. 18.

This demonstrated that the frequencies of occurrence of Taurine, Pyrazole-1-Ala, 1-MeHis, Spd, Kyn/BCAA, SDMA, Kyn, Kyn/Trp, 3-MeHis, and Thioproline were as high as 35,000 times or more. In particular, it was demonstrated that the frequencies of occurrence of Spd and Kyn/BCAA were as high as 40,000 times or more. It was also demonstrated that the frequencies of occurrence of Taurine, Pyrazole-1-Ala, and 1-MeHis were as high as 100,000 times or more.

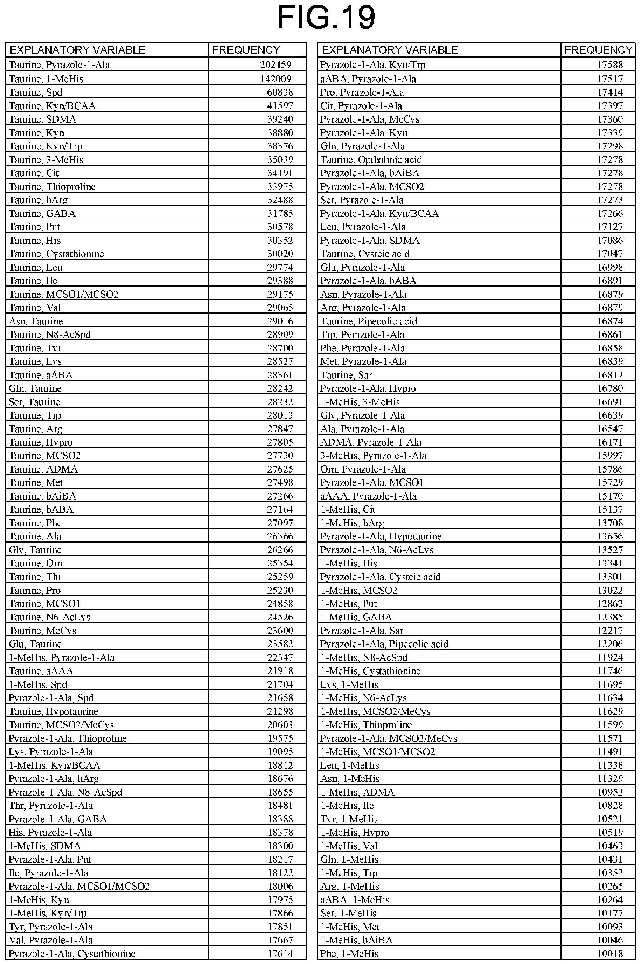

The frequencies of occurrence of combinations of two kinds of amino acid/amino acid related metabolite explanatory variables were calculated and listed in FIG. 19.

This demonstrated that the frequencies of occurrence of the following combinations of two kinds of amino acid/amino acid related metabolite explanatory variables were as high as 10,000 times or more. Taurine,Pyrazole-1-Ala;Taurine,1-MeHis;Taurine,Spd;Taurine, Kyn/BCAA;Taurine,SDMA;Taurine,Kyn;Taurine,Kyn/Trp;Taurine,3 -MeHis;Taurine,Cit;Taurine,Thioproline;Taurine,hArg;Taurine, GABA;Taurine,Put;Taurine,His;Taurine,Cystathionine;Taurine, Leu;Taurine,lie;Taurine,MCSO1/MCSO2;Taurine,Val;Asn,Taurin e;Taurine,N8-AcSpd;Taurine,Tyr;Taurine,Lys;Taurine,aABA;Gln, Taurine;Ser,Taurine;Taurine,Trp;Taurine,Arg;Taurine,Hypro;T aurine,MCSO2;Taurine,ADMA;Taurine,Met;Taurine,bAiBA;Taurine, bABA;Taurine,Phe;Taurine,Ala;Gly,Taurine;Taurine,Orn;Taurin e,Thr;Taurine,Pro;Taurine,MCSO1;Taurine,N6-AcLys;Taurine,Me Cys;Glu,Taurine;1-MeHis,Pyrazole-1-Ala;Taurine,aAAA;1-MeHis, Spd;Pyrazole-1-Ala,Spd;Taurine,Hypotaurine;Taurine,MCSO2/Me Cys;Pyrazole-1-Ala,Thioproline;Lys,Pyrazole-1-Ala;1-MeHis,K yn/BCAA;Pyrazole-1-Ala,hArg;Pyrazole-1-Ala,N8-AcSpd;Thr,Pyr azole-1-Ala;Pyrazole-1-Ala,GABA;His,Pyrazole-1-Ala;1-MeHis, SDMA;Pyrazole-1-Ala,Put;Ile,Pyrazole-1-Ala;Pyrazole-1-Ala,M CSO1/MCSO2;1-MeHis,Kyn;1-MeHis,Kyn/Trp;Tyr,Pyrazole-1-Ala;V al,Pyrazole-1-Ala;Pyrazole-1-Ala,Cystathionine;Pyrazole-1-A la,Kyn/Trp;aABA,Pyrazole-1-Ala;Pro,Pyrazole-1-Ala;Cit,Pyraz ole-1-Ala;Pyrazole-1-Ala,MeCys;Pyrazole-1-Ala,Kyn;Gln,Pyraz ole-1-Ala;Taurine,Opthalmic acid;Pyrazole-1-Ala,bAiBA;Pyraz ole-1-Ala,MCSO2;Ser,Pyrazole-1-Ala;Pyrazole-1-Ala,Kyn/BCAA; Leu,Pyrazole-1-Ala;Pyrazole-1-Ala,SDMA;Taurine,Cysteic aci d;Glu,Pyrazole-1-Ala;Pyrazole-1-Ala,bABA;Asn,Pyrazole-1-Al a;Arg,Pyrazole-1-Ala;Taurine,Pipecolic acid;Trp,Pyrazole-1-Ala;Phe,Pyrazole-1-Ala;Met,Pyrazole-1-Ala;Taurine,Sar;Pyraz ole-1-Ala,Hypro;1-MeHis,3-MeHis;Gly,Pyrazole-1-Ala;Ala,Pyra zole-1-Ala;ADMA,Pyrazole-1-Ala;3-MeHis,Pyrazole-1-Ala;Orn,P yrazole-1-Ala;Pyrazole-1-Ala,MCSO1;aAAA,Pyrazole-1-Ala;1-Me His,Cit;1-MeHis,hArg;Pyrazole-1-Ala,Hypotaurine;Pyrazole-1-Ala,N6-AcLys;1-MeHis,His;Pyrazole-1-Ala,Cysteic acid;1-MeHi s,MCSO2;1-MeHis,Put;1-MeHis,GABA;Pyrazole-1-Ala,Sar;Pyrazol e-1-Ala,Pipecolic acid;1-MeHis,N8-AcSpd;1-MeHis,Cystathioni ne;Lys,1-MeHis;1-MeHis,N6-AcLys;1-MeHis,MCSO2/MeCys;1-MeHis, Thioproline;Pyrazole-1-Ala,MCSO2/MeCys;1-MeHis,MCSO1/MCSO2; Leu,1-MeHis;Asn,1-MeHis;1-MeHis,ADMA;1-MeHis,Ile;Tyr,1-MeHi s;1-MeHis,Hypro;1-MeHis,Val;Gln,1-MeHis;1-MeHis,Trp;Arg,1-M eHis;aABA,1-MeHis;Ser,1-MeHis;1-MeHis,Met;1-MeHis,bAiBA;Phe, 1-MeHis

In particular, it was demonstrated that the frequencies of occurrence of the following combinations of two kinds of amino acid/amino acid related metabolite explanatory variables were as high as 20,000 times or more. Taurine,Kyn/BCAA;Taurine,SDMA;Taurine,Kyn;Taurine,Kyn/Trp;T aurine,3-MeHis;Taurine,Cit;Taurine,Thioproline;Taurine,hAr g;Taurine,GABA;Taurine,Put;Taurine,His;Taurine,Cystathionin e;Taurine,Leu;Taurine,Ile;Taurine,MCSO1/MCSO2;Taurine,Val;A sn,Taurine;Taurine,N8-AcSpd;Taurine,Tyr;Taurine,Lys;Taurine, aABA;Gln,Taurine;Ser,Taurine;Taurine,Trp;Taurine,Arg;Taurin e,Hypro;Taurine,MCSO2;Taurine,ADMA;Taurine,Met;Taurine,bAiB A;Taurine,bABA;Taurine,Phe;Taurine,Ala;Gly,Taurine;Taurine, Orn;Taurine,Thr;Taurine,Pro;Taurine,MCSO1;Taurine,N6-AcLys; Taurine,MeCys;Glu,Taurine;1-MeHis,Pyrazole-1-Ala;Taurine,aA AA;1-MeHis,Spd;Pyrazole-1-Ala,Spd;Taurine,Hypotaurine;Tauri ne,MCSO2/MeCys

It was further demonstrated that the frequency of occurrence of the combination of Taurine and Pyrazole-1-Ala, the frequency of occurrence of the combination of Taurine and 1-MeHis, and the frequency of occurrence of the combination of Taurine and Spd were as high as 60,000 times or more.

### Example 6

The serum samples of MCI people aged 60 years old or older with clinical diagnosis of MCI (MCI group: 120 subjects) and healthy elderly people aged 60 years old or older presumably with normal cognitive function (healthy group: 120 subjects) were measured by the modified BCP method to determine the blood concentration of albumin.

The ability to discriminate between the MCI group and the healthy group was evaluated by ROC_AUC using data of the serum concentration of albumin (g/dL), and ROC_AUC was 0.615. The concentration of albumin is presumed to be valuable for evaluating the state of MCI in consideration of a healthy state.

### Example 7

The plasma samples and the serum samples of MCI people aged 60 years old or older with clinical diagnosis of MCI (MCI group: 120 subjects) and healthy elderly people aged 60 years old or older presumably with normal cognitive function (healthy group: 120 subjects) were used. The blood concentrations of amino acids in the plasma samples were measured by the amino acid analyzing method (A), and the blood concentration of albumin in the serum samples was measured by the modified BCP method.

A multivariate discriminant (multivariate function) for discriminating between the two groups, namely, the MCI group and the healthy group, including an explanatory variable to be substituted with the serum concentration of albumin and an explanatory variable to be substituted with the plasma concentration of an amino acid was found.

A logistic regression expression was used as the multivariate discriminant. The serum concentration of albumin (g/dL) and the plasma concentrations (nmol/ml) of 19 kinds of amino acids (Ala, Arg, Asn, Cit, Gln, Gly, His, Ile, Leu, Lys, Met, Orn, Phe, Pro, Ser, Thr, Trp, Tyr, Val) were searched for a combination of two explanatory variables to be included in the logistic regression expression (where the explanatory variable of albumin (Alb) was essential), and a search for logistic regression expressions having a satisfactory ability to discriminate between the MCI group and the healthy group was conducted.

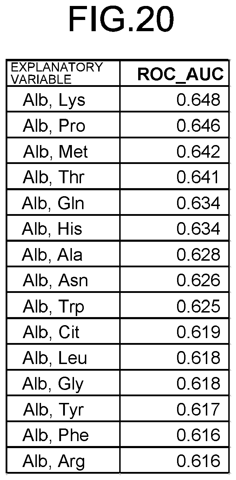

FIG. 20 shows a list of logistic regression expressions with two explanatory variables, in which ROC_AUC for the MCI group and the healthy group is equal to or greater than 0.615 described in Example 6. These logistic regression expressions are presumed to be valuable in the evaluation because ROC_AUC is high.

### Example 8

The sample data used in Example 7 was used. A multivariate discriminant (multivariate function) for discriminating between the two groups, namely, the MCI group and the healthy group, including an explanatory variable to be substituted with the serum concentration of albumin and explanatory variables to be substituted with the plasma concentrations of amino acids was found.

A logistic regression expression was used as the multivariate discriminant. The serum concentration of albumin (g/dL) and the plasma concentrations of the 19 kinds of amino acids (nmol/ml) were searched for a combination of three explanatory variables to be included in the logistic regression expression (where the explanatory variable of albumin (Alb) was essential) in the same manner as in Example 7, and a search for logistic regression expressions having a satisfactory ability to discriminate between the MCI group and the healthy group was conducted.

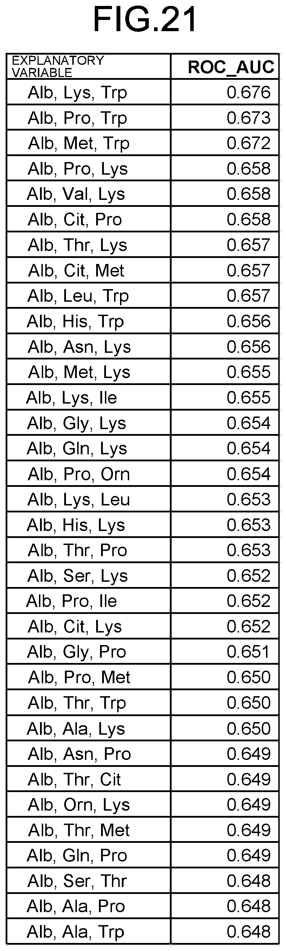

FIG. 21 shows a list of logistic regression expressions with three explanatory variables, in which ROC_AUC for the MCI group and the healthy group is equal to or greater than 0.648 shown in FIG. 20. These logistic regression expressions are presumed to be valuable in the evaluation because ROC_AUC is high.

### Example 9

The sample data used in Example 7 was used. A multivariate discriminant (multivariate function) for discriminating between the two groups, namely, the MCI group and the healthy group, including an explanatory variable to be substituted with the serum concentration of albumin and explanatory variables to be substituted with the plasma concentrations of amino acids was found.

A logistic regression expression was used as the multivariate discriminant. The serum concentration of albumin (g/dL) and the plasma concentrations of the 19 kinds of amino acids (nmol/ml) were searched for a combination of six explanatory variables to be included in the logistic regression expression (where the explanatory variable of albumin (Alb) was essential) in the same manner as in Example 7, and a search for logistic regression expressions having a satisfactory ability to discriminate between the MCI group and the healthy group was conducted.

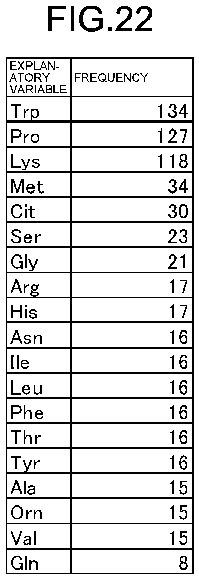

Among the obtained logistic regression expressions (11,628 expressions (=₁₉C₅)), the frequencies of occurrence of amino acid explanatory variables included in 135 expressions with ROC_AUC equal to or greater than 0.70 were calculated and shown in FIG. 22.

This demonstrated that the frequencies of occurrence of Trp, Pro, Lys, Met, Cit, Ser, and Gly were as high as 20 times or more. In particular, it was demonstrated that the frequencies of occurrence of Met and Cit were as high as 30 times or more. It was also demonstrated that the frequencies of occurrence of Trp, Pro, and Lys were as high as 100 times or more.

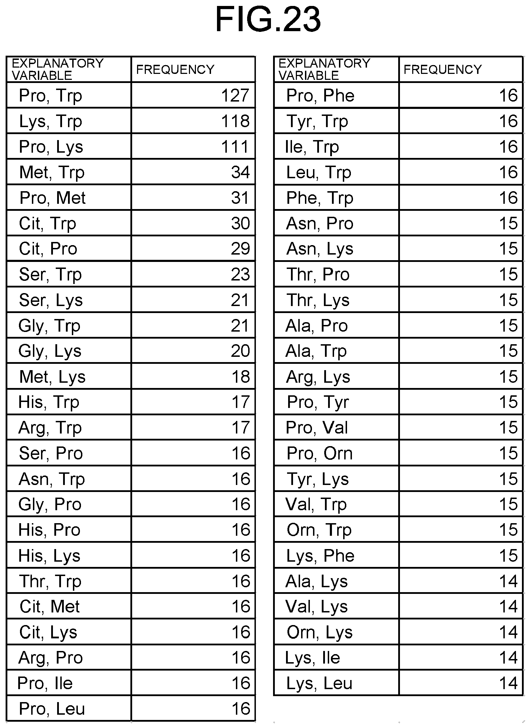

The frequencies of occurrence of combinations of 2 kinds of amino acid explanatory variables included in the 135 expressions were calculated and shown in FIG. 23.

This demonstrated that the frequencies of occurrence of the following combinations of 2 kinds of amino acid explanatory variables were as high as 10 times or more. Pro,Trp;Lys,Trp;Pro,Lys;Met,Trp;Pro,Met;Cit,Trp;Cit,Pro;Ser ,Trp;Ser,Lys;Gly,Trp;Gly,Lys;Met,Lys;His,Trp;Arg,Trp;Ser,Pr o;Asn,Trp;Gly,Pro;His,Pro;His,Lys;Thr,Trp;Cit,Met;Cit,Lys;A rg,Pro;Pro,Ile;Pro,Leu;Pro,Phe;Tyr,Trp;Ile,Trp;Leu,Trp;Phe, Trp;Asn,Pro;Asn,Lys;Thr,Pro;Thr,Lys;Ala,Pro;Ala,Trp;Arg,Lys ;Pro,Tyr;Pro,Val;Pro,Orn;Tyr,Lys;Val,Trp;Orn,Trp;Lys,Phe;Al a,Lys;Val,Lys;Orn,Lys;Lys,Ile;Lys,Leu

In particular, it was demonstrated that the frequencies of occurrence of the following combinations of 2 kinds of amino acid explanatory variables were as high as 20 times or more.
Met,Trp;Pro,Met;Cit,Trp;Cit,Pro;Ser,Trp;Ser,Lys;Gly,Trp;Gly ,Lys

It was demonstrated that the frequency of occurrence of the combination of Pro and Trp, the frequency of occurrence of the combination of Lys and Trp, and the frequency of occurrence of the combination of Pro and Lys were as high as 100 times or more.

For the top 100 expressions of ROC_AUC among the obtained logistic regression expressions (11,628 expressions), the explanatory variables included in each expression and ROC_AUC are listed below. These logistic regression expressions are presumed to be valuable in the evaluation because ROC_AUC is high.
Alb, Ser, Cit, Pro, Met, Trp, 0.717; Alb, Cit, Pro, Met, L ys, Trp, 0.716; Alb, Arg, Pro, Met, Lys, Trp, 0.712; Alb, His, Cit, Pro, Met, Trp, 0.710; Alb, Cit, Pro, Met, Phe, Trp, 0.710; Alb, Cit, Pro, Met, Leu, Trp, 0.710; Alb, Ser, Gly, Pro, Lys, Trp, 0.709; Alb, Cit, Pro, Met, Orn, Trp, 0.709; Alb, Cit, Pro, Lys, Phe, Trp, 0.709; Alb, Cit, Pro, Val, Met, Trp, 0.709; Alb, Cit, Arg, Pro, Met, Trp, 0.70 8; Alb, Asn, Cit, Pro, Met, Trp, 0.708; Alb, Gly, Cit, Pr o, Met, Trp, 0.708; Alb, Thr, Cit, Pro, Met, Trp, 0.708; Alb, Cit, Pro, Met, Ile, Trp, 0.708; Alb, Ser, Pro, Met, L ys, Trp, 0.708; Alb, Gln, Cit, Pro, Met, Trp, 0.708; Alb, Ala, Cit, Pro, Met, Trp, 0.708; Alb, Cit, Pro, Tyr, Met, Trp, 0.708; Alb, Pro, Met, Orn, Lys, Trp, 0.707; Alb, His, Cit, Pro, Lys, Trp, 0.707; Alb, Thr, Cit, Pro, Lys, Trp, 0.707; Alb, Gln, Pro, Met, Lys, Trp, 0.706; Alb, Gly, Pro, Lys, Phe, Trp, 0.705; Alb, Pro, Tyr, Met, Lys, Trp, 0.70 5; Alb, Pro, Met, Lys, Ile, Trp, 0.705; Alb, Ser, Asn, Gl y, Lys, Trp, 0.705; Alb, Ser, Asn, Pro, Lys, Trp, 0.705; Alb, Pro, Met, Lys, Leu, Trp, 0.705; Alb, Gly, Arg, Pro, L ys, Trp, 0.705; Alb, Gly, Pro, Met, Lys, Trp, 0.705; Alb, Cit, Pro, Lys, Leu, Trp, 0.705; Alb, Gly, Cit, Pro, Lys, Trp, 0.705; Alb, Thr, Pro, Met, Lys, Trp, 0.705; Alb, Cit, Pro, Tyr, Lys, Trp, 0.705; Alb, Pro, Lys, Ile, Leu, Trp, 0.705; Alb, Pro, Val, Met, Lys, Trp, 0.705; Alb, Ser, Gly, Thr, Lys, Trp, 0.705; Alb, Pro, Lys, Ile, Phe, Trp, 0.70 5; Alb, Ala, Cit, Pro, Lys, Trp, 0.704; Alb, Ala, Pro, Me t, Lys, Trp, 0.704; Alb, Ser, His, Pro, Lys, Trp, 0.704; Alb, Asn, Cit, Pro, Lys, Trp, 0.704; Alb, Gly, Pro, Orn, L ys, Trp, 0.704; Alb, Cit, Arg, Pro, Lys, Trp, 0.704; Alb, Ser, Cit, Met, Lys, Trp, 0.704; Alb, Thr, Arg, Pro, Lys, Trp, 0.704; Alb, Cit, Pro, Orn, Lys, Trp, 0.704; Alb, His, Pro, Val, Lys, Trp, 0.704; Alb, Ser, Arg, Pro, Lys, Trp, 0.704; Alb, His, Pro, Lys, Leu, Trp, 0.704; Alb, Ala, Pro, Orn, Lys, Trp, 0.704; Alb, Ser, Pro, Lys, Phe, Trp, 0.70 4; Alb, Cit, Pro, Lys, Ile, Trp, 0.704; Alb, His, Thr, Pr o, Lys, Trp, 0.704; Alb, Thr, Pro, Lys, Ile, Trp, 0.704; Alb, Pro, Val, Orn, Lys, Trp, 0.704; Alb, Pro, Orn, Lys, I le, Trp, 0.704; Alb, Pro, Orn, Lys, Phe, Trp, 0.704; Alb, Ser, Cit, Pro, Lys, Trp, 0.704; Alb, Cit, Pro, Val, Lys, Trp, 0.704; Alb, Pro, Orn, Lys, Leu, Trp, 0.704; Alb, His, Pro, Met, Lys, Trp, 0.704; Alb, His, Pro, Orn, Lys, Trp, 0.704; Alb, Thr, Pro, Tyr, Lys, Trp, 0.704; Alb, Pro, Met, Lys, Phe, Trp, 0.703; Alb, Gly, His, Pro, Lys, Trp, 0.70 3; Alb, Gln, Thr, Pro, Lys, Trp, 0.703; Alb, His, Pro, Ly s, Ile, Trp, 0.703; Alb, Thr, Pro, Val, Lys, Trp, 0.703; Alb, Ala, Pro, Lys, Ile, Trp, 0.703; Alb, Gly, Pro, Lys, I le, Trp, 0.703; Alb, Ser, Pro, Orn, Lys, Trp, 0.703; Alb, Asn, Pro, Orn, Lys, Trp, 0.703; Alb, His, Ala, Pro, Lys, Trp, 0.703; Alb, Arg, Pro, Lys, Phe, Trp, 0.703; Alb, Gly, Pro, Lys, Leu, Trp, 0.703; Alb, Asn, Arg, Pro, Lys, Trp, 0.703; Alb, Gly, Thr, Pro, Lys, Trp, 0.703; Alb, His, Pro, Tyr, Lys, Trp, 0.703; Alb, Thr, Pro, Orn, Lys, Trp, 0.70 3; Alb, Ser, Ala, Pro, Lys, Trp, 0.703; Alb, Ser, Pro, Ly s, Ile, Trp, 0.703; Alb, Pro, Tyr, Lys, Phe, Trp, 0.703; Alb, Thr, Ala, Pro, Lys, Trp, 0.703; Alb, Thr, Pro, Lys, P he, Trp, 0.703; Alb, Asn, Pro, Met, Lys, Trp, 0.702; Alb, Pro, Val, Lys, Phe, Trp, 0.702; Alb, His, Pro, Lys, Phe, Trp, 0.702; Alb, Ala, Pro, Tyr, Lys, Trp, 0.702; Alb, Pro, Lys, Leu, Phe, Trp, 0.702; Alb, Ser, Thr, Pro, Lys, Trp, 0.702; Alb, Arg, Pro, Orn, Lys, Trp, 0.702; Alb, Ser, Gly, Met, Lys, Trp, 0.702; Alb, Ser, Arg, Pro, Met, Trp, 0.70 2; Alb, Ser, Pro, Val, Lys, Trp, 0.702; Alb, Gln, Cit, Pr o, Lys, Trp, 0.702; Alb, Ser, Gly, His, Lys, Trp, 0.702; Alb, Gly, Pro, Tyr, Lys, Trp, 0.702; Alb, Gly, Pro, Val, L ys, Trp, 0.702;

### Example 10

The serum samples of MCI people aged 60 years old or older with clinical diagnosis of MCI (MCI group: 147 subjects) and healthy elderly people aged 60 years old or older presumably with normal cognitive function (healthy group: 38 subjects) were measured by CLEIA to determine the blood concentration of folate.

The ability to discriminate between the MCI group and the healthy group was evaluated by ROC_AUC using data of the serum concentration of folate (ng/mL), and ROC_AUC was 0.679. The folate concentration is presumed to be valuable for evaluating the state of MCI in consideration of a healthy state.

### Example 11

The plasma samples and the serum samples of MCI people aged 60 years old or older with clinical diagnosis of MCI (MCI group: 147 subjects) and healthy elderly people aged 60 years old or older presumably with normal cognitive function (healthy group: 38 subjects) were used. The blood concentrations of amino acids in the plasma samples were measured by the amino acid analyzing method (A), the blood concentration of folate in the serum samples was measured by CLEIA, and the blood concentration of albumin in the serum samples was measured by the modified BCP method.

A multivariate discriminant (multivariate function) for discriminating between the two groups, namely, the MCI group and the healthy group, including an explanatory variable to be substituted with the serum concentration of folate and an explanatory variable to be substituted with the serum concentration of albumin or the plasma concentration of an amino acid was found.

A logistic regression expression was used as the multivariate discriminant. The serum concentration of folate (ng/mL), the serum concentration of albumin (g/dL), and the plasma concentrations of the 19 kinds of amino acids (nmol/ml) were searched for a combination of two explanatory variables to be included in the logistic regression expression (where the explanatory variable of folate (Folate) was essential), and a search for logistic regression expressions having a satisfactory ability to discriminate between the MCI group and the healthy group was conducted.

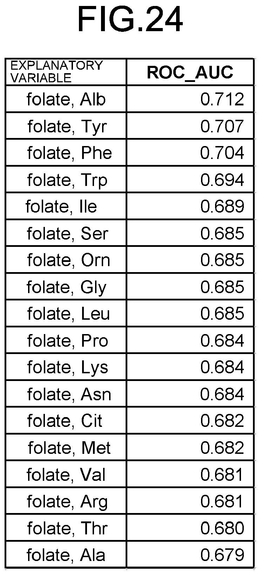

FIG. 24 shows a list of logistic regression expressions with two explanatory variables, in which ROC_AUC for the MCI group and the healthy group is equal to or greater than 0.679 described in Example 10. These logistic regression expressions are presumed to be valuable in the evaluation because ROC_AUC is high.

### Example 12

The sample data used in Example 11 was used. A multivariate discriminant (multivariate function) for discriminating between the two groups, namely, the MCI group and the healthy group, including an explanatory variable to be substituted with the serum concentration of folate and an explanatory variable to be substituted with the serum concentration of albumin or the plasma concentration of an amino acid was found.

A logistic regression expression was used as the multivariate discriminant. The serum concentration of folate, the serum concentration of albumin, and the plasma concentrations of the 19 kinds of amino acids were searched for a combination of three explanatory variables to be included in the logistic regression expression (where the explanatory variable of folate was essential) in the same manner as in Example 11, and a search for logistic regression expressions having a satisfactory ability to discriminate between the MCI group and the healthy group was conducted.

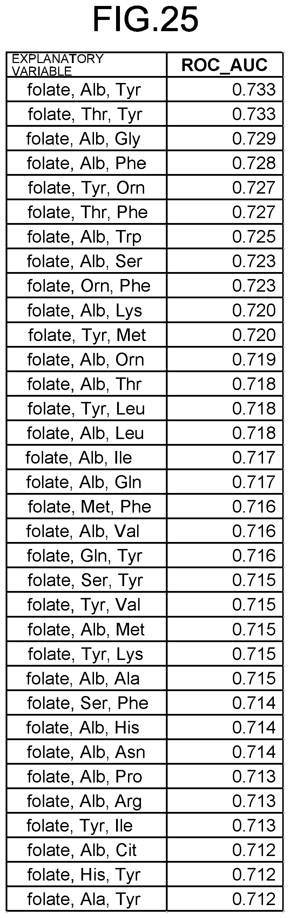

FIG. 25 shows a list of logistic regression expressions with three explanatory variables, in which ROC_AUC for the MCI group and the healthy group is equal to or greater than 0.712 shown in FIG. 24. These logistic regression expressions are presumed to be valuable in the evaluation because ROC_AUC is high.

### Example 13

The sample data used in Example 11 was used. A multivariate discriminant (multivariate function) for discriminating between the two groups, namely, the MCI group and the healthy group, including an explanatory variable to be substituted with the serum concentration of folate and an explanatory variable to be substituted with the serum concentration of albumin or the plasma concentration of an amino acid was found.

A logistic regression expression was used as the multivariate discriminant. The serum concentration of folate, the serum concentration of albumin, and the plasma concentrations of the 19 kinds of amino acids were searched for a combination of six explanatory variables to be included in the logistic regression expression (where the explanatory variable of folate was essential) in the same manner as in Example 11, and a search for logistic regression expressions having a satisfactory ability to discriminate between the MCI group and the healthy group was conducted.

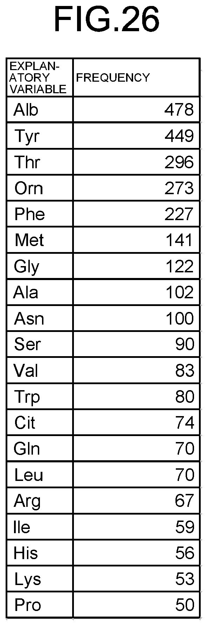

Among the obtained logistic regression expressions (15,504 expressions (=₂₀C₅)), the frequencies of occurrence of amino acid/albumin explanatory variables included in 588 expressions with ROC_AUC equal to or greater than 0.75 were calculated and shown in FIG. 26.

This demonstrated that the frequencies of occurrence of Alb, Tyr, Thr, Orn, Phe, Met, Gly, Ala, and Asn were as high as 100 times or more. In particular, it was demonstrated that the frequencies of occurrence of Thr, Orn, and Phe were as high as 200 times or more. It was also demonstrated that the frequencies of occurrence of Alb and Tyr were as high as 100 times or more.

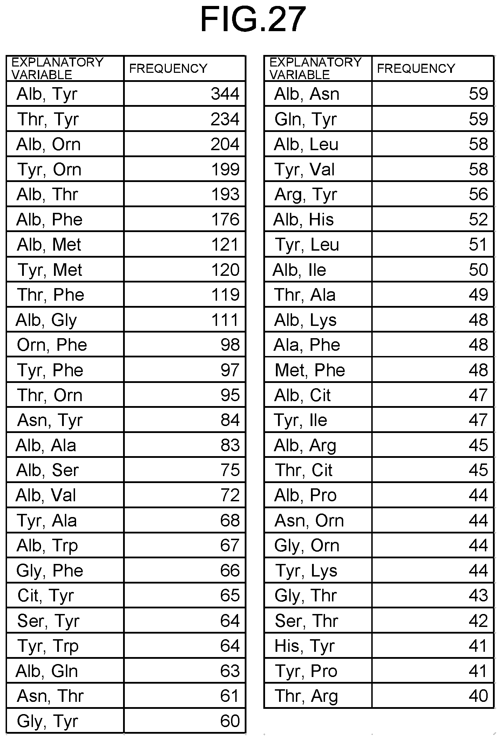

The frequencies of occurrence of combinations of 2 kinds of amino acid/albumin explanatory variables included in the 588 expressions were calculated and shown in FIG. 27.

This demonstrated that the frequencies of occurrence of the following combinations of 2 kinds of amino acid/albumin explanatory variables were as high as 40 times or more. Alb,Tyr;Thr,Tyr;Alb,Orn;Tyr,Orn;Alb,Thr;Alb,Phe;Alb,Met;Tyr ,Met;Thr,Phe;Alb,Gly;Orn,Phe;Tyr,Phe;Thr,Orn;Asn,Tyr;Alb,Al a;Alb,Ser;Alb,Val;Tyr,Ala;Alb,Trp;Gly,Phe;Cit,Tyr;Ser,Tyr;T yr,Trp;Alb,Gln;Asn,Thr;Gly,Tyr;Alb,Asn;Gln,Tyr;Alb,Leu;Tyr, Val;Arg,Tyr;Alb,His;Tyr,Leu;Alb,Ile;Thr,Ala;Alb,Lys;Ala,Phe ;Met,Phe;Alb,Cit;Tyr,Ile;Alb,Arg;Thr,Cit;Alb,Pro;Asn,Orn;Gl y,Orn;Tyr,Lys;Gly,Thr;Ser,Thr;His,Tyr;Tyr,Pro;Thr,Arg

In particular, it was demonstrated that the frequencies of occurrence of the following combinations of 2 kinds of amino acid/albumin explanatory variables were as high as 100 times or more.
Tyr,Orn;Alb,Thr;Alb,Phe;Alb,Met;Tyr,Met;Thr,Phe;Alb,Gly

It was demonstrated the frequency of occurrence of the combination of Alb and Tyr, the frequency of occurrence of the combination of Thr and Tyr, and the frequency of occurrence of the combination of Alb and Orn were as high as 200 times or more.

For the top 100 expressions of ROC_AUC among the obtained logistic regression expressions (15,504 expressions), the explanatory variables included in each expression and ROC_AUC are listed below. These logistic regression expressions are presumed to be valuable in the evaluation because ROC_AUC is high.
folate, Alb, Thr, Tyr, Orn, Phe, 0.779; folate, Alb, Asn, T hr, Tyr, Orn, 0.774; folate, Alb, Thr, Cit, Tyr, Orn, 0.77 4; folate, Alb, Gly, Thr, Tyr, Orn, 0.774; folate, Alb, Thr, Ala, Tyr, Orn, 0.772; folate, Alb, Asn, Tyr, Met, Orn, 0.7 72; folate, Alb, Ser, Thr, Tyr, Orn, 0.772; folate, Alb, Th r, Tyr, Orn, Lys, 0.771; folate, Alb, Thr, Pro, Tyr, Orn, 0. 771; folate, Alb, Gln, Thr, Tyr, Orn, 0.771; folate, Alb, A la, Tyr, Met, Orn, 0.771; folate, Alb, Cit, Tyr, Met, Orn, 0.771; folate, Alb, Thr, Tyr, Met, Orn, 0.771; folate, Alb, Ser, Ala, Tyr, Orn, 0.770; folate, Alb, Thr, Tyr, Orn, Ile, 0.770; folate, Alb, Thr, Arg, Tyr, Orn, 0.770; folate, Asn, Thr, Cit, Tyr, Orn, 0.770; folate, Alb, Thr, Tyr, Orn, Trp, 0.769; folate, Alb, Gly, Tyr, Met, Orn, 0.769; folate, Alb, Tyr, Met, Orn, Phe, 0.769; folate, Alb, Gly, Thr, Orn, Phe, 0.769; folate, Alb, Tyr, Met, Orn, Ile, 0.769; folate, Alb, Ser, Tyr, Met, Orn, 0.769; folate, Alb, Tyr, Val, Met, Orn, 0.768; folate, Alb, Thr, Tyr, Orn, Leu, 0.767; folate, Alb, Thr, Tyr, Val, Orn, 0.767; folate, Alb, Tyr, Met, Orn, Lys, 0.767; folate, Alb, Gly, Thr, Tyr, Phe, 0.767; folate, Alb, Thr, Tyr, Met, Phe, 0.766; folate, Thr, Cit, Tyr, Orn, Phe, 0.766; folate, Alb, Tyr, Met, Orn, Leu, 0.766; folate, Alb, His, Tyr, Met, Orn, 0.766; folate, Alb, Pro, Tyr, Met, Orn, 0.766; folate, Alb, Gln, Tyr, Met, Orn, 0.766; folate, Alb, His, Thr, Tyr, Orn, 0.766; folate, Alb, Gly, Tyr, Val, Orn, 0.765; folate, Alb, Gly, Val, Orn, Phe, 0.765; folate, Alb, Asn, Gly, Thr, Tyr, 0.765; folate, Alb, Arg, Tyr, Met, Orn, 0.765; folate, Alb, Gln, Thr, Tyr, Phe, 0.764; folate, Alb, Asn, Thr, Tyr, Met, 0.764; folate, Alb, Gly, Tyr, Orn, Leu, 0.764; folate, Alb, Gln, Ala, Tyr, Orn, 0.764; folate, Alb, Ala, Pro, Tyr, Orn, 0.764; folate, Alb, Asn, Gln, Thr, Tyr, 0.764; folate, Alb, Gly, Ala, Tyr, Orn, 0.764; folate, Alb, Gly, Orn, Leu, Phe, 0.764; folate, Alb, Ser, Thr, Orn, Phe, 0.764; folate, Thr, Arg, Tyr, Orn, Phe, 0.764; folate, Alb, Ser, Gln, Tyr, Orn, 0.764; folate, Alb, Thr, Ala, Tyr, Trp, 0.764; folate, Alb, Ser, Tyr, Val, Orn, 0.763; folate, Alb, Gly, Tyr, Orn, Lys, 0.763; folate, Alb, Gly, Cit, Tyr, Orn, 0.763; folate, Alb, Ala, Cit, Tyr, Orn, 0.763; folate, Alb, Asn, Thr, Tyr, Trp, 0.763; folate, Alb, Thr, Tyr, Leu, Phe, 0.763; folate, Alb, Ala, Tyr, Val, Orn, 0.763; folate, Alb, Ala, Tyr, Orn, Ile, 0.763; folate, Ser, Asn, Thr, Tyr, Orn, 0.763; folate, Asn, Thr, Tyr, Orn, Phe, 0.763; folate, Alb, Ala, Tyr, Orn, Lys, 0.762; folate, Alb, Ser, Gly, Tyr, Orn, 0.762; folate, Alb, Gly, Ala, Orn, Phe, 0.762; folate, Alb, Thr, Tyr, Met, Trp, 0.762; folate, Alb, Ala, Arg, Tyr, Orn, 0.762; folate, Alb, Ser, Asn, Tyr, Orn, 0.762; folate, Alb, Gly, Met, Orn, Phe, 0.762; folate, Alb, Ala, Tyr, Orn, Leu, 0.762; folate, Alb, Asn, Thr, Tyr, Phe, 0.762; folate, Alb, Gly, Tyr, Orn, Phe, 0.762; folate, Alb, Ser, Thr, Tyr, Phe, 0.762; folate, Alb, Ser, Tyr, Orn, Leu, 0.762; folate, Alb, Gly, His, Orn, Phe, 0.762; folate, Alb, Gly, Thr, Arg, Tyr, 0.762; folate, Alb, Gln, Pro, Tyr, Orn, 0.762; folate, Alb, Thr, Cit, Tyr, Phe, 0.762; folate, Alb, Thr, Tyr, Val, Trp, 0.762; folate, Alb, Ser, Tyr, Orn, Lys, 0.761; folate, Alb, Ser, Tyr, Orn, Phe, 0.761; folate, Alb, Asn, Tyr, Met, Phe, 0.761; folate, Alb, Gly, Thr, Leu, Phe, 0.761; folate, Alb, Thr, Ala, Tyr, Phe, 0.761; folate, Alb, Thr, Tyr, Leu, Trp, 0.761; folate, Alb, Ala, Tyr, Orn, Phe, 0.761; folate, Alb, Gly, Thr, Val, Phe, 0.761; folate, Alb, His, Ala, Tyr, Orn, 0.761; folate, Alb, Thr, Tyr, Ile, Trp, 0.761; folate, Alb, Ser, Cit, Tyr, Orn, 0.761; folate, Alb, Asn, Thr, Orn, Phe, 0.761; folate, Alb, His, Tyr, Orn, Phe, 0.761; folate, Alb, Gly, Tyr, Orn, Ile, 0.761; folate, Alb, Thr, Ala, Orn, Phe, 0.761; folate, Alb, Thr, Cit, Orn, Phe, 0.761; folate, Alb, Asn, Gly, Orn, Phe, 0.761; folate, Alb, Gln, Tyr, Val, Orn, 0.761; folate, Alb, Thr, Tyr, Lys, Phe, 0.761; folate, Alb, Gly, Gln, Thr, Tyr, 0.761; folate, Alb, Gly, Gln, Tyr, Orn, 0.761; folate, Alb, Gln, Thr, Cit, Tyr, 0.761

### Example 14

The whole blood samples of MCI people aged 60 years old or older with clinical diagnosis of MCI (MCI group: 120 subjects) and healthy elderly people aged 60 years old or older presumably with normal cognitive function (healthy group: 87 subjects) were measured by flow cytometry using semiconductor laser to determine the leukocyte count in blood.

The ability to discriminate between the MCI group and the healthy group was evaluated by ROC_AUC using data of the white cell count (/µL) in whole blood, and ROC_AUC was 0.580. The white cell count is presumed to be valuable for evaluating the state of MCI in consideration of a healthy state.

### Example 15

The plasma samples, the whole blood samples, and the serum samples of MCI people aged 60 years old or older with clinical diagnosis of MCI (MCI group: 120 subjects) and healthy elderly people aged 60 years old or older presumably with normal cognitive function (healthy group: 87 subjects) were used. The blood concentrations of amino acids in the plasma samples were measured by the amino acid analyzing method (A), the white cell count in blood in the whole blood samples was measured by flow cytometry using semiconductor laser, and the blood concentration of albumin in the serum samples was measured by the modified BCP method.

A multivariate discriminant (multivariate function) for discriminating between the two groups, namely, the MCI group and the healthy group, including an explanatory variable to be substituted with the white cell count in whole blood and an explanatory variable to be substituted with the serum concentration of albumin or the plasma concentration of an amino acid was found.

A logistic regression expression was used as the multivariate discriminant. The white cell count (/pL) in blood, the serum concentration of albumin (g/dL), and the plasma concentrations of the 19 kinds of amino acids (nmol/ml) were searched for a combination of two explanatory variables to be included in the logistic regression expression (where the explanatory variable of white cell count in blood (WBC) was essential), and a search for logistic regression expressions having a satisfactory ability to discriminate between the MCI group and the healthy group was conducted.

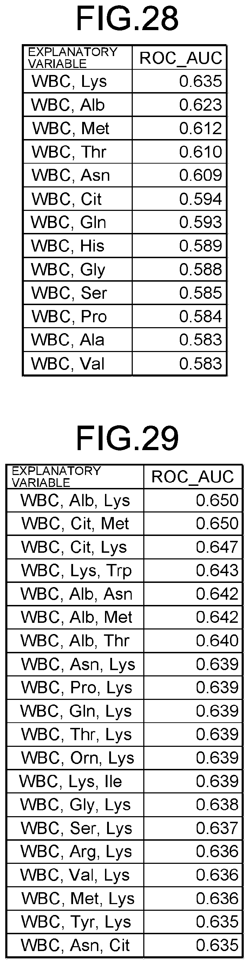

FIG. 28 shows a list of logistic regression expressions with two explanatory variables, in which ROC_AUC for the MCI group and the healthy group is equal to or greater than 0.580 described in Example 14. These logistic regression expressions are presumed to be valuable in the evaluation because ROC_AUC is high.

### Example 16

The sample data used in Example 15 was used. A multivariate discriminant (multivariate function) for discriminating between the two groups, namely, the MCI group and the healthy group, including an explanatory variable to be substituted with the white cell count in whole blood and an explanatory variable to be substituted with the serum concentration of albumin or the plasma concentration of an amino acid was found.

A logistic regression expression was used as the multivariate discriminant. The white cell count in whole blood, the serum concentration of albumin, and the plasma concentrations of the 19 kinds of amino acids were searched for a combination of three explanatory variables to be included in the logistic regression expression (where the explanatory variable of white cell count in blood was essential) in the same manner as in Example 15, and a search for logistic regression expressions having a satisfactory ability to discriminate between the MCI group and the healthy group was conducted.

FIG. 29 shows a list of logistic regression expressions with three explanatory variables, in which ROC_AUC for the MCI group and the healthy group is equal to or greater than 0.635 shown in FIG. 28. These logistic regression expressions are presumed to be valuable in the evaluation because ROC_AUC is high.

### Example 17

The whole blood samples of MCI people aged 60 years old or older with clinical diagnosis of MCI (MCI group: 120 subjects) and healthy elderly people aged 60 years old or older presumably with normal cognitive function (healthy group: 87 subjects) were measured by the sheath flow DC detecting method to determine the red cell count in blood.

The ability to discriminate between the MCI group and the healthy group was evaluated by ROC_AUC using data of the red cell count (×10⁴/µL) in whole blood, and ROC_AUC was 0.562. The red cell count in blood is presumed to be valuable for evaluating the state of MCI in consideration of a healthy state.

### Example 18

The plasma samples, the whole blood samples, and the serum samples of MCI people aged 60 years old or older with clinical diagnosis of MCI (MCI group: 120 subjects) and healthy elderly people aged 60 years old or older presumably with normal cognitive function (healthy group: 87 subjects) were used. The blood concentrations of amino acids in the plasma samples were measured by the amino acid analyzing method (A), the red cell count in blood in the whole blood samples was measured by the sheath flow DC detecting method, and the blood concentration of albumin in the serum samples was measured by the modified BCP method.

A multivariate discriminant (multivariate function) for discriminating between the two groups, namely, the MCI group and the healthy group, including an explanatory variable to be substituted with the red cell count in whole blood and an explanatory variable to be substituted with the serum concentration of albumin or the plasma concentration of an amino acid was found.

A logistic regression expression was used as the multivariate discriminant. The red cell count in blood (×10⁴/µL), the serum concentration of albumin (g/dL), and the plasma concentrations of the 19 kinds of amino acids (nmol/ml) were searched for a combination of two explanatory variables to be included in the logistic regression expression (where the explanatory variable of red cell count in blood (RBC) was essential), and a search for logistic regression expressions having a satisfactory ability to discriminate between the MCI group and the healthy group was conducted.

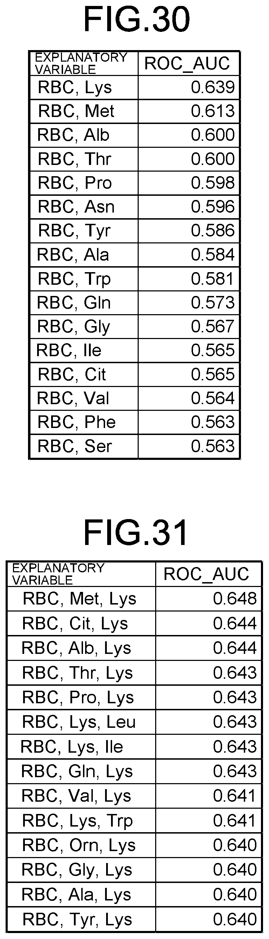

FIG. 30 shows a list of logistic regression expressions with two explanatory variables, in which ROC_AUC for the MCI group and the healthy group is equal to or greater than 0.562 described in Example 17. These logistic regression expressions are presumed to be valuable in the evaluation because ROC_AUC is high.

### Example 19

The sample data used in Example 18 was used. A multivariate discriminant (multivariate function) for discriminating between the two groups, namely, the MCI group and the healthy group, including an explanatory variable to be substituted with the red cell count in whole blood and an explanatory variable to be substituted with the serum concentration of albumin or the plasma concentration of an amino acid was found.

A logistic regression expression was used as the multivariate discriminant. The red cell count in whole blood, the serum concentration of albumin, and the plasma concentrations of the 19 kinds of amino acids were searched for a combination of three explanatory variables to be included in the logistic regression expression (where the explanatory variable of red cell count in blood was essential) in the same manner as in Example 18, and a search for logistic regression expressions having a satisfactory ability to discriminate between the MCI group and the healthy group was conducted.

FIG. 31 shows a list of logistic regression expressions with three explanatory variables, in which ROC_AUC for the MCI group and the healthy group is equal to or greater than 0.639 shown in FIG. 30. These logistic regression expressions are presumed to be valuable in the evaluation because ROC_AUC is high.

### Example 20

The whole blood samples of MCI people aged 60 years old or older with clinical diagnosis of MCI (MCI group: 120 subjects) and healthy elderly people aged 60 years old or older presumably with normal cognitive function (healthy group: 87 subjects) were measured by the SLS-hemoglobin method to determine the hemoglobin content.

The ability to discriminate between the MCI group and the healthy group was evaluated by ROC_AUC using data of the hemoglobin content (g/dL) in whole blood, and ROC_AUC was 0.513. The hemoglobin content is presumed to be valuable for evaluating the state of MCI in consideration of a healthy state.

### Example 21

The plasma samples, the whole blood samples, and the serum samples of MCI people aged 60 years old or older with clinical diagnosis of MCI (MCI group: 120 subjects) and healthy elderly people aged 60 years old or older presumably with normal cognitive function (healthy group: 87 subjects) were used. The blood concentrations of amino acids in the plasma samples were measured by the amino acid analyzing method (A), the hemoglobin content in the whole blood samples was measured by the SLS-hemoglobin method, and the blood concentration of albumin in the serum samples was measured by the modified BCP method.

A multivariate discriminant (multivariate function) for discriminating between the two groups, namely, the MCI group and the healthy group, including an explanatory variable to be substituted with the hemoglobin content in whole blood and an explanatory variable to be substituted with the serum concentration of albumin or the plasma concentration of an amino acid was found.

A logistic regression expression was used as the multivariate discriminant. The hemoglobin content (g/dL), the serum concentration of albumin (g/dL), and the plasma concentrations of the 19 kinds of amino acids (nmol/ml) were searched for a combination of two explanatory variables to be included in the logistic regression expression (where the explanatory variable of hemoglobin content (Hb) was essential), and a search for logistic regression expressions having a satisfactory ability to discriminate between the MCI group and the healthy group was conducted.

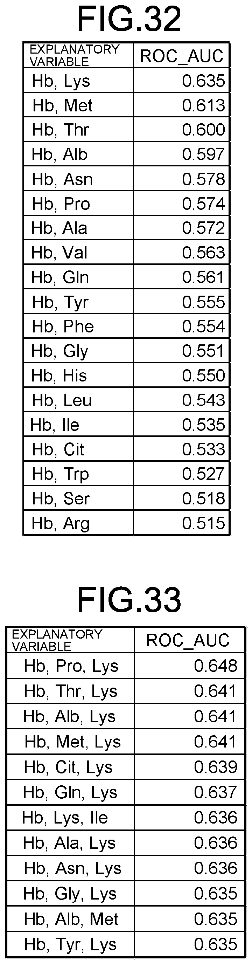

FIG. 32 shows a list of logistic regression expressions with two explanatory variables, in which ROC_AUC for the MCI group and the healthy group is equal to or greater than 0.513 described in Example 20. These logistic regression expressions are presumed to be valuable in the evaluation because ROC_AUC is high.

### Example 22

The sample data used in Example 21 was used. A multivariate discriminant (multivariate function) for discriminating between the two groups, namely, the MCI group and the healthy group, including an explanatory variable to be substituted with the hemoglobin content in whole blood and an explanatory variable to be substituted with the serum concentration of albumin or the plasma concentration of an amino acid was found.

A logistic regression expression was used as the multivariate discriminant. The hemoglobin content in whole blood, the serum concentration of albumin, and the plasma concentrations of the 19 kinds of amino acids were searched for a combination of three explanatory variables to be included in the logistic regression expression (where the explanatory variable of hemoglobin content was essential) in the same manner as in Example 21, and a search for logistic regression expressions having a satisfactory ability to discriminate between the MCI group and the healthy group was conducted.

FIG. 33 shows a list of logistic regression expressions with three explanatory variables, in which ROC_AUC for the MCI group and the healthy group is equal to or greater than 0.635 shown in FIG. 32. These logistic regression expressions are presumed to be valuable in the evaluation because ROC_AUC is high.

### Example 23

The whole blood samples of MCI people aged 60 years old or older with clnical diagnosis of MCI (MCI group: 120 subjects) and healthy elderly people aged 60 years old or older presumably with normal cognitive function (healthy group: 87 subjects) were measured by the red blood cell pulse peak value detecting method to determine the hematocrit.

The ability to discriminate between the MCI group and the healthy group was evaluated by ROC_AUC using data of the hematocrit (%) in whole blood, and ROC_AUC was 0.555. The hematocrit is presumed to be valuable for evaluating the state of MCI in consideration of a healthy state.

### Example 24

The plasma samples, the whole blood samples, and the serum samples of MCI people aged 60 years old or older with clinical diagnosis of MCI (MCI group: 120 subjects) and healthy elderly people aged 60 years old or older presumably with normal cognitive function (healthy group: 87 subjects) were used. The blood concentrations of amino acids in the plasma samples were determined by the amino acid analyzing method (A), the hematocrit in the whole blood samples was measured by the red blood cell pulse peak value detecting method, and the blood concentration of albumin in the serum samples was measured by the modified BCP method.

A multivariate discriminant (multivariate function) for discriminating between the two groups, namely, the MCI group and the healthy group, including an explanatory variable to be substituted with the hematocrit in whole blood and an explanatory variable to be substituted with the serum concentration of albumin or the plasma concentration of an amino acid was found.

A logistic regression expression was used as the multivariate discriminant. The hematocrit (%), the serum concentration of albumin (g/dL), and the plasma concentrations of the 19 kinds of amino acids (nmol/ml) were searched for a combination of two explanatory variables to be included in the logistic regression expression (where the explanatory variable of hematocrit (Ht) was essential), and a search for logistic regression expressions having a satisfactory ability to discriminate between the MCI group and the healthy group was conducted.

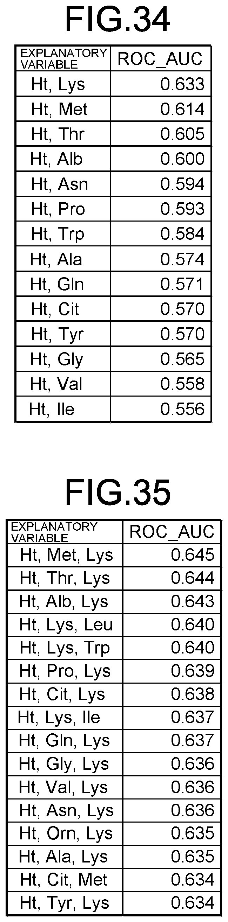

FIG. 34 shows a list of logistic regression expressions with two explanatory variables, in which ROC_AUC for the MCI group and the healthy group is equal to or greater than 0.555 described in Example 23. These logistic regression expressions are presumed to be valuable in the evaluation because ROC_AUC is high.

### Example 25

The sample data used in Example 24 was used. A multivariate discriminant (multivariate function) for discriminating between the two groups, namely, the MCI group and the healthy group, including an explanatory variable to be substituted with the hematocrit in whole blood and an explanatory variable to be substituted with the serum concentration of albumin or the plasma concentration of an amino acid was found.

A logistic regression expression was used as the multivariate discriminant. The hematocrit in whole blood, the serum concentration of albumin, and the plasma concentrations of the 19 kinds of amino acids were searched for a combination of three explanatory variables to be included in the logistic regression expression (where the explanatory variable of hematocrit was essential) in the same manner as in Example 24, and a search for logistic regression expressions having a satisfactory ability to discriminate between the MCI group and the healthy group was conducted.

FIG. 35 shows a list of logistic regression expressions with three explanatory variables, in which ROC_AUC for the MCI group and the healthy group is equal to or greater than 0.633 shown in FIG. 34. These logistic regression expressions are presumed to be valuable in the evaluation because ROC_AUC is high.

### Example 26

The whole blood samples of MCI people aged 60 years old or older with clinical diagnosis of MCI (MCI group: 120 subjects) and healthy elderly people aged 60 years old or older presumably with normal cognitive function (healthy group: 87 subjects) were measured by the sheath flow DC detecting method to determine the platelet count in blood.

The ability to discriminate between the MCI group and the healthy group was evaluated by ROC_AUC using data of the platelet count (×10⁴/µL) in whole blood, and ROC_AUC was 0.541. The platelet count is presumed to be valuable for evaluating the state of MCI in consideration of a healthy state.

### Example 27

The plasma samples, the whole blood samples, and the serum samples of MCI people aged 60 years old or older with clinical diagnosis of MCI (MCI group: 120 subjects) and healthy elderly people aged 60 years old or older presumably with normal cognitive function (healthy group: 87 subjects) were used. The blood concentrations of amino acids in the plasma samples were measured by the amino acid analyzing method (A), the platelet count in blood in the whole blood samples was measured by the sheath flow DC detecting method, and the blood concentration of albumin in the serum samples was measured by the modified BCP method.

A multivariate discriminant (multivariate function) for discriminating between the two groups, namely, the MCI group and the healthy group, including an explanatory variable to be substituted with the platelet count in whole blood and an explanatory variable to be substituted with the serum concentration of albumin or the plasma concentration of an amino acid was found.

A logistic regression expression was used as the multivariate discriminant. The platelet count (×10⁴/µL) in whole blood, the serum concentration of albumin (g/dL), and the plasma concentrations of the 19 kinds of amino acids (nmol/ml) were searched for a combination of two explanatory variables to be included in the logistic regression expression (where the explanatory variable of platelet count in blood (PLT) was essential), and a search for logistic regression expressions having a satisfactory ability to discriminate between the MCI group and the healthy group was conducted.

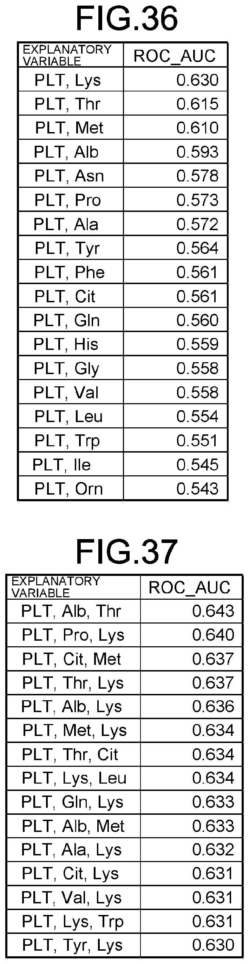

FIG. 36 shows a list of logistic regression expressions with two explanatory variables, in which ROC_AUC for the MCI group and the healthy group is equal to or greater than 0.541 described in Example 26. These logistic regression expressions are presumed to be valuable in the evaluation because ROC_AUC is high.

### Example 28

The sample data used in Example 27 was used. A multivariate discriminant (multivariate function) for discriminating between the two groups, namely, the MCI group and the healthy group, including an explanatory variable to be substituted with the platelet count in whole blood and an explanatory variable to be substituted with the serum concentration of albumin or the plasma concentration of an amino acid was found.

A logistic regression expression was used as the multivariate discriminant. The platelet count in whole blood, the serum concentration of albumin, and the plasma concentrations of the 19 kinds of amino acids were searched for a combination of three explanatory variables to be included in the logistic regression expression (where the explanatory variable of platelet count in blood was essential) in the same manner as in Example 27, and a search for logistic regression expressions having a satisfactory ability to discriminate between the MCI group and the healthy group was conducted.

FIG. 37 shows a list of logistic regression expressions with three explanatory variables, in which ROC_AUC for the MCI group and the healthy group is equal to or greater than 0.630 shown in FIG. 36. These logistic regression expressions are presumed to be valuable in the evaluation because ROC_AUC is high.

### INDUSTRIAL APPLICABILITY

As described above, the present invention may be practiced widely in many industrial fields, in particular in pharmaceutical, food, and medical fields, and is extremely useful in the field of bioinformatics, for example, performing progress prediction of the state of MCI, disease risk prediction, and proteome or metabolome analysis.

### EXPLANATION OF LETTERS OR NUMERALS

100 Evaluating apparatus
102 Control device
102a Obtaining part
102b Designating part
102c Formula-preparing part
102d Evaluating part
102d1 Calculating part
102d2 Converting part
102d3 Generating part
102d4 Classifying part
102e Result outputting part
102f Sending part
104 Communication interface
106 Memory device
106a Blood data file
106b Index state information file
106c Designated index state information file
106d Formula-related information database
106d1 Formula file
106e Evaluation result file
108 Input/output interface
112 Input device
114 Output device
200 Client apparatus (terminal apparatus (information communication terminal apparatus))
300 Network
400 Database apparatus

### [1. Two-Explanatory Variable Expressions]

Taurine, aAiBA, 0.933; Taurine, EtGly, 0.919; Taurine, Pyra zole-1-Ala, 0.912; Taurine, Spd, 0.896; Taurine, 1-MeHis, 0. 893; Taurine, SDMA, 0.881; Taurine, 3-MeHis, 0.879; Taurine, Kyn/BCAA, 0.879; Taurine, Kyn/Trp, 0.878; Taurine, Kyn, 0. 876; Taurine, Put, 0.871; Taurine, Cit, 0.871; Taurine, ADM A, 0.87; Taurine, MCSO2, 0.87; Taurine, GABA, 0.869; Taurin e, N8-AcSpd, 0.869; Taurine, Phe, 0.869; Taurine, Cystathio nine, 0.869; Pyrazole-1-Ala, Spd, 0.869; Gln, Taurine, 0.86 8; Taurine, bAiBA, 0.868; Taurine, Ile, 0.868; Taurine, Opt halmic acid, 0.868; Gly, Taurine, 0.867; Taurine, Tyr, 0.86 7; Taurine, Hypotaurine, 0.867; Taurine, Hypro, 0.867; Asn, Taurine, 0.867; Taurine, Val, 0.867; Taurine, N6-AcLys, 0. 867; Taurine, Arg, 0.867; Taurine, Trp, 0.867; Taurine, His, 0.866; Taurine, bABA, 0.866; Ser, Taurine, 0.866; Taurine, hArg, 0.866; Taurine, MeCys, 0.866; Taurine, MCSO1/MCSO2, 0.865; Taurine, Thioproline, 0.865; Taurine, Met, 0.864; Ta urine, Orn, 0.864; Taurine, MCSO2/MeCys, 0.864; Taurine, Pr o, 0.864; Taurine, Ala, 0.863; Taurine, Leu, 0.863; Taurine, aABA, 0.863; Taurine, MCSO1, 0.861; Taurine, Lys, 0.861; T aurine, Sar, 0.86; Taurine, aAAA, 0.86; Taurine, Thr, 0.86; EtGly, PEA, 0.859; Glu, Taurine, 0.858; Taurine, Pipecolic acid, 0.851; Taurine, PEA, 0.849; Taurine, Cysteic acid, 0. 847; 1-MeHis, Spd, 0.834; EtGly, Spd, 0.83; aAiBA, MCSO2, 0. 829; MCSO1/MCSO2, Spd, 0.824; N8-AcSpd, Spd, 0.822; MCSO2, Spd, 0.822; aAiBA, PEA, 0.82; Cit, Spd, 0.819; Pyrazole-1-A la, MCSO2, 0.818; aAAA, Spd, 0.818; aABA, Spd, 0.816; Spd, Thioproline, 0.815; Kyn/BCAA, Spd, 0.815; Pro, Spd, 0.815; EtGly, Pyrazole-1-Ala, 0.815; Pipecolic acid, Spd, 0.814; K yn, Spd, 0.814; Ile, Spd, 0.814; Hypro, Spd, 0.814; Gln, Sp d, 0.813; GABA, Spd, 0.813; Cystathionine, Spd, 0.812; Tyr, Spd, 0.811; N6-AcLys, Spd, 0.811; Trp, Spd, 0.811; Val, Sp d, 0.811; MCSO2, Put, 0.811; Ser, Spd, 0.81; Leu, Spd, 0.8 1; Pyrazole-1-Ala, Put, 0.81; SDMA, Spd, 0.81; bAiBA, Spd, 0.81; Phe, Spd, 0.809; 3-MeHis, Spd, 0.809; Kyn/Trp, Spd, 0. 809; ADMA, Spd, 0.808; Asn, Spd, 0.808; MeCys, Spd, 0.807; MCSO2/MeCys, Spd, 0.807; His, Spd, 0.806; Ala, Spd, 0.806; EtGly, Put, 0.806; Met, Spd, 0.806; Hypotaurine, Spd, 0.80 5; hArg, Spd, 0.805; Gly, Spd, 0.805; Orn, Spd, 0.804; Arg, Spd, 0.804; Pyrazole-1-Ala, MeCys, 0.804; EtGly, Opthalmic acid, 0.802; bABA, Spd, 0.802; Thr, Spd, 0.8; Lys, Spd, 0. 799; Pyrazole-1-Ala, PEA, 0.799; aAiBA, MCSO2/MeCys, 0.798; MCSO1, Spd, 0.797; hArg, MCSO2, 0.794; MCSO2, Spm, 0.792; Lys, Pyrazole-1-Ala, 0.792; Put, Spd, 0.792; Sar, Spd, 0.79 1; Opthalmic acid, Spd, 0.791; His, Pyrazole-1-Ala, 0.79; G lu, Spd, 0.79; aAiBA, MeCys, 0.788; Lys, MCSO2, 0.788; 3-Me His, PEA, 0.786; bAiBA, PEA, 0.785; Gln, PEA, 0.785; EtGly, MeCys, 0.785; bABA, PEA, 0.785; MCSO2/MeCys, Put, 0.784; 1 -MeHis, PEA, 0.784; MeCys, Put, 0.782; Hypro, PEA, 0.781; M et, MCSO2, 0.78; Thr, Pyrazole-1-Ala, 0.78; Taurine, Allyl Cys, 0.778; aAAA, PEA, 0.778; Pyrazole-1-Ala, MCSO2/MeCys, 0.778; MCSO2, PEA, 0.777; 1-MeHis, MCSO2, 0.777; hArg, MeCy s, 0.777; Allyl Cys, Pyrazole-1-Ala, 0.777; Cit, MCSO2, 0.7 76; Ser, PEA, 0.775; Phe, PEA, 0.775; MCSO2, MCSO1, 0.774; aAiBA, Spd, 0.774; N8-AcSpd, Put, 0.773; MCSO2, N6-AcLys, 0. 773; Orn, MCSO2, 0.772; Pro, MCSO2, 0.772; Met, Pyrazole-1-Ala, 0.772; PEA, Thioproline, 0.772; Allyl Cys, MCSO2, 0.77 2; Gly, MCSO2, 0.771; Ala, MCSO2, 0.771; aAAA, MCSO2, 0.77 1; Pyrazole-1-Ala, N6-AcLys, 0.77; bABA, MCSO2, 0.77; Thr, MCSO2, 0.77; Arg, MCSO2, 0.77; Lys, MCSO2/MeCys, 0.77; MCSO 2/MeCys, Spm, 0.77; MCSO2, Sar, 0.77; MCSO2, N8-AcSpd, 0.76 9; MCSO2, MCSO1/MCSO2, 0.769; His, MCSO2, 0.769; Tyr, MCSO2, 0.769; bAiBA, MCSO2, 0.767; Trp, MCSO2, 0.767; Cysteic aci d, MCSO2, 0.767; MCSO2, Pipecolic acid, 0.767; aABA, MCSO2, 0.767; MCSO2, SDMA, 0.767; Hypotaurine, PEA, 0.767; hArg, Pyrazole-1-Ala, 0.766; Kyn/Trp, MCSO2, 0.766; PEA, Spd, 0.7 66; hArg, MCSO2/MeCys, 0.766; Hypro, MCSO2, 0.766; Kyn/BCAA, MCSO2, 0.766; Asn, Pyrazole-1-Ala, 0.765; Leu, MCSO2, 0.76 5; ADMA, MCSO2, 0.765; Ser, MCSO2, 0.764; GABA, MCSO2, 0.76 4; Phe, MCSO2, 0.764; 3-MeHis, MCSO2, 0.764; Hypotaurine, M CSO2, 0.762; MCSO2, MCSO2/MeCys, 0.762; Asn, MCSO2, 0.762; Kyn, MCSO2, 0.761; Ile, PEA, 0.761; MCSO2, MeCys, 0.761; Gl u, MCSO2, 0.761; Ile, MCSO2, 0.76; Cystathionine, MCSO2, 0. 76; Cysteic acid, Spd, 0.76; Lys, MeCys, 0.76; Pyrazole-1-A la, MCSO1/MCSO2, 0.76; Gln, MCSO2, 0.76; Pyrazole-1-Ala, Sp m, 0.759; Val, MCSO2, 0.759; MCSO2/MeCys, MeCys, 0.759; aAi BA, Pyrazole-1-Ala, 0.759; MCSO2, Thioproline, 0.758; His, Opthalmic acid, 0.758; MCSO1/MCSO2, MCSO2/MeCys, 0.757; Leu, PEA, 0.757; Ala, Pyrazole-1-Ala, 0.756; MCSO1/MCSO2, MeCys, 0.756; Trp, PEA, 0.756; aABA, Pyrazole-1-Ala, 0.756; MCSO2 /MeCys, PEA, 0.755; Thr, MCSO2/MeCys, 0.755; Arg, Pyrazole-1-Ala, 0.755; Taurine, Spm, 0.754; ADMA, PEA, 0.754; Hypota urine, Pyrazole-1-Ala, 0.753; Kyn/BCAA, PEA, 0.753; Pro, Py razole-1-Ala, 0.753; Kyn/Trp, Pyrazole-1-Ala, 0.753; Opthal mic acid, Put, 0.753; PEA, Pipecolic acid, 0.752; Cystathio nine, PEA, 0.752; Pyrazole-1-Ala, Sar, 0.752; Tyr, PEA, 0.7 52; MeCys, PEA, 0.751; aAAA, Pyrazole-1-Ala, 0.751; MeCys, Sar, 0.75; Val, PEA, 0.75; hArg, Put, 0.749; MeCys, N6-AcLy s, 0.749; Allyl Cys, MCSO2/MeCys, 0.749; Pro, MCSO2/MeCys, 0.749; MCSO2, Opthalmic acid, 0.748; EtGly, Spm, 0.748; Gly, Pyrazole-1-Ala, 0.746; Met, MeCys, 0.746; Lys, Allyl Cys, 0.746; 1-MeHis, MCSO2/MeCys, 0.746; Met, MCSO2/MeCys, 0.74 6; Cystathionine, Pyrazole-1-Ala, 0.745; MCSO1, MCSO1/MCSO2, 0.745; MeCys, SDMA, 0.745; His, MeCys, 0.745; Orn, Pyrazol e-1-Ala, 0.744; Allyl Cys, MeCys, 0.744; ADMA, Pyrazole-1-A la, 0.744; aAiBA, Put, 0.744; MCSO1, MeCys, 0.744; Met, PEA, 0.743; Put, SDMA, 0.743; His, PEA, 0.743; 3-MeHis, Pyrazol e-1-Ala, 0.742; His, MCSO2/MeCys, 0.742; Kyn/Trp, MCSO2/MeC ys, 0.742; Arg, MeCys, 0.742; Pyrazole-1-Ala, SDMA, 0.742; EtGly, MCSO1/MCSO2, 0.741; Tyr, MeCys, 0.741; Cysteic acid, Pyrazole-1-Ala, 0.74; Pyrazole-1-Ala, Pipecolic acid, 0.7 4; GABA, Pyrazole-1-Ala, 0.74; PEA, Put, 0.74; Orn, MCSO2/M eCys, 0.739; GABA, PEA, 0.739; Kyn/BCAA, MCSO2/MeCys, 0.73 9; MCSO1/MCSO2, Put, 0.739; Thr, MeCys, 0.739; 1-MeHis, Pyr azole-1-Ala, 0.738; Ala, MCSO2/MeCys, 0.738; MeCys, N8-AcSp d, 0.737; aABA, EtGly, 0.737; Trp, Pyrazole-1-Ala, 0.737; b AiBA, Pyrazole-1-Ala, 0.737; Glu, Pyrazole-1-Ala, 0.737; Ar g, MCSO2/MeCys, 0.737; Leu, MeCys, 0.737; Ser, Pyrazole-1-A la, 0.736; Ala, MeCys, 0.736; Leu, MCSO2/MeCys, 0.736; Hypr o, Pyrazole-1-Ala, 0.736; Pro, MeCys, 0.736; Pyrazole-1-Ala, Thioproline, 0.736; Cit, MeCys, 0.736; Orn, MeCys, 0.736; Trp, MeCys, 0.736; Kyn/BCAA, Pyrazole-1-Ala, 0.736; Kyn, Py razole-1-Ala, 0.735; Cit, Pyrazole-1-Ala, 0.735; bABA, MeCy s, 0.735; 1-MeHis, Put, 0.735; Gly, MeCys, 0.735; bABA, Pyr azole-1-Ala, 0.735; aAAA, MeCys, 0.735; Allyl Cys, Put, 0.7 35; Ile, Pyrazole-1-Ala, 0.734; ADMA, MeCys, 0.734; MCSO2/M eCys, N6-AcLys, 0.734; MCSO2/MeCys, Pipecolic acid, 0.734; MCSO1, MCSO2/MeCys, 0.734; Asn, PEA, 0.734; Kyn/BCAA, MeCys, 0.733; EtGly, MCSO2, 0.733; Asn, MCSO2/MeCys, 0.733; Cit, MCSO2/MeCys, 0.733; Trp, MCSO2/MeCys, 0.733; Kyn/Trp, Put, 0.733; aAAA, MCSO2/MeCys, 0.732; bAiBA, MeCys, 0.732; Pyraz ole-1-Ala, MCSO1, 0.732; His, Put, 0.732; aABA, MeCys, 0.73 2; bABA, MCSO2/MeCys, 0.732; Gln, Pyrazole-1-Ala, 0.732; Ly s, Put, 0.732; Kyn/Trp, MeCys, 0.732; MCSO2/MeCys, SDMA, 0. 732; 3-MeHis, MeCys, 0.731; 1-MeHis, MeCys, 0.731; Lys, PEA, 0.731; Gly, MCSO2/MeCys, 0.731; Cysteic acid, MeCys, 0.73 1; Allyl Cys, Spd, 0.73; Ser, MeCys, 0.73; Cystathionine, M eCys, 0.73; GABA, MCSO2/MeCys, 0.73; MCSO2/MeCys, N8-AcSpd, 0.73; Pyrazole-1-Ala, Opthalmic acid, 0.73; ADMA, MCSO2/Me Cys, 0.73; Ile, MeCys, 0.729; Pyrazole-1-Ala, N8-AcSpd, 0.7 29; Gln, MeCys, 0.729; Asn, MeCys, 0.728; Hypro, MeCys, 0.7 28; bAiBA, MCSO2/MeCys, 0.728; hArg, Opthalmic acid, 0.728; Ala, Put, 0.728; Leu, Pyrazole-1-Ala, 0.728; Phe, MeCys, 0. 728; MCSO1, PEA, 0.727; Kyn/BCAA, Put, 0.727; Val, Pyrazole -1-Ala, 0.727; Ile, MCSO2/MeCys, 0.727; Tyr, Pyrazole-1-Ala, 0.727; Val, MeCys, 0.727; Glu, MeCys, 0.726; Thr, Put, 0.7 26; Kyn, MeCys, 0.726; PEA, SDMA, 0.726; MCSO2/MeCys, Sar, 0.726; Kyn, Put, 0.726; MeCys, Pipecolic acid, 0.726; Tyr, MCSO2/MeCys, 0.725; Hypro, MCSO2/MeCys, 0.725; Phe, Pyrazol e-1-Ala, 0.725; Hypotaurine, MeCys, 0.725; Gly, PEA, 0.725; aABA, Put, 0.725; MeCys, Thioproline, 0.724; Val, MCSO2/Me Cys, 0.724; Cystathionine, Put, 0.724; GABA, MeCys, 0.724; Orn, PEA, 0.724; Cystathionine, MCSO2/MeCys, 0.723; Hypotau rine, MCSO2/MeCys, 0.723; Put, Thioproline, 0.723; Kyn, MCS O2/MeCys, 0.723; Glu, MCSO2/MeCys, 0.723; Hypotaurine, Put, 0.722; MeCys, Spm, 0.722; Cit, EtGly, 0.722; Kyn/Trp, PEA, 0.722; Hypro, Put, 0.722; Gln, MCSO2/MeCys, 0.721; aABA, M CSO2/MeCys, 0.72; Cit, Put, 0.72; 3-MeHis, MCSO2/MeCys, 0.7 2; Cysteic acid, MCSO2/MeCys, 0.72; Phe, MCSO2/MeCys, 0.71 9; aABA, PEA, 0.719; MCSO2/MeCys, Thioproline, 0.719; Met, Put, 0.719; EtGly, Hypro, 0.719; EtGly, Kyn/BCAA, 0.719; Se r, MCSO2/MeCys, 0.718; Gly, Put, 0.718; Cysteic acid, PEA, 0.718; Trp, Put, 0.717; Orn, Put, 0.717; 3-MeHis, Put, 0.71 7; N6-AcLys, PEA, 0.716; Pro, PEA, 0.716; Ala, PEA, 0.715; Thr, PEA, 0.715; Pro, Put, 0.715; GABA, Put, 0.713; Asn, Pu t, 0.713; Tyr, Put, 0.713; Phe, Put, 0.713; MCSO1, Put, 0.7 13; Cysteic acid, Put, 0.713; Arg, Put, 0.712; Ser, Put, 0. 712; N8-AcSpd, PEA, 0.712; N6-AcLys, Put, 0.712; Put, Sar, 0.711; Val, Put, 0.711; Leu, Put, 0.711; bAiBA, Put, 0.71; Ile, Put, 0.71; hArg, PEA, 0.709; ADMA, Put, 0.709; Arg, PE A, 0.709; Kyn, PEA, 0.709; His, Cit, 0.709; Cit, PEA, 0.70 9; Cysteic acid, MCSO1/MCSO2, 0.708; Gln, Put, 0.707; MeCys, Opthalmic acid, 0.706; Thr, Opthalmic acid, 0.705; His, SD MA, 0.704; Glu, Put, 0.704; Cit, Lys, 0.703; MCSO2/MeCys, O pthalmic acid, 0.703; N6-AcLys, SDMA, 0.703; His, Kyn, 0.70 2; Lys, MCSO1/MCSO2, 0.702; bABA, Put, 0.702; Lys, N8-AcSpd, 0.702; Thr, Spm, 0.701; His, 1-MeHis, 0.701; aAAA, Put, 0. 701; Lys, 1-MeHis, 0.7; His, hArg, 0.7; Lys, Hypotaurine, 0. 7; Lys, SDMA, 0.7; hArg, Hypotaurine, 0.7

### [2. Three-Explanatory Variable Expressions]

Taurine, 1-MeHis, Spd, 0.923; Taurine, 1-MeHis, Kyn/Trp, 0. 914; Taurine, 1-MeHis, SDMA, 0.914; Taurine, 1-MeHis, Kyn/B CAA, 0.912; Taurine, 1-MeHis, Kyn, 0.91; Taurine, SDMA, Spd, 0.909; Taurine, 1-MeHis, 3-MeHis, 0.908; Taurine, Cit, 1-M eHis, 0.908; Taurine, Kyn, Spd, 0.905; Taurine, Kyn/BCAA, S pd, 0.905; Taurine, 3-MeHis, Spd, 0.905; Taurine, Kyn/Trp, Spd, 0.903; Taurine, Spd, Thioproline, 0.902; Taurine, Cit, Spd, 0.902; Taurine, 1-MeHis, MCSO2, 0.902; Taurine, 1-MeH is, GABA, 0.901; Taurine, 1-MeHis, hArg, 0.9; Taurine, 1-Me His, N6-AcLys, 0.9; Taurine, 1-MeHis, N8-AcSpd, 0.9; Taurin e, His, 1-MeHis, 0.9; Taurine, 1-MeHis, Put, 0.899; Taurine, 1-MeHis, MCSO2/MeCys, 0.899; Taurine, GABA, Spd, 0.899; Ta urine, Cystathionine, Spd, 0.898; Taurine, Hypro, Spd, 0.89 8; Asn, Taurine, Spd, 0.897; Taurine, ADMA, Spd, 0.897; Tau rine, Phe, Spd, 0.897; Taurine, 1-MeHis, ADMA, 0.896; Tauri ne, 1-MeHis, Cystathionine, 0.896; Taurine, 1-MeHis, MCSO1/ MCSO2, 0.896; Taurine, 1-MeHis, bAiBA, 0.896; Taurine, Tyr, Spd, 0.896; Taurine, Val, Spd, 0.896; Taurine, Orn, Spd, 0. 896; Taurine, Ile, Spd, 0.896; Taurine, Met, 1-MeHis, 0.89 6; Taurine, Trp, 1-MeHis, 0.896; Taurine, 1-MeHis, Hypro, 0. 896; Gln, Taurine, Spd, 0.896; Taurine, Arg, 1-MeHis, 0.89 6; Taurine, 1-MeHis, Thioproline, 0.896; Ser, Taurine, Spd, 0.896; Taurine, Tyr, 1-MeHis, 0.895; Taurine, 1-MeHis, bAB A, 0.895; Taurine, bAiBA, Spd, 0.895; Taurine, Arg, Spd, 0. 895; Taurine, Val, 1-MeHis, 0.895; Taurine, Trp, Spd, 0.89 5; Taurine, N8-AcSpd, Spd, 0.895; Asn, Taurine, 1-MeHis, 0. 895; Gly, Taurine, 1-MeHis, 0.895; Taurine, Orn, 1-MeHis, 0. 895; Taurine, aABA, Spd, 0.895; Taurine, N6-AcLys, Spd, 0.8 95; Taurine, aABA, 1-MeHis, 0.894; Taurine, Leu, 1-MeHis, 0. 894; Gly, Taurine, Spd, 0.894; Taurine, Ile, 1-MeHis, 0.89 4; Taurine, Phe, 1-MeHis, 0.894; Taurine, hArg, Spd, 0.894; Ser, Taurine, 1-MeHis, 0.893; Taurine, Ala, 1-MeHis, 0.89 3; Taurine, 1-MeHis, Hypotaurine, 0.893; Taurine, bABA, Spd, 0.893; Taurine, Met, Spd, 0.893; Gln, Taurine, 1-MeHis, 0. 893; Taurine, Leu, Spd, 0.893; Taurine, aAAA, Spd, 0.893; T aurine, 1-MeHis, MeCys, 0.893; Taurine, Put, SDMA, 0.893; T aurine, Thr, 1-MeHis, 0.893; Taurine, Lys, 1-MeHis, 0.892; Taurine, 1-MeHis, MCSO1, 0.892; Taurine, Kyn/Trp, Put, 0.89 2; Taurine, Pro, Spd, 0.892; Taurine, MCSO1, Spd, 0.892; Ta urine, Pro, 1-MeHis, 0.892; Taurine, Kyn/BCAA, Put, 0.891; Taurine, MCSO1/MCSO2, Spd, 0.891; Taurine, Ala, Spd, 0.89; Taurine, 1-MeHis, aAAA, 0.889; Taurine, Put, Spd, 0.889; Gl u, Taurine, 1-MeHis, 0.888; Taurine, 1-MeHis, Sar, 0.888; T aurine, His, Kyn, 0.887; Taurine, Hypotaurine, Spd, 0.887; Taurine, 1-MeHis, PEA, 0.887; Taurine, 3-MeHis, Put, 0.886; Taurine, 1-MeHis, Pipecolic acid, 0.886; Glu, Taurine, Spd, 0.886; Taurine, His, Spd, 0.886; Taurine, 1-MeHis, Cysteic acid, 0.886; Taurine, Lys, Spd, 0.886; Taurine, Cit, SDMA, 0.885; Taurine, Pipecolic acid, Spd, 0.884; Taurine, Leu, Kyn, 0.884; Taurine, Thr, Spd, 0.884; Taurine, Hypro, SDMA, 0.883; Taurine, MCSO2, Put, 0.883; Taurine, Kyn/BCAA, SDMA, 0.883; Gln, Taurine, SDMA, 0.883; Taurine, Ala, SDMA, 0.88 3; Taurine, Cystathionine, Kyn/BCAA, 0.883; Taurine, GABA, SDMA, 0.883; Taurine, His, SDMA, 0.882; Taurine, Kyn, Put, 0.882; Gly, Taurine, SDMA, 0.882; Taurine, Met, SDMA, 0.88 2; Taurine, Arg, SDMA, 0.882; Taurine, MCSO2/MeCys, Spd, 0. 882; Taurine, aABA, SDMA, 0.882; Taurine, His, Kyn/Trp, 0.8 81; Taurine, Ile, SDMA, 0.881; Taurine, hArg, SDMA, 0.881; Taurine, aAAA, SDMA, 0.881; Ser, Taurine, SDMA, 0.881; Taur ine, Val, SDMA, 0.881; Taurine, aABA, Kyn/BCAA, 0.881; Taur ine, Hypotaurine, SDMA, 0.881; Taurine, SDMA, Thioproline, 0.88; Taurine, Cit, Kyn/BCAA, 0.88; Taurine, Ile, Kyn, 0.8 8; Taurine, Phe, SDMA, 0.88; Taurine, Trp, SDMA, 0.88; Taur ine, Kyn/Trp, MCSO1/MCSO2, 0.88; Taurine, Met, 3-MeHis, 0.8 8; Taurine, 3-MeHis, Kyn/BCAA, 0.88; Taurine, hArg, Kyn, 0. 88; Taurine, Kyn/BCAA, Thioproline, 0.88; Taurine, Val, Kyn /BCAA, 0.88; Taurine, ADMA, SDMA, 0.88; Taurine, N8-AcSpd, Put, 0.88; Taurine, bABA, SDMA, 0.88; Taurine, Arg, Kyn/BCA A, 0.88; Taurine, 3-MeHis, Hypro, 0.88; Taurine, Cystathion ine, SDMA, 0.88; Taurine, Hypotaurine, Kyn/BCAA, 0.88; Gln, Taurine, Kyn/BCAA, 0.879; Taurine, Val, Kyn, 0.879; Taurin e, Phe, Kyn/BCAA, 0.879; Taurine, Kyn, SDMA, 0.879; Ser, Ta urine, Kyn/BCAA, 0.879; Taurine, Ala, 3-MeHis, 0.879; Tauri ne, Tyr, 3-MeHis, 0.879; Taurine, Tyr, SDMA, 0.879; Taurine, Trp, Kyn/BCAA, 0.879; Taurine, GABA, Kyn/BCAA, 0.879; Taur ine, hArg, Kyn/Trp, 0.879; Taurine, hArg, Kyn/BCAA, 0.879; Taurine, Kyn, Kyn/BCAA, 0.879; Taurine, Kyn/BCAA, MCSO1/MCS O2, 0.879; Taurine, Arg, 3-MeHis, 0.879; Taurine, Tyr, Kyn/ BCAA, 0.879; Taurine, Ile, Kyn/BCAA, 0.879; Taurine, MCSO2, Spd, 0.879; Asn, Taurine, SDMA, 0.879; Taurine, Cit, Kyn, 0.879; Taurine, Trp, 3-MeHis, 0.879; Taurine, Kyn/Trp, SDMA, 0.879; Taurine, Sar, Spd, 0.878; Taurine, Cit, Put, 0.878; Taurine, aABA, 3-MeHis, 0.878; Taurine, Val, 3-MeHis, 0.87 8; Taurine, Leu, Kyn/BCAA, 0.878; Taurine, hArg, Put, 0.87 8; Taurine, Met, Kyn/BCAA, 0.878; Taurine, Ile, 3-MeHis, 0. 878; Taurine, 3-MeHis, ADMA, 0.878; Taurine, 3-MeHis, Hypot aurine, 0.878; Taurine, Kyn/BCAA, MCSO2, 0.878; Taurine, Ky n, Sar, 0.878; Taurine, 3-MeHis, aAAA, 0.878; Taurine, aAAA, Kyn/BCAA, 0.878; Taurine, bAiBA, SDMA, 0.878; Gln, Taurine, Kyn/Trp, 0.878; Taurine, Pro, Kyn/BCAA, 0.878; Taurine, aA BA, Kyn, 0.878; Taurine, 3-MeHis, SDMA, 0.878; Taurine, Hyp ro, Kyn/BCAA, 0.878; Taurine, Kyn/Trp, Kyn/BCAA, 0.878; Tau rine, MCSO2/MeCys, Put, 0.878; Taurine, bABA, Kyn/BCAA, 0.8 78; Taurine, Pro, 3-MeHis, 0.878; Taurine, Orn, Kyn/BCAA, 0. 878; Taurine, Orn, SDMA, 0.878; Taurine, Ile, Kyn/Trp, 0.87 8; Taurine, ADMA, Kyn/BCAA, 0.878; Taurine, Kyn, MCSO2, 0.8 78; Taurine, Kyn/BCAA, N8-AcSpd, 0.878; Taurine, MeCys, Spd, 0.878; Ser, Taurine, 3-MeHis, 0.877; Asn, Taurine, Kyn/BCA A, 0.877; Gln, Taurine, 3-MeHis, 0.877; Taurine, His, Kyn/B CAA, 0.877; Taurine, Lys, 3-MeHis, 0.877; Taurine, Leu, SDM A, 0.877; Taurine, Kyn/Trp, Thioproline, 0.877; Gly, Taurin e, 3-MeHis, 0.877; Gly, Taurine, Kyn/BCAA, 0.877; Taurine, Cit, Kyn/Trp, 0.877; Taurine, Met, Kyn, 0.877; Taurine, Trp, Kyn/Trp, 0.877; Taurine, Cystathionine, Kyn/Trp, 0.877; Ta urine, Hypotaurine, Kyn/Trp, 0.877; Taurine, Kyn/BCAA, MeCy s, 0.877; Taurine, Kyn, Thioproline, 0.877; Taurine, 3-MeHi s, bABA, 0.877; Ser, Taurine, MCSO2, 0.877; Taurine, Ala, K yn/BCAA, 0.877; Taurine, Orn, 3-MeHis, 0.877; Taurine, Lys, SDMA, 0.877; Taurine, 3-MeHis, hArg, 0.877; Taurine, GABA, Kyn, 0.877; Taurine, MCSO1/MCSO2, SDMA, 0.877; Taurine, Me Cys, Put, 0.877; Taurine, bAiBA, Kyn/BCAA, 0.877; Taurine, Cit, 3-MeHis, 0.877; Taurine, Arg, Kyn/Trp, 0.877; Taurine, Tyr, Kyn/Trp, 0.877; Taurine, bABA, Kyn/Trp, 0.877; Taurin e, Kyn, Kyn/Trp, 0.877; Taurine, Kyn/Trp, MCSO2, 0.877; Tau rine, N8-AcSpd, SDMA, 0.877; Taurine, 3-MeHis, bAiBA, 0.87 6; Ser, Taurine, Kyn/Trp, 0.876; Taurine, Ala, Kyn/Trp, 0.8 76; Taurine, Val, Kyn/Trp, 0.876; Taurine, Lys, Kyn, 0.876; Asn, Taurine, Kyn/Trp, 0.876; Taurine, Leu, 3-MeHis, 0.87 6; Taurine, 3-MeHis, Thioproline, 0.876; Ser, Taurine, Kyn, 0.876; Taurine, Pro, SDMA, 0.876; Taurine, Put, Thioprolin e, 0.876; Asn, Taurine, 3-MeHis, 0.876; Taurine, Ala, Kyn, 0.876; Taurine, Tyr, Kyn, 0.876; Taurine, Met, Kyn/Trp, 0.8 76; Taurine, Hypotaurine, Kyn, 0.876; Taurine, Hypro, Kyn/T rp, 0.876; Taurine, Kyn, MCSO1/MCSO2, 0.876; Taurine, 3-MeH is, Sar, 0.876; Gln, Taurine, Kyn, 0.875; Taurine, Phe, 3-M eHis, 0.875; Taurine, Phe, Kyn, 0.875; Gly, Taurine, Kyn/Tr p, 0.875; Taurine, MCSO1, SDMA, 0.875; Taurine, aABA, Kyn/T rp, 0.875; Taurine, Cystathionine, Kyn, 0.875; Taurine, bAB A, Kyn, 0.875; Taurine, Thr, MCSO2, 0.875; Taurine, 3-MeHis, GABA, 0.875; Taurine, 3-MeHis, MCSO1/MCSO2, 0.875; Taurine, hArg, MCSO2, 0.875; Taurine, Kyn/Trp, MeCys, 0.875; Taurin e, bAiBA, Put, 0.875; Taurine, His, 3-MeHis, 0.875; Taurine, Thr, Kyn/BCAA, 0.875; Taurine, Cit, MCSO2, 0.875; Taurine, Cit, MCSO1/MCSO2, 0.875; Taurine, Lys, Cystathionine, 0.87 5; Taurine, Leu, Kyn/Trp, 0.875; Taurine, MCSO1/MCSO2, Put, 0.875; Taurine, N6-AcLys, Put, 0.875; Taurine, bAiBA, Kyn, 0.875; Taurine, His, Cystathionine, 0.874; Taurine, Thr, S DMA, 0.874; Taurine, Met, Cystathionine, 0.874; Taurine, Tr p, Put, 0.874; Taurine, ADMA, Kyn/Trp, 0.874; Taurine, Kyn/ BCAA, MCSO1, 0.874; Taurine, MCSO2, SDMA, 0.874; Gly, Tauri ne, Kyn, 0.874; Taurine, Trp, Kyn, 0.874; Taurine, Hypro, K yn, 0.874; Taurine, Kyn/Trp, N8-AcSpd, 0.874; Taurine, bAiB A, Kyn/Trp, 0.874; Taurine, Thr, Kyn, 0.874; Taurine, Cit, aABA, 0.874; Taurine, Kyn/BCAA, Sar, 0.874; Taurine, Arg, K yn, 0.874; Taurine, Pro, MCSO2, 0.874; Taurine, aABA, Put, 0.874; Taurine, Lys, Kyn/BCAA, 0.874; Taurine, Phe, Kyn/Trp, 0.874; Taurine, GABA, Put, 0.874; Taurine, MeCys, SDMA, 0. 874; Taurine, Orn, Kyn/Trp, 0.873; Taurine, 3-MeHis, Cystat hionine, 0.873; Taurine, GABA, MCSO2, 0.873; Taurine, Kyn/B CAA, N6-AcLys, 0.873; Taurine, Cit, Hypro, 0.873; Taurine, Tyr, Put, 0.873; Glu, Taurine, Kyn/BCAA, 0.873; Asn, Taurin e, Kyn, 0.873; Gly, Taurine, Cit, 0.873; Gln, Taurine, Cit, 0.873; Taurine, Cit, Arg, 0.873; Taurine, GABA, MCSO1/MCSO 2, 0.873; Taurine, aAAA, Kyn, 0.873; Glu, Taurine, MCSO2, 0. 873; Ser, Taurine, Cit, 0.873; Taurine, His, N8-AcSpd, 0.87 3; Taurine, Arg, Put, 0.873; Taurine, Leu, Cystathionine, 0. 873; Taurine, Cystathionine, Put, 0.873; Taurine, aAAA, Kyn /Trp, 0.872; Taurine, 3-MeHis, Kyn/Trp, 0.872; Taurine, 3-M eHis, MCSO1, 0.872; Taurine, GABA, hArg, 0.872; Taurine, hA rg, MeCys, 0.872; Taurine, Kyn, MeCys, 0.872; Taurine, Cit, hArg, 0.872; Taurine, Pro, Kyn/Trp, 0.872; Taurine, Ile, P ut, 0.872; Taurine, Leu, Put, 0.872; Taurine, ADMA, Kyn, 0. 872; Taurine, Cystathionine, MCSO2, 0.872; Ser, Taurine, Pu t, 0.872; Gln, Taurine, Put, 0.872; Taurine, Cit, Trp, 0.87 2; Taurine, Cit, Hypotaurine, 0.872; Taurine, Arg, N8-AcSpd, 0.872; Taurine, Val, Cystathionine, 0.872; Taurine, GABA, Kyn/Trp, 0.872; Taurine, Hypro, Put, 0.872; Taurine, MCSO2/ MeCys, MeCys, 0.872; Taurine, Cit, Thioproline, 0.872; Taur ine, MCSO2, Thioproline, 0.872; Asn, Taurine, Put, 0.872; T aurine, Arg, Cystathionine, 0.872; Taurine, Pro, Kyn, 0.87 2; Taurine, Orn, Put, 0.872; Taurine, Phe, Put, 0.872; Taur ine, 3-MeHis, Kyn, 0.872; Taurine, MCSO2, MCSO1/MCSO2, 0.87 2; Taurine, bABA, MCSO2, 0.871; Taurine, Sar, SDMA, 0.871; Taurine, Cit, Tyr, 0.871; Taurine, Cit, Phe, 0.871; Taurine, aABA, N8-AcSpd, 0.871; Taurine, Val, Put, 0.871; Taurine, 3-MeHis, N8-AcSpd, 0.871; Gly, Taurine, MCSO2, 0.871; Tauri ne, His, Put, 0.871; Taurine, Met, N8-AcSpd, 0.871; Taurine, Met, Put, 0.871; Taurine, Orn, Kyn, 0.871; Taurine, Leu, P he, 0.871; Taurine, 3-MeHis, N6-AcLys, 0.871; Taurine, Cyst eic acid, MCSO1/MCSO2, 0.871; Asn, Taurine, His, 0.871; Tau rine, Cit, Leu, 0.871; Taurine, Phe, MCSO2, 0.871; Taurine, Cystathionine, GABA, 0.871; Taurine, hArg, N6-AcLys, 0.87 1; Taurine, Hypotaurine, Put, 0.871; Taurine, Hypro, MCSO2, 0.871; Taurine, MCSO2, MCSO1, 0.871; Gln, Taurine, N8-AcSp d, 0.871; Taurine, Cit, Met, 0.871; Taurine, Val, N8-AcSpd, 0.871; Taurine, Lys, Put, 0.871; Taurine, 3-MeHis, MCSO2, 0.871; Taurine, Kyn, MCSO1, 0.871; Taurine, Kyn, N8-AcSpd, 0.871; Taurine, Kyn/BCAA, MCSO2/MeCys, 0.871; Asn, Taurine, N8-AcSpd, 0.87; Taurine, Ala, Cit, 0.87; Taurine, Cit, Val, 0.87; Taurine, Cit, Ile, 0.87; Taurine, Met, GABA, 0.87; T aurine, Lys, MCSO2, 0.87; Taurine, Lys, N6-AcLys, 0.87; Tau rine, Leu, MCSO2, 0.87; Taurine, Trp, MCSO2, 0.87; Taurine, Kyn/Trp, MCSO2/MeCys, 0.87; Taurine, bABA, Put, 0.87; Taur ine, aAAA, N8-AcSpd, 0.87; Taurine, Ala, Cystathionine, 0.8 7; Taurine, Ala, Put, 0.87; Taurine, Cit, Pro, 0.87; Taurin e, Tyr, MCSO2, 0.87; Taurine, Ile, hArg, 0.87; Taurine, Ile, MCSO2, 0.87; Taurine, Leu, GABA, 0.87; Taurine, ADMA, Hypo taurine, 0.87; Taurine, ADMA, MCSO2, 0.87; Taurine, ADMA, M CSO1/MCSO2, 0.87; Taurine, Kyn/Trp, MCSO1, 0.87; Taurine, M CSO2, MCSO2/MeCys, 0.87; Taurine, MCSO2, MeCys, 0.87; Tauri ne, MCSO2, N8-AcSpd, 0.87; Taurine, Cit, bAiBA, 0.87; Tauri ne, bAiBA, GABA, 0.87; Asn, Taurine, MCSO2, 0.87; Gln, Taur ine, GABA, 0.87; Gln, Taurine, MCSO2, 0.87; Taurine, Cit, A DMA, 0.87; Taurine, aABA, Cystathionine, 0.87; Taurine, Orn, MCSO2, 0.87; Taurine, Lys, Kyn/Trp, 0.87; Taurine, Phe, N8 -AcSpd, 0.87; Taurine, ADMA, hArg, 0.87; Taurine, ADMA, Put, 0.87; Taurine, Cystathionine, Hypro, 0.87; Ser, Taurine, T yr, 0.87; Ser, Taurine, GABA, 0.87; Taurine, Thr, Kyn/Trp, 0.87; Taurine, Cit, N8-AcSpd, 0.87; Taurine, Trp, N8-AcSpd, 0.87; Taurine, ADMA, N8-AcSpd, 0.87; Taurine, MCSO1, Put, 0.87; Taurine, Cysteic acid, Spd, 0.87; Gly, Taurine, Cysta thionine, 0.869; Gln, Taurine, ADMA, 0.869; Gln, Taurine, C ystathionine, 0.869; Taurine, His, MCSO2, 0.869; Taurine, C it, Lys, 0.869; Taurine, Val, GABA, 0.869; Taurine, Ile, Le u, 0.869; Taurine, Ile, ADMA, 0.869; Taurine, Ile, Cystathi onine, 0.869; Taurine, Trp, Cystathionine, 0.869; Taurine, ADMA, Cystathionine, 0.869; Taurine, Cystathionine, N8-AcSp d, 0.869; Taurine, hArg, Thioproline, 0.869; Asn, Taurine, Cystathionine, 0.869; Taurine, Cit, Cystathionine, 0.869; T aurine, Tyr, Ile, 0.869; Taurine, Val, MCSO2, 0.869; Taurin e, ADMA, Hypro, 0.869; Taurine, GABA, N8-AcSpd, 0.869; Taur ine, hArg, Hypotaurine, 0.869; Taurine, Hypro, N8-AcSpd, 0. 869; Taurine, MCSO1/MCSO2, MeCys, 0.869; Taurine, Cysteic a cid, MCSO2, 0.869; Taurine, bAiBA, MCSO2, 0.869; Taurine, P EA, Spd, 0.869; Gly, Taurine, ADMA, 0.869; Gly, Taurine, N8 -AcSpd, 0.869; Gly, Taurine, Put, 0.869; Taurine, His, Ile, 0.869; Taurine, Arg, MCSO2, 0.869; Taurine, Pro, N6-AcLys, 0.869; Taurine, Pro, Put, 0.869; Taurine, Lys, N8-AcSpd, 0. 869; Taurine, Ile, N8-AcSpd, 0.869; Taurine, 3-MeHis, MeCys, 0.869; Taurine, GABA, Hypro, 0.869; Taurine, Hypotaurine, N8-AcSpd, 0.869; Taurine, Kyn/Trp, Sar, 0.869; Taurine, ADM A, bAiBA, 0.869; Taurine, Cystathionine, Thioproline, 0.86 9; Taurine, Cit, bABA, 0.869; Taurine, ADMA, bABA, 0.869; S er, Taurine, Ile, 0.869; Ser, Taurine, ADMA, 0.869; Ser, Ta urine, Cystathionine, 0.869; Ser, Taurine, N8-AcSpd, 0.869; Taurine, Pro, Cystathionine, 0.869; Taurine, aABA, MCSO2, 0.869; Taurine, Tyr, Hypro, 0.869; Taurine, Tyr, N8-AcSpd, 0.869; Taurine, Ile, Hypro, 0.869; Taurine, Phe, Hypro, 0.8 69; Taurine, Cystathionine, Hypotaurine, 0.869; Taurine, GA BA, MeCys, 0.869; Asn, Taurine, Tyr, 0.868; Gly, Taurine, T yr, 0.868; Taurine, Thr, MeCys, 0.868; Taurine, Ala, MCSO2, 0.868; Taurine, Cit, Orn, 0.868; Taurine, Arg, Ile, 0.868; Taurine, Tyr, Cystathionine, 0.868; Taurine, Tyr, MCSO1/MC SO2, 0.868; Taurine, Phe, Hypotaurine, 0.868; Taurine, Trp, ADMA, 0.868; Taurine, Trp, GABA, 0.868; Taurine, Cystathio nine, hArg, 0.868; Ser, Taurine, bAiBA, 0.868; Taurine, GAB A, Thioproline, 0.868; Taurine, Kyn/BCAA, Pipecolic acid, 0. 868; Ser, Gln, Taurine, 0.868; Gly, Taurine, GABA, 0.868; G ln, Taurine, Ile, 0.868; Gln, Taurine, Phe, 0.868; Taurine, Thr, 3-MeHis, 0.868; Taurine, Tyr, hArg, 0.868; Taurine, T yr, N6-AcLys, 0.868; Taurine, Ile, GABA, 0.868; Taurine, Le u, MeCys, 0.868; Taurine, Phe, ADMA, 0.868; Taurine, Phe, C ystathionine, 0.868; Taurine, ADMA, MeCys, 0.868; Taurine, Cystathionine, MCSO1/MCSO2, 0.868; Taurine, Cystathionine, MeCys, 0.868; Taurine, GABA, N6-AcLys, 0.868; Gln, Taurine, bAiBA, 0.868; Taurine, Ile, bAiBA, 0.868; Taurine, Phe, bA BA, 0.868; Taurine, bABA, Cystathionine, 0.868; Taurine, bA BA, GABA, 0.868; Asn, Taurine, ADMA, 0.868; Gln, Taurine, H ypro, 0.868; Gln, Taurine, N6-AcLys, 0.868; Taurine, His, C it, 0.868; Taurine, His, Hypotaurine, 0.868; Taurine, Cit, GABA, 0.868; Taurine, Cit, MeCys, 0.868; Taurine, Arg, hArg, 0.868; Taurine, Tyr, ADMA, 0.868; Taurine, Val, Hypotaurin e, 0.868; Taurine, Met, MCSO2, 0.868; Taurine, GABA, Hypota urine, 0.868; Taurine, GABA, MCSO2/MeCys, 0.868; Taurine, H ypotaurine, Hypro, 0.868; Taurine, Kyn, MCSO2/MeCys, 0.868; Taurine, Phe, Thioproline, 0.868; Taurine, bAiBA, Hypotaur ine, 0.868; Taurine, bAiBA, Hypro, 0.868; Taurine, MCSO1/MC SO2, Thioproline, 0.868; Glu, Taurine, Kyn, 0.868; Asn, Tau rine, Cit, 0.868; Gln, Taurine, Tyr, 0.868; Gln, Taurine, H ypotaurine, 0.868; Taurine, Thr, Cystathionine, 0.868; Taur ine, Arg, Tyr, 0.868; Taurine, Arg, N6-AcLys, 0.868; Taurin e, aABA, GABA, 0.868; Taurine, Val, Trp, 0.868; Taurine, Va l, hArg, 0.868; Taurine, Orn, Cystathionine, 0.868; Taurine, Ile, Hypotaurine, 0.868; Taurine, Phe, MCSO1/MCSO2, 0.868; Taurine, MCSO1, N8-AcSpd, 0.868; Taurine, bAiBA, N8-AcSpd, 0.868; Glu, Taurine, Kyn/Trp, 0.867; Glu, Taurine, MCSO2/M eCys, 0.867; Ser, Asn, Taurine, 0.867; Ser, Gly, Taurine, 0. 867; Ser, Taurine, His, 0.867; Ser, Taurine, Phe, 0.867; Se r, Taurine, Hypro, 0.867; Asn, Gly, Taurine, 0.867; Asn, Gl n, Taurine, 0.867; Gly, Gln, Taurine, 0.867; Taurine, His, Met, 0.867; Taurine, Ala, MeCys, 0.867; Taurine, Arg, GABA, 0.867; Taurine, Tyr, Orn, 0.867; Taurine, Tyr, Hypotaurine, 0.867; Taurine, Val, ADMA, 0.867; Taurine, Trp, MCSO1/MCSO 2, 0.867; Taurine, ADMA, GABA, 0.867; Taurine, Cystathionin e, MCSO1, 0.867; Taurine, Tyr, Thioproline, 0.867; Taurine, bABA, N8-AcSpd, 0.867; Glu, Taurine, Put, 0.867; Asn, Taur ine, N6-AcLys, 0.867; Taurine, Ala, Tyr, 0.867; Taurine, Ar g, Phe, 0.867; Taurine, Arg, Trp, 0.867; Taurine, Arg, ADMA, 0.867; Taurine, Pro, MeCys, 0.867; Taurine, Val, N6-AcLys, 0.867; Taurine, Orn, Hypro, 0.867; Taurine, Lys, Ile, 0.86 7; Taurine, Ile, Phe, 0.867; Taurine, Ile, N6-AcLys, 0.867; Taurine, Phe, GABA, 0.867; Taurine, Phe, hArg, 0.867; Taur ine, Trp, Hypro, 0.867; Taurine, Hypotaurine, MCSO1/MCSO2, 0.867; Taurine, Hypotaurine, N6-AcLys, 0.867; Taurine, His, bAiBA, 0.867; Taurine, His, Thioproline, 0.867; Taurine, T yr, bAiBA, 0.867; Taurine, Val, bAiBA, 0.867; Taurine, MeCy s, Thioproline, 0.867; Ser, Taurine, bABA, 0.867; Ser, Taur ine, Trp, 0.867; Asn, Taurine, GABA, 0.867; Gln, Taurine, V al, 0.867; Gln, Taurine, Trp, 0.867; Taurine, His, GABA, 0. 867; Taurine, His, Hypro, 0.867; Taurine, Tyr, Phe, 0.867; Taurine, Tyr, GABA, 0.867; Taurine, Met, Ile, 0.867; Taurin e, Orn, GABA, 0.867; Taurine, Orn, N6-AcLys, 0.867; Taurine, Lys, GABA, 0.867; Taurine, hArg, MCSO1/MCSO2, 0.867; Tauri ne, MeCys, N8-AcSpd, 0.867; Asn, Taurine, bAiBA, 0.867; Tau rine, Trp, bAiBA, 0.867; Taurine, bAiBA, Cystathionine, 0.8 67; Glu, Taurine, 3-MeHis, 0.867; Glu, Taurine, SDMA, 0.86 7; Ser, Taurine, Arg, 0.867; Asn, Taurine, hArg, 0.867; Gly, Taurine, Hypotaurine, 0.867; Gln, Taurine, Arg, 0.867; Tau rine, His, Arg, 0.867; Taurine, His, Leu, 0.867; Taurine, C it, MCSO1, 0.867; Taurine, Arg, Hypotaurine, 0.867; Taurine, aABA, hArg, 0.867; Taurine, Met, MeCys, 0.867; Taurine, Me t, N6-AcLys, 0.867; Taurine, Orn, Ile, 0.867; Taurine, Lys, ADMA, 0.867; Taurine, Leu, N8-AcSpd, 0.867; Taurine, ADMA, N6-AcLys, 0.867; Taurine, MCSO1/MCSO2, N8-AcSpd, 0.867; Ta urine, MCSO2/MeCys, SDMA, 0.867; Taurine, bABA, bAiBA, 0.86 7; Taurine, Pipecolic acid, SDMA, 0.867; Taurine, Cystathio nine, Sar, 0.867; Asn, Taurine, Thioproline, 0.867; Gln, Ta urine, Thioproline, 0.867; Taurine, Orn, bAiBA, 0.867; Taur ine, ADMA, Thioproline, 0.867; Taurine, Cit, aAAA, 0.867; T aurine, Tyr, bABA, 0.867; Taurine, Ile, bABA, 0.867; Asn, T aurine, Val, 0.866; Asn, Taurine, Hypotaurine, 0.866; Gly, Taurine, Ile, 0.866; Gly, Taurine, Trp, 0.866; Gln, Taurine, hArg, 0.866; Taurine, His, Tyr, 0.866; Taurine, His, N6-Ac Lys, 0.866; Taurine, Thr, Put, 0.866; Taurine, Ala, GABA, 0. 866; Taurine, Arg, Hypro, 0.866; Taurine, Tyr, Trp, 0.866; Taurine, Val, Leu, 0.866; Taurine, Lys, Phe, 0.866; Taurine, Ile, Trp, 0.866; Taurine, Trp, Hypotaurine, 0.866; Taurine, Trp, MeCys, 0.866; Taurine, MCSO2, N6-AcLys, 0.866; Gly, T aurine, bAiBA, 0.866; Taurine, Arg, bAiBA, 0.866; Taurine, Met, Thioproline, 0.866; Ser, Taurine, Val, 0.866; Ser, Tau rine, N6-AcLys, 0.866; Asn, Taurine, Met, 0.866; Asn, Tauri ne, Trp, 0.866; Gly, Taurine, N6-AcLys, 0.866; Taurine, His, Phe, 0.866; Taurine, His, ADMA, 0.866; Taurine, Ala, hArg, 0.866; Taurine, Orn, N8-AcSpd, 0.866; Taurine, Lys, MCSO2/ MeCys, 0.866; Taurine, Lys, MeCys, 0.866; Taurine, Leu, MCS O1/MCSO2, 0.866; Taurine, hArg, Hypro, 0.866; Taurine, 3-Me His, Pipecolic acid, 0.866; Taurine, Kyn/BCAA, PEA, 0.866; Taurine, Ala, Thioproline, 0.866; Taurine, Phe, bAiBA, 0.86 6; Asn, Taurine, bABA, 0.866; Gly, Taurine, bABA, 0.866; Ta urine, Val, bABA, 0.866; Taurine, bABA, Hypotaurine, 0.866; Ser, Taurine, hArg, 0.866; Ser, Taurine, Hypotaurine, 0.86 6; Ser, Taurine, MeCys, 0.866; Gly, Taurine, Hypro, 0.866; Taurine, His, Trp, 0.866; Taurine, Tyr, Lys, 0.866; Taurine, Tyr, Leu, 0.866; Taurine, Met, hArg, 0.866; Taurine, Orn, ADMA, 0.866; Taurine, Phe, MCSO2/MeCys, 0.866; Taurine, Trp, N6-AcLys, 0.866; Taurine, 3-MeHis, MCSO2/MeCys, 0.866; Tau rine, MCSO1, MCSO1/MCSO2, 0.866; Gly, Taurine, Thioproline, 0.866; Taurine, bABA, N6-AcLys, 0.866; Asn, Taurine, MeCys, 0.866; Gly, Taurine, Val, 0.866; Taurine, Arg, Val, 0.866; Taurine, Val, Phe, 0.866; Taurine, Val, MeCys, 0.866; Taur ine, Met, ADMA, 0.866; Taurine, Lys, Hypro, 0.866; Taurine, Leu, Trp, 0.866; Taurine, Cystathionine, MCSO2/MeCys, 0.86 6; Taurine, Hypro, MeCys, 0.866; Taurine, bAiBA, N6-AcLys, 0.866; Taurine, Arg, bABA, 0.866; Taurine, bABA, hArg, 0.86 6; Taurine, bABA, MeCys, 0.866; Asn, Taurine, Lys, 0.865; A sn, Taurine, Phe, 0.865; Asn, Taurine, Hypro, 0.865; Gly, T aurine, Orn, 0.865; Gly, Taurine, Phe, 0.865; Taurine, His, Val, 0.865; Taurine, Thr, N8-AcSpd, 0.865; Taurine, Ala, T rp, 0.865; Taurine, aABA, Tyr, 0.865; Taurine, aABA, Phe, 0. 865; Taurine, Tyr, Val, 0.865; Taurine, Tyr, MeCys, 0.865; Taurine, Val, Hypro, 0.865; Taurine, Ile, MCSO1/MCSO2, 0.86 5; Taurine, Leu, hArg, 0.865; Taurine, Phe, MeCys, 0.865; T aurine, Trp, hArg, 0.865; Taurine, hArg, N8-AcSpd, 0.865; T aurine, Hypro, MCSO1/MCSO2, 0.865; Taurine, N6-AcLys, N8-Ac Spd, 0.865; Taurine, Val, Thioproline, 0.865; Gln, Taurine, bABA, 0.865; Taurine, bABA, Hypro, 0.865; Ser, Taurine, Me t, 0.865; Asn, Taurine, Ile, 0.865; Gly, Taurine, Arg, 0.86 5; Gly, Taurine, hArg, 0.865; Gly, Taurine, MCSO1/MCSO2, 0. 865; Gly, Taurine, MeCys, 0.865; Taurine, Ala, Arg, 0.865; Taurine, Ala, ADMA, 0.865; Taurine, Arg, MeCys, 0.865; Taur ine, Val, Ile, 0.865; Taurine, Met, MCSO2/MeCys, 0.865; Tau rine, Orn, MeCys, 0.865; Taurine, Lys, MCSO1/MCSO2, 0.865; Taurine, Kyn/Trp, N6-AcLys, 0.865; Taurine, Arg, Thioprolin e, 0.865; Ser, Taurine, MCSO1/MCSO2, 0.865; Asn, Taurine, A rg, 0.865; Gln, Taurine, Orn, 0.865; Taurine, His, Ala, 0.8 65; Taurine, Ala, Met, 0.865; Taurine, Cit, MCSO2/MeCys, 0. 865; Taurine, aABA, MCSO1/MCSO2, 0.865; Taurine, aABA, MeCy s, 0.865; Taurine, Val, MCSO2/MeCys, 0.865; Taurine, Met, H ypro, 0.865; Taurine, Orn, Phe, 0.865; Taurine, Ile, MeCys, 0.865; Taurine, Leu, Hypro, 0.865; Taurine, hArg, MCSO2/Me Cys, 0.865; Taurine, Trp, Thioproline, 0.865; Taurine, aAAA, MCSO2, 0.865; Taurine, His, bABA, 0.865; Taurine, Trp, bAB A, 0.865; Taurine, His, hArg, 0.865; Taurine, Thr, Cit, 0.8 65; Taurine, Ala, Ile, 0.865; Taurine, Ala, MCSO2/MeCys, 0. 865; Taurine, Arg, Met, 0.865; Taurine, Arg, MCSO1/MCSO2, 0. 865; Taurine, Pro, ADMA, 0.865; Taurine, aABA, Hypro, 0.86 5; Taurine, Val, Met, 0.865; Taurine, Val, Orn, 0.865; Taur ine, Met, Trp, 0.865; Taurine, Met, Hypotaurine, 0.865; Tau rine, Leu, N6-AcLys, 0.865; Taurine, Phe, MCSO1, 0.865; Tau rine, bAiBA, Thioproline, 0.865; Ser, Taurine, Thioproline, 0.865; Taurine, Ala, bAiBA, 0.865; Taurine, Pro, Thioproli ne, 0.865; Taurine, N6-AcLys, Thioproline, 0.865; Taurine, N8-AcSpd, Thioproline, 0.865; Taurine, bABA, MCSO2/MeCys, 0. 865; Ser, Taurine, aABA, 0.864; Gln, Taurine, Met, 0.864; T aurine, His, Thr, 0.864; Taurine, His, MeCys, 0.864; Taurin e, Pro, Met, 0.864; Taurine, Tyr, MCSO2/MeCys, 0.864; Tauri ne, Val, MCSO1/MCSO2, 0.864; Taurine, Met, Lys, 0.864; Taur ine, Orn, Trp, 0.864; Taurine, Phe, Trp, 0.864; Taurine, Cy stathionine, N6-AcLys, 0.864; Taurine, N6-AcLys, Sar, 0.86 4; Taurine, aABA, Thioproline, 0.864; Taurine, Met, bAiBA, 0.864; Taurine, Orn, Thioproline, 0.864; Taurine, Ile, Thio proline, 0.864; Taurine, Hypro, Thioproline, 0.864; Asn, Ta urine, Pro, 0.864; Gly, Taurine, His, 0.864; Gly, Taurine, MCSO2/MeCys, 0.864; Taurine, His, Orn, 0.864; Taurine, Ala, Orn, 0.864; Taurine, Ala, Hypotaurine, 0.864; Taurine, Arg, aABA, 0.864; Taurine, Leu, Hypotaurine, 0.864; Taurine, Ph e, N6-AcLys, 0.864; Taurine, Hypro, N6-AcLys, 0.864; Taurin e, MCSO2/MeCys, Thioproline, 0.864; Taurine, Sar, Thioproli ne, 0.864; Taurine, Cit, Sar, 0.864; Glu, Taurine, MeCys, 0. 864; Ser, Taurine, Ala, 0.864; Ser, Taurine, Orn, 0.864; Gl n, Taurine, Pro, 0.864; Taurine, His, Pro, 0.864; Taurine, Ala, MCSO1/MCSO2, 0.864; Taurine, Pro, Leu, 0.864; Taurine, Met, Orn, 0.864; Taurine, Orn, Hypotaurine, 0.864; Taurine, Orn, MCSO1/MCSO2, 0.864; Taurine, N6-AcLys, SDMA, 0.864; T aurine, Cysteic acid, Kyn/BCAA, 0.864; Taurine, Leu, Thiopr oline, 0.864; Taurine, bAiBA, MeCys, 0.864; Taurine, Hypota urine, Thioproline, 0.864; Taurine, Met, bABA, 0.864; Tauri ne, bABA, MCSO1/MCSO2, 0.864; Glu, Taurine, N8-AcSpd, 0.86 4; Asn, Taurine, aABA, 0.864; Gly, Taurine, Ala, 0.864; Gln, Taurine, MCSO1/MCSO2, 0.864; Taurine, Thr, MCSO2/MeCys, 0. 864; Taurine, Ala, N6-AcLys, 0.864; Taurine, Ala, N8-AcSpd, 0.864; Taurine, Pro, Phe, 0.864; Taurine, Pro, Trp, 0.864; Taurine, Pro, hArg, 0.864; Taurine, Tyr, MCSO1, 0.864; Tau rine, Orn, hArg, 0.864; Taurine, Hypotaurine, MCSO2, 0.864; Taurine, Kyn, N6-AcLys, 0.864; Taurine, MCSO1, MeCys, 0.86 4; Taurine, MCSO2, Sar, 0.864; Taurine, bABA, Thioproline, 0.864; Taurine, MCSO2, Pipecolic acid, 0.864; Ser, Taurine, Pro, 0.864; Ser, Taurine, Leu, 0.864; Gly, Taurine, Met, 0. 864; Gln, Taurine, MeCys, 0.864; Taurine, Thr, N6-AcLys, 0. 864; Taurine, Ala, Val, 0.864; Taurine, Ala, Hypro, 0.864; Taurine, Arg, Orn, 0.864; Taurine, Pro, Hypotaurine, 0.864; Taurine, aABA, Met, 0.864; Taurine, Tyr, Met, 0.864; Tauri ne, Met, Phe, 0.864; Taurine, Trp, MCSO2/MeCys, 0.864; Taur ine, Hypro, MCSO2/MeCys, 0.864; Taurine, MCSO1/MCSO2, MCSO2 /MeCys, 0.864; Taurine, MCSO1/MCSO2, N6-AcLys, 0.864; Tauri ne, aAAA, Cystathionine, 0.863; Taurine, aAAA, MCSO2/MeCys, 0.863; Asn, Taurine, Ala, 0.863; Asn, Taurine, Orn, 0.863; Gln, Taurine, His, 0.863; Gln, Taurine, Ala, 0.863; Gln, T aurine, Leu, 0.863; Taurine, Thr, GABA, 0.863; Taurine, Ala, aABA, 0.863; Taurine, Arg, Leu, 0.863; Taurine, aABA, Trp, 0.863; Taurine, MeCys, N6-AcLys, 0.863; Gln, Taurine, aAAA, 0.863; Gln, Taurine, aABA, 0.863; Taurine, Ala, Phe, 0.86 3; Taurine, Pro, Tyr, 0.863; Taurine, aABA, ADMA, 0.863; Ta urine, Leu, MCSO1, 0.863; Taurine, GABA, MCSO1, 0.863; Taur ine, Lys, Thioproline, 0.863; Taurine, bAiBA, hArg, 0.863; Taurine, bAiBA, MCSO1/MCSO2, 0.863; Glu, Taurine, Cit, 0.86 3; Asn, Taurine, Leu, 0.863; Asn, Taurine, MCSO2/MeCys, 0.8 63; Gln, Taurine, MCSO2/MeCys, 0.863; Taurine, Arg, MCSO2/M eCys, 0.863; Taurine, Met, Leu, 0.863; Taurine, Lys, hArg, 0.863; Taurine, Ile, MCSO2/MeCys, 0.863; Taurine, hArg, MCS O1, 0.863; Taurine, bAiBA, MCSO1, 0.863; Taurine, His, aAAA, 0.863; Taurine, Tyr, aAAA, 0.863; Taurine, Cysteic acid, h Arg, 0.863; Gly, Taurine, Pro, 0.863; Gly, Taurine, Leu, 0. 863; Taurine, Arg, Pro, 0.863; Taurine, aABA, Hypotaurine, 0.863; Taurine, Orn, MCSO2/MeCys, 0.863; Taurine, Leu, ADMA, 0.863; Taurine, Ala, bABA, 0.863; Taurine, Pro, bABA, 0.86 3; Taurine, Orn, bABA, 0.863; Taurine, Kyn/Trp, Pipecolic a cid, 0.862; Taurine, GABA, Sar, 0.862; Taurine, MeCys, Sar, 0.862; Taurine, aABA, bAiBA, 0.862; Taurine, Arg, Lys, 0.8 62; Taurine, Pro, Val, 0.862; Taurine, aABA, Val, 0.862; Ta urine, aABA, Ile, 0.862; Taurine, Leu, bABA, 0.862; Taurine, PEA, SDMA, 0.862; Taurine, Pro, bAiBA, 0.862; Taurine, MCS O1, Thioproline, 0.862; Ser, Taurine, MCSO2/MeCys, 0.862; T aurine, His, aABA, 0.862; Taurine, Ala, Leu, 0.862; Taurine, Pro, Orn, 0.862; Taurine, Pro, Hypro, 0.862; Taurine, aABA, N6-AcLys, 0.862; Taurine, MCSO2/MeCys, N8-AcSpd, 0.862; Ta urine, Cysteic acid, Put, 0.862; Taurine, 3-MeHis, PEA, 0.8 62; Taurine, Thr, Thioproline, 0.862; Glu, Taurine, Cystath ionine, 0.862; Glu, Taurine, GABA, 0.862; Taurine, His, MCS O1/MCSO2, 0.862; Taurine, Thr, hArg, 0.862; Taurine, aABA, MCSO2/MeCys, 0.862; Taurine, Ile, MCSO1, 0.862; Taurine, Le u, MCSO2/MeCys, 0.862; Taurine, Hypro, MCSO1, 0.862; Taurin e, MCSO1, MCSO2/MeCys, 0.862; Taurine, aABA, bABA, 0.862; T aurine, Pipecolic acid, Put, 0.862; Taurine, Leu, bAiBA, 0. 862; Asn, Taurine, Thr, 0.862; Gly, Taurine, aABA, 0.862; T aurine, Thr, Arg, 0.862; Taurine, Pro, Ile, 0.862; Taurine, aABA, Lys, 0.862; Taurine, bAiBA, MCSO2/MeCys, 0.861; Gln, Taurine, MCSO1, 0.861; Taurine, His, Lys, 0.861; Taurine, Thr, Ala, 0.861; Taurine, Pro, GABA, 0.861; Taurine, aAAA, ADMA, 0.861; Taurine, Met, Sar, 0.861; Taurine, Hypotaurine, Sar, 0.861; Asn, Taurine, MCSO1/MCSO2, 0.861; Taurine, Met, MCSO1/MCSO2, 0.861; Taurine, Lys, Leu, 0.861; Taurine, MCS O2/MeCys, N6-AcLys, 0.861; Ser, Taurine, aAAA, 0.861; Tauri ne, ADMA, Sar, 0.861; Taurine, bAiBA, Sar, 0.861; Taurine, aABA, Orn, 0.861; Taurine, Lys, Trp, 0.861; Taurine, Lys, b AiBA, 0.861; Taurine, Kyn, Pipecolic acid, 0.861; Taurine, Ile, aAAA, 0.861; Taurine, Phe, aAAA, 0.861; Taurine, aAAA, Hypro, 0.861; Gly, Taurine, MCSO1, 0.861; Taurine, Thr, Va l, 0.861; Taurine, Thr, ADMA, 0.861; Taurine, Thr, Hypro, 0. 861; Taurine, Pro, N8-AcSpd, 0.861; Taurine, aABA, MCSO1, 0. 861; Taurine, Val, Lys, 0.861; Taurine, Orn, Leu, 0.861; Ta urine, aAAA, bAiBA, 0.861; Taurine, Arg, Sar, 0.86; Glu, Ta urine, Thioproline, 0.86; Ser, Taurine, MCSO1, 0.86; Taurin e, Val, MCSO1, 0.86; Taurine, Orn, Lys, 0.86; Taurine, Orn, MCSO1, 0.86; Taurine, ADMA, MCSO2/MeCys, 0.86; Taurine, Cy steic acid, Kyn, 0.86; Taurine, Trp, Sar, 0.86; Taurine, Ly s, bABA, 0.86; Taurine, aAAA, Put, 0.86; Ser, Taurine, Sar, 0.86; Glu, Taurine, aABA, 0.86; Glu, Taurine, Tyr, 0.86; T aurine, Thr, aABA, 0.86; Taurine, Thr, Orn, 0.86; Taurine, Thr, Trp, 0.86; Taurine, Arg, MCSO1, 0.86; Taurine, Val, aA AA, 0.86; Taurine, aAAA, GABA, 0.86; Gly, Taurine, Lys, 0.8 6; Taurine, His, MCSO2/MeCys, 0.86; Taurine, Thr, Tyr, 0.8 6; Taurine, Ala, Pro, 0.86; Taurine, aABA, Leu, 0.86; Tauri ne, Met, MCSO1, 0.86; Taurine, Lys, Hypotaurine, 0.86; Taur ine, aAAA, MCSO1/MCSO2, 0.859; Taurine, aAAA, Thioproline, 0.859; Glu, Taurine, ADMA, 0.859; Ser, Taurine, Lys, 0.859; Taurine, Cit, N6-AcLys, 0.859; Taurine, Cit, Pipecolic aci d, 0.859; Taurine, bABA, MCSO1, 0.859; Asn, Taurine, aAAA, 0.859; Taurine, Trp, aAAA, 0.859; Taurine, aAAA, N6-AcLys, 0.859; Taurine, Kyn/Trp, PEA, 0.859; Taurine, Thr, bAiBA, 0. 859; Taurine, Pro, MCSO2/MeCys, 0.859; Taurine, Hypotaurine, MCSO1, 0.859; Taurine, Hypotaurine, MCSO2/MeCys, 0.859; Ta urine, Hypotaurine, MeCys, 0.859; 1-MeHis, N8-AcSpd, Spd, 0. 859; Glu, Taurine, bABA, 0.859; Taurine, Cysteic acid, MCSO 2/MeCys, 0.859; Taurine, Cysteic acid, SDMA, 0.859; Taurine, Tyr, Sar, 0.859; Taurine, Phe, Sar, 0.859; Taurine, Thr, P ro, 0.859; Taurine, Thr, Phe, 0.859; Taurine, Thr, Hypotaur ine, 0.859; Taurine, Thr, MCSO1/MCSO2, 0.859; Taurine, Ala, Lys, 0.859; Taurine, Pro, aABA, 0.859; Taurine, ADMA, MCSO 1, 0.859; Taurine, Thr, bABA, 0.859; Taurine, Kyn, PEA, 0.8 59; Glu, Gly, Taurine, 0.859; Glu, Taurine, Phe, 0.859; Tau rine, Pro, MCSO1/MCSO2, 0.859; Taurine, Lys, MCSO1, 0.859; Gln, Taurine, Sar, 0.859; Taurine, MCSO1/MCSO2, Sar, 0.859; Taurine, N8-AcSpd, Pipecolic acid, 0.858; Taurine, Cysteic acid, Kyn/Trp, 0.858; Taurine, Cysteic acid, MeCys, 0.858; Glu, Taurine, Arg, 0.858; Glu, Taurine, Hypro, 0.858; Asn, Taurine, MCSO1, 0.858; Taurine, His, MCSO1, 0.858; Gly, Ta urine, aAAA, 0.858; Taurine, Arg, aAAA, 0.858; Taurine, Put, Sar, 0.858; Glu, Taurine, Met, 0.858; Gly, Taurine, Thr, 0. 858; Taurine, Thr, Leu, 0.858; Taurine, bABA, Sar, 0.858; T aurine, Met, aAAA, 0.858; Glu, Taurine, bAiBA, 0.858; Glu, Ser, Taurine, 0.858; Glu, Taurine, Orn, 0.858; Glu, Taurine, hArg, 0.858; Taurine, Thr, Met, 0.858; Taurine, Thr, Ile, 0.858; Taurine, Pro, Lys, 0.858; Taurine, Trp, MCSO1, 0.85 8; Taurine, aAAA, MeCys, 0.858; Taurine, Cystathionine, Pip ecolic acid, 0.858; Glu, Taurine, MCSO1/MCSO2, 0.858; 1-MeH is, MCSO1/MCSO2, Spd, 0.858; Taurine, Ala, aAAA, 0.857; Tau rine, aAAA, Hypotaurine, 0.857; Gln, Taurine, Thr, 0.857; T aurine, aABA, Cysteic acid, 0.857; Asn, Taurine, Sar, 0.85 7; Gly, Taurine, Sar, 0.857; Taurine, Val, Sar, 0.857; Taur ine, aAAA, bABA, 0.857; Glu, Taurine, His, 0.857; Taurine, MCSO1, N6-AcLys, 0.857; Taurine, Ile, Sar, 0.857; Glu, Asn, Taurine, 0.857; Taurine, Orn, Sar, 0.857; Taurine, aAAA, h Arg, 0.857; Taurine, N8-AcSpd, PEA, 0.857; Taurine, Thr, MC SO1, 0.856; Taurine, N8-AcSpd, Sar, 0.856; Glu, Gln, Taurin e, 0.856; Taurine, MCSO2, PEA, 0.856; Glu, Taurine, MCSO1, 0.856; Gln, Taurine, Lys, 0.856; Taurine, Thr, Lys, 0.856; Taurine, Orn, aAAA, 0.856; Taurine, hArg, Sar, 0.856; Tauri ne, Hypro, Sar, 0.856; Glu, Taurine, Trp, 0.855; Glu, Tauri ne, N6-AcLys, 0.855; Taurine, Ala, MCSO1,0.855; Taurine, a ABA, aAAA, 0.855; Glu, Taurine, Ile, 0.855; Ser, Taurine, T hr, 0.855; Taurine, Pipecolic acid, Thioproline, 0.855; Tau rine, Trp, Pipecolic acid, 0.855; Taurine, Lys, Cysteic aci d, 0.855; Taurine, Cysteic acid, N8-AcSpd, 0.855; Glu, Taur ine, Lys, 0.855; Taurine, Pro, aAAA, 0.855; Taurine, aAAA, MCSO1,0.855; Taurine, hArg, Pipecolic acid, 0.855; Taurine, Cit, PEA, 0.854; Taurine, Leu, aAAA, 0.854; Glu, Taurine, Val, 0.854; Glu, Taurine, Leu, 0.854; Taurine, Thr, Cysteic acid, 0.854; Taurine, GABA, Pipecolic acid, 0.854; Taurine, Hypotaurine, Pipecolic acid, 0.854; Taurine, Phe, Pipecoli c acid, 0.853; Taurine, Ala, Sar, 0.853; Gln, Taurine, Pipe colic acid, 0.853; Taurine, His, PEA, 0.853; Glu, Taurine, Ala, 0.853; Glu, Taurine, Hypotaurine, 0.853; Taurine, Hypr o, Pipecolic acid, 0.853; Taurine, bAiBA, Pipecolic acid, 0. 853; Taurine, Leu, Sar, 0.853; Ser, Taurine, Pipecolic acid, 0.853; Taurine, Met, Pipecolic acid, 0.853; Taurine, Lys, Pipecolic acid, 0.853; Taurine, MeCys, Pipecolic acid, 0.85 3; Taurine, GABA, PEA, 0.853; Taurine, Cysteic acid, GABA, 0.853; Taurine, His, Sar, 0.852; Gly, Taurine, Pipecolic ac id, 0.852; Taurine, Tyr, Pipecolic acid, 0.852; Taurine, Va l, Pipecolic acid, 0.852; Taurine, PEA, Put, 0.852; Taurine, MCSO1/MCSO2, Pipecolic acid, 0.852; Taurine, aABA, Sar, 0. 852; Glu, Taurine, Thr, 0.852; Glu, Taurine, Pro, 0.852; Ta urine, Ile, Pipecolic acid, 0.852; Taurine, Leu, Cysteic ac id, 0.852; 1-MeHis, MCSO2, Spd, 0.852; Taurine, Thr, aAAA, 0.852; Asn, Taurine, Pipecolic acid, 0.851; Taurine, Leu, P ipecolic acid, 0.851; Taurine, ADMA, Pipecolic acid, 0.851; Taurine, hArg, PEA, 0.851; Taurine, ADMA, PEA, 0.851; Taur ine, aABA, Pipecolic acid, 0.851; Taurine, His, Cysteic aci d, 0.851; Taurine, MeCys, PEA, 0.851; Taurine, Pro, MCSO1, 0.851; Taurine, Cystathionine, PEA, 0.851; MCSO2, N8-AcSpd, Put, 0.851; Taurine, Pro, Sar, 0.851; Taurine, Tyr, PEA, 0. 85; Taurine, MCSO2/MeCys, PEA, 0.85; Taurine, N6-AcLys, PEA, 0.85; Taurine, Ala, Pipecolic acid, 0.85; Taurine, MCSO2/M eCys, Pipecolic acid, 0.85; Taurine, bABA, Pipecolic acid, 0.85; Taurine, Lys, aAAA, 0.85; Taurine, Arg, PEA, 0.85; As n, Taurine, Cysteic acid, 0.85; Taurine, Orn, Cysteic acid, 0.85; Taurine, Trp, PEA, 0.85; Taurine, Val, Cysteic acid, 0.85; Taurine, Cysteic acid, Thioproline, 0.85; Ser, Tauri ne, PEA, 0.85; Asn, Taurine, PEA, 0.85; Taurine, Phe, PEA, 0.849; Taurine, Hypotaurine, PEA, 0.849; Taurine, His, Pipe colic acid, 0.849; Taurine, Lys, Sar, 0.849; Taurine, Met, Cysteic acid, 0.849; Taurine, 3-MeHis, Cysteic acid, 0.849; Gln, Taurine, PEA, 0.849; Taurine, Ile, PEA, 0.849; Taurin e, bAiBA, PEA, 0.849; Taurine, Arg, Pipecolic acid, 0.849; Taurine, Cystathionine, Cysteic acid, 0.849; Taurine, Thr, Sar, 0.849; 1-MeHis, Kyn/BCAA, Spd, 0.849; Gly, Taurine, PE A, 0.849; Taurine, PEA, Thioproline, 0.849; Taurine, Cit, C ysteic acid, 0.848; Taurine, Lys, PEA, 0.848; Taurine, Orn, Pipecolic acid, 0.848; Taurine, bABA, PEA, 0.848; Taurine, Tyr, Cysteic acid, 0.848; Taurine, Phe, Cysteic acid, 0.84 8; Taurine, N6-AcLys, Pipecolic acid, 0.848; Taurine, MCSO2 /MeCys, Sar, 0.848; Taurine, Hypro, PEA, 0.848; Taurine, Pr o, Pipecolic acid, 0.847; Taurine, Ile, Cysteic acid, 0.84 7; Taurine, Val, PEA, 0.847; Taurine, Orn, PEA, 0.847; Taur ine, MCSO1,Pipecolic acid, 0.847; Taurine, Cysteic acid, M CSO1, 0.847; Taurine, Met, PEA, 0.847; Ser, Taurine, Cystei c acid, 0.847; Gly, Taurine, Cysteic acid, 0.847; Gln, Taur ine, Cysteic acid, 0.847; Taurine, aAAA, Sar, 0.847; 1-MeHi s, MCSO2/MeCys, Spd, 0.847; Taurine, Ala, Cysteic acid, 0.8 46; Taurine, Cysteic acid, Hypro, 0.846; Taurine, Leu, PEA, 0.846; Glu, Taurine, aAAA, 0.846; Taurine, Arg, Cysteic ac id, 0.846; Taurine, Trp, Cysteic acid, 0.846; Taurine, MCSO 1, PEA, 0.846; Taurine, Cysteic acid, Hypotaurine, 0.846; T aurine, bABA, Cysteic acid, 0.846; Taurine, Ala, PEA, 0.84 5; Taurine, MCSO1,Sar, 0.845; Cit, 1-MeHis, Spd, 0.845; Ta urine, Pro, PEA, 0.845; 1-MeHis, Spd, Thioproline, 0.845; T aurine, Cysteic acid, N6-AcLys, 0.845; Glu, Taurine, Sar, 0. 844; Taurine, Thr, Pipecolic acid, 0.844; Taurine, bAiBA, C ysteic acid, 0.844; Taurine, ADMA, Cysteic acid, 0.844; Tau rine, aAAA, Pipecolic acid, 0.844; Taurine, Pro, Cysteic ac id, 0.843; Taurine, Thr, PEA, 0.843; Glu, Taurine, Cysteic acid, 0.842; Taurine, Pipecolic acid, Sar, 0.842; 1-MeHis, Kyn/Trp, Spd, 0.842; Taurine, aABA, PEA, 0.842; 1-MeHis, Ky n, Spd, 0.841; Taurine, MCSO1/MCSO2, PEA, 0.84; 1-MeHis, Pi pecolic acid, Spd, 0.84; Taurine, aAAA, Cysteic acid, 0.83 9; aABA, 1-MeHis, Spd, 0.839; Gln, 1-MeHis, Spd, 0.839; Glu, Taurine, Pipecolic acid, 0.839; Glu, Taurine, PEA, 0.838; 1-MeHis, hArg, Spd, 0.838; Val, 1-MeHis, Spd, 0.838; Taurin e, aAAA, PEA, 0.837; Pro, 1-MeHis, Spd, 0.837; 1-MeHis, SDM A, Spd, 0.837; 1-MeHis, GABA, Spd, 0.837; Taurine, Cysteic acid, PEA, 0.837; Thr, 1-MeHis, Spd, 0.837; Ile, 1-MeHis, S pd, 0.836; MCSO1/MCSO2, Spd, Thioproline, 0.836; His, 1-MeH is, Spd, 0.836; MCSO2, Put, Spd, 0.836; Leu, 1-MeHis, Spd, 0.835; 1-MeHis, Hypro, Spd, 0.835; MCSO2, N8-AcSpd, Spd, 0. 835; Asn, 1-MeHis, Spd, 0.835; 1-MeHis, MeCys, Spd, 0.835; Kyn/BCAA, MCSO1/MCSO2, Spd, 0.834; 1-MeHis, N6-AcLys, Spd, 0.834; 1-MeHis, bAiBA, Spd, 0.834; Met, 1-MeHis, Spd, 0.83 3; Trp, 1-MeHis, Spd, 0.833; 1-MeHis, Cystathionine, Spd, 0. 833; MCSO2, Spd, Thioproline, 0.833; Taurine, Cysteic acid, Pipecolic acid, 0.833; Arg, 1-MeHis, Spd, 0.833; Phe, 1-Me His, Spd, 0.833; Kyn/BCAA, MCSO2, Spd, 0.833; Ser, 1-MeHis, Spd, 0.833; Cit, Pro, Spd, 0.832; Gly, 1-MeHis, Spd, 0.83 2; Ala, 1-MeHis, Spd, 0.832; Taurine, PEA, Pipecolic acid, 0.832; Taurine, Cysteic acid, Sar, 0.832; Lys, MCSO2, Spd, 0.832; aAAA, N8-AcSpd, Spd, 0.832; Lys, 1-MeHis, Spd, 0.83 2; 1-MeHis, bABA, Spd, 0.831; 1-MeHis, 3-MeHis, Spd, 0.831; MCSO1/MCSO2, N8-AcSpd, Spd, 0.831; 1-MeHis, ADMA, Spd, 0.8 31; MCSO2, Put, SDMA, 0.83; Taurine, MCSO2, Spm, 0.83; N8-A cSpd, Spd, Thioproline, 0.83; 1-MeHis, aAAA, Spd, 0.83; hAr g, MCSO2, Put, 0.83; MCSO2/MeCys, N8-AcSpd, Put, 0.829; Cit, MCSO2, Spd, 0.829; hArg, MCSO2, Spd, 0.829; MCSO2, SDMA, S pd, 0.829; 1-MeHis, MCSO1,Spd, 0.829; Kyn, MCSO1/MCSO2, Sp d, 0.829; Tyr, 1-MeHis, Spd, 0.829; MCSO2, Pipecolic acid, Spd, 0.829; MCSO1,MCSO1/MCSO2, Spd, 0.829; N8-AcSpd, Pipec olic acid, Spd, 0.828; aABA, MCSO2, Spd, 0.828; Orn, 1-MeHi s, Spd, 0.828; Kyn/Trp, MCSO2, Spd, 0.828; Cit, Pipecolic a cid, Spd, 0.828; Ser, MCSO2, Spd, 0.828; MCSO2, MCSO1,Spd, 0.828; Phe, MCSO1/MCSO2, Spd, 0.827; GABA, N8-AcSpd, Spd, 0.827; Kyn/Trp, MCSO1/MCSO2, Spd, 0.827; SDMA, Spd, Thiopro line, 0.827; Kyn, MCSO2, Spd, 0.827; Taurine, PEA, Sar, 0.8 27; Thr, MCSO2, Spd, 0.827; 1-MeHis, MCSO2, Put, 0.827; Kyn /BCAA, Spd, Thioproline, 0.826; Pipecolic acid, SDMA, Spd, 0.826; Gln, MCSO2, Spd, 0.826; MCSO2/MeCys, N8-AcSpd, Spd, 0.826; Kyn, Spd, Thioproline, 0.826; bABA, MCSO2, Spd, 0.82 6; Pro, MCSO1/MCSO2, Spd, 0.826; MCSO2, MCSO1/MCSO2, Spd, 0. 826; Leu, MCSO2, Spd, 0.826; GABA, MCSO2, Spd, 0.826; MeCys, N8-AcSpd, Put, 0.826; MeCys, N8-AcSpd, Spd, 0.826; 1-MeHis, Put, Spd, 0.825; N8-AcSpd, Put, Spd, 0.825; Thr, MCSO2/MeC ys, Spm, 0.825; Cit, Ile, Spd, 0.825; Cit, MCSO1/MCSO2, Spd, 0.825; His, MCSO2, Spd, 0.825; Trp, MCSO2, Spd, 0.825; hAr g, MeCys, Put, 0.825; Val, MCSO2, Spd, 0.824; Kyn, N8-AcSpd, Spd, 0.824; Kyn/BCAA, MCSO2, Put, 0.824; MeCys, SDMA, Spd, 0.824; Cit, Spd, Thioproline, 0.824; Trp, MCSO1/MCSO2, Spd, 0.824; hArg, MeCys, Spd, 0.824; MCSO1/MCSO2, Pipecolic aci d, Spd, 0.824; 3-MeHis, MCSO1/MCSO2, Spd, 0.824; Cystathion ine, MCSO1/MCSO2, Spd, 0.824; Kyn/BCAA, Pipecolic acid, Spd, 0.824; Gln, N8-AcSpd, Spd, 0.824; Cit, N8-AcSpd, Spd, 0.82 4; Pro, Hypro, Spd, 0.824; Ile, MCSO2, Spd, 0.824; Kyn/Trp, Spd, Thioproline, 0.823; Thr, MCSO2/MeCys, Spd, 0.823; Arg, MCSO1/MCSO2, Spd, 0.823; Phe, MCSO2, Spd, 0.823; Trp, N8-A cSpd, Spd, 0.823; MCSO2, MCSO1/MCSO2, Put, 0.823; Ile, MCSO 1/MCSO2, Spd, 0.823; Kyn/Trp, MCSO2, Put, 0.823; MCSO2, MCS O2/MeCys, Spd, 0.823; MCSO2, N6-AcLys, SDMA, 0.823; aAAA, K yn/BCAA, Spd, 0.823; Cit, Val, Spd, 0.823; Pro, MCSO2, Spd, 0.823; aABA, MCSO1/MCSO2, Spd, 0.823; Tyr, MCSO2, Spd, 0.8 23; MCSO1/MCSO2, N6-AcLys, Spd, 0.823; bAiBA, N8-AcSpd, Spd, 0.823; Kyn/Trp, Pipecolic acid, Spd, 0.823; Lys, MCSO2/MeC ys, Spd, 0.823; ADMA, MCSO1/MCSO2, Spd, 0.823; Glu, 1-MeHis, Spd, 0.823; Cit, Kyn/BCAA, Spd, 0.822; Tyr, MCSO1/MCSO2, S pd, 0.822; 3-MeHis, MCSO2, Spd, 0.822; Trp, Spd, Thioprolin e, 0.822; aABA, MCSO2, Put, 0.822; Orn, MCSO2, Spd, 0.822; Kyn/BCAA, MeCys, Spd, 0.822; MCSO1/MCSO2, MCSO2/MeCys, Spd, 0.822;Hypro, MCSO2, Spd, 0.822; Hypro, N8-AcSpd, Spd, 0.82 2; Pro, Spd, Thioproline, 0.822; Leu, Kyn, Spd, 0.822; hArg, MCSO2/MeCys, Spd, 0.822; MCSO2/MeCys, MeCys, Spd, 0.822; M CSO2/MeCys, Pipecolic acid, Spd, 0.822; GABA, MCSO1/MCSO2, Spd, 0.822; Ser, N8-AcSpd, Spd, 0.821; Ala, MCSO1/MCSO2, Sp d, 0.821; aABA, Spd, Thioproline, 0.821; Phe, N8-AcSpd, Spd, 0.821; MCSO2, N6-AcLys, Spd, 0.821; ADMA, N8-AcSpd, Spd, 0. 821; Ser, MCSO1/MCSO2, Spd, 0.821; Arg, MCSO2, Spd, 0.821; Met, MCSO2, Spd, 0.821; Kyn/Trp, N8-AcSpd, Spd, 0.821; aAAA, Kyn, Spd, 0.821; Lys, MCSO2, Put, 0.821; Leu, SDMA, Spd, 0. 821; MCSO1/MCSO2, SDMA, Spd, 0.821; Ala, Spd, Thioproline, 0.821; bAiBA, MCSO1/MCSO2, Spd, 0.821; aAAA, Spd, Thioproli ne, 0.821; Cysteic acid, MCSO1/MCSO2, Spd, 0.821; His, Cit, Spd, 0.821; Cit, Leu, Spd, 0.821; Cystathionine, MCSO2, Sp d, 0.821; Kyn/BCAA, SDMA, Spd, 0.821; Gly, MCSO2, Spd, 0.8 2; Leu, Spd, Thioproline, 0.82; Tyr, Pipecolic acid, Spd, 0. 82; Kyn, Pipecolic acid, Spd, 0.82; Gln, Pro, Spd, 0.82; Ty r, N8-AcSpd, Spd, 0.82; 1-MeHis, Hypotaurine, Spd, 0.82; MC SO2, Put, Sar, 0.82; Gln, MCSO1/MCSO2, Spd, 0.82; Arg, N8-A cSpd, Spd, 0.82; Leu, MCSO1/MCSO2, Spd, 0.82; ADMA, MCSO2, Spd, 0.82; 1-MeHis, MCSO2, Spm, 0.82; Cit, MCSO2, Put, 0.8 2; Pro, Cystathionine, Spd, 0.82; Pipecolic acid, Spd, Thio proline, 0.82; Asn, MCSO2, Spd, 0.819; Pro, N8-AcSpd, Spd, 0.819; aABA, N8-AcSpd, Spd, 0.819; Ala, MCSO2, Spd, 0.819; Cit, ADMA, Spd, 0.819; Val, N8-AcSpd, Spd, 0.819; Cystathio nine, N8-AcSpd, Spd, 0.819; MCSO2, MeCys, Spd, 0.819; N8-Ac Spd, SDMA, Spd, 0.819; Tyr, Spd, Thioproline, 0.819; Ile, S pd, Thioproline, 0.819; MeCys, Spd, Thioproline, 0.819; aAA A, bAiBA, Spd, 0.819; Ala, MCSO2, Put, 0.819; Cit, Tyr, Spd, 0.819; Cit, Cystathionine, Spd, 0.819; Cit, Hypro, Spd, 0. 819; Ile, N8-AcSpd, Spd, 0.819; MCSO2/MeCys, Put, Spd, 0.81 9; MCSO2/MeCys, Spd, Thioproline, 0.819; Glu, MCSO2, Spd, 0. 819; His, MCSO1/MCSO2, Spd, 0.819; Cit, Trp, Spd, 0.819; Ci t, GABA, Spd, 0.819; Cit, MeCys, Spd, 0.819; Ile, Kyn, Spd, 0.819; Pro, aAAA, Spd, 0.819; Ser, Cit, Spd, 0.818; Cit, P he, Spd, 0.818; Pro, aABA, Spd, 0.818; aABA, Kyn/BCAA, Spd, 0.818; Orn, MCSO1/MCSO2, Spd, 0.818; bAiBA, MCSO2, Spd, 0. 818; Cystathionine, Spd, Thioproline, 0.818; Val, Pipecolic acid, Spd, 0.818; aABA, aAAA, Spd, 0.818; aAAA, MCSO2, Spd, 0.818; MeCys, Put, SDMA, 0.818; MeCys, Put, Spd, 0.818; aA AA, Pipecolic acid, Spd, 0.818; Cit, 3-MeHis, Spd, 0.818; C it, Kyn, Spd, 0.818; Val, Kyn/BCAA, Spd, 0.818; Val, MCSO1/ MCSO2, Spd, 0.818; Val, SDMA, Spd, 0.818; Ile, SDMA, Spd, 0. 818; hArg, MCSO1/MCSO2, Spd, 0.818; MCSO1/MCSO2, Put, Spd, 0.818; Cit, aAAA, Spd, 0.818; Ile, Hypro, Spd, 0.818; Leu, Phe, Spd, 0.818; Leu, Hypro, Spd, 0.818; bABA, N8-AcSpd, Sp d, 0.818; Trp, Pipecolic acid, Spd, 0.818; GABA, Pipecolic acid, Spd, 0.818; Ala, Cit, Spd, 0.818; Pro, SDMA, Spd, 0.8 18; Kyn, MeCys, Spd, 0.818; Asn, Spd, Thioproline, 0.817; M et, Spd, Thioproline, 0.817; N6-AcLys, N8-AcSpd, Spd, 0.81 7; Val, aAAA, Spd, 0.817; Cit, SDMA, Spd, 0.817; GABA, SDMA, Spd, 0.817; MCSO1/MCSO2, MeCys, Spd, 0.817; Val, Spd, Thio proline, 0.817; Phe, Spd, Thioproline, 0.817; Cit, bABA, Sp d, 0.817; aAAA, GABA, Spd, 0.817; Gly, N8-AcSpd, Spd, 0.81 7; Tyr, MCSO2, Put, 0.817; Orn, N8-AcSpd, Spd, 0.817; Kyn/B CAA, MCSO2/MeCys, Spd, 0.817; Trp, aAAA, Spd, 0.817; Cit, b AiBA, Spd, 0.817; Orn, Spd, Thioproline, 0.817; Hypro, Spd, Thioproline, 0.817; bABA, MCSO1/MCSO2, Spd, 0.817; Ile, Pi pecolic acid, Spd, 0.817; Phe, Pipecolic acid, Spd, 0.817; His, MCSO2, Put, 0.817; 3-MeHis, SDMA, Spd, 0.817; Hypro, M CSO1/MCSO2, Spd, 0.817; Gln, aAAA, Spd, 0.817; Ile, aAAA, S pd, 0.817; aAAA, Hypro, Spd, 0.817; Hypro, Pipecolic acid, Spd, 0.817; Gly, MCSO2, Put, 0.817; Gln, Kyn/BCAA, Spd, 0.8 17; Thr, Cit, Spd, 0.817; aABA, Trp, Spd, 0.817; Kyn/Trp, M eCys, Spd, 0.817; MCSO2, MCSO1,Put, 0.817; Ser, Spd, Thiop roline, 0.816; GABA, Spd, Thioproline, 0.816; aABA, Pipecol ic acid, Spd, 0.816; Leu, Pipecolic acid, Spd, 0.816; Asn, N8-AcSpd, Spd, 0.816; Gln, Leu, Spd, 0.816; Arg, MCSO2, Put, 0.816; aABA, Leu, Spd, 0.816; 3-MeHis, Spd, Thioproline, 0. 816; N6-AcLys, Spd, Thioproline, 0.816; Ser, aAAA, Spd, 0.8 16; aABA, Ile, Spd, 0.816; aABA, Cystathionine, Spd, 0.816; aABA, Hypro, Spd, 0.816; Phe, Kyn/BCAA, Spd, 0.816; Kyn, K yn/BCAA, Spd, 0.816; ADMA, Spd, Thioproline, 0.816; bAiBA, Kyn, Spd, 0.816; 3-MeHis, aAAA, Spd, 0.816; Asn, MCSO1/MCSO 2, Spd, 0.816; Gln, Ile, Spd, 0.816; Cit, Met, Spd, 0.816; aABA, Val, Spd, 0.816; aABA, Phe, Spd, 0.816; aABA, 3-MeHis, Spd, 0.816; Leu, MCSO2/MeCys, Spd, 0.816; Cystathionine, K yn/BCAA, Spd, 0.816; GABA, Kyn/BCAA, Spd, 0.816; MCSO2/MeCy s, MeCys, Put, 0.816; Cit, aABA, Spd, 0.816; Pro, Kyn/BCAA, Spd, 0.816; Lys, MCSO1/MCSO2, Spd, 0.816; Trp, Kyn/BCAA, S pd, 0.816; GABA, Kyn, Spd, 0.816; Hypro, Kyn/BCAA, Spd, 0.8 16; Kyn/Trp, Kyn/BCAA, Spd, 0.816; Kyn/BCAA, N8-AcSpd, Spd, 0.816; bABA, MCSO2, Put, 0.815; GABA, MCSO2/MeCys, Spd, 0. 815; Gln, Cit, Spd, 0.815; Ala, N8-AcSpd, Spd, 0.815; Pro, Leu, Spd, 0.815; Pro, MCSO2/MeCys, Spd, 0.815; Val, Kyn, Sp d, 0.815; Ile, Cystathionine, Spd, 0.815; Leu, N8-AcSpd, Sp d, 0.815; Gln, Pipecolic acid, Spd, 0.815; Pro, Pipecolic a cid, Spd, 0.815; Cystathionine, Pipecolic acid, Spd, 0.815; Gln, Spd, Thioproline, 0.815; Cysteic acid, MCSO2, Spd, 0. 815; Ser, aABA, Spd, 0.815; Gln, aABA, Spd, 0.815; Cit, Orn, Spd, 0.815; Met, MCSO1/MCSO2, Spd, 0.815; Met, N8-AcSpd, S pd, 0.815; bAiBA, Pipecolic acid, Spd, 0.815; Ser, Kyn/BCAA, Spd, 0.815; Cit, hArg, Spd, 0.815; Pro, Tyr, Spd, 0.815; P ro, Ile, Spd, 0.815; Tyr, Kyn, Spd, 0.815; Tyr, Kyn/BCAA, S pd, 0.815; Orn, MCSO2/MeCys, Spd, 0.815; Orn, SDMA, Spd, 0. 815; Ile, GABA, Spd, 0.815; Leu, Trp, Spd, 0.815; Ser, Pipe colic acid, Spd, 0.815; 3-MeHis, Pipecolic acid, Spd, 0.81 5; bAiBA, MCSO2, Put, 0.815; Cit, Kyn/Trp, Spd, 0.815; Pro, Val, Spd, 0.815; Pro, Trp, Spd, 0.815; Pro, 3-MeHis, Spd, 0.815; Val, Hypro, Spd, 0.815; Ile, 3-MeHis, Spd, 0.815; Il e, Kyn/BCAA, Spd, 0.815; Leu, Kyn/BCAA, Spd, 0.815; 3-MeHis, Kyn/BCAA, Spd, 0.815; MCSO2/MeCys, SDMA, Spd, 0.815; Ala, Pipecolic acid, Spd, 0.814; aAAA, SDMA, Spd, 0.814; Gln, Al a, Spd, 0.814; Arg, Kyn, Spd, 0.814; Orn, Kyn, Spd, 0.814; Lys, Kyn, Spd, 0.814; Trp, MCSO2, Put, 0.814; 1-MeHis, MCSO 2/MeCys, Spm, 0.814; aAAA, Cystathionine, Spd, 0.814; aAAA, N6-AcLys, Spd, 0.814; Ser, MeCys, Spd, 0.814; Gln, Hypro, Spd, 0.814; Arg, MCSO2/MeCys, Spd, 0.814; aABA, MeCys, Spd, 0.814; Tyr, Ile, Spd, 0.814; Phe, Kyn, Spd, 0.814; 3-MeHis, Hypro, Spd, 0.814; Cystathionine, Kyn, Spd, 0.814; Cystath ionine, MCSO2, Put, 0.814; hArg, Spd, Thioproline, 0.814; P ro, ADMA, Spd, 0.814; Pro, GABA, Spd, 0.814; aABA, Kyn, Spd, 0.814; ADMA, Kyn/BCAA, Spd, 0.814; hArg, N8-AcSpd, Spd, 0. 814; Hypotaurine, MCSO2, Spd, 0.814; aAAA, ADMA, Spd, 0.81 4; Asn, Pipecolic acid, Spd, 0.814; Leu, aAAA, Spd, 0.814; aAAA, MCSO1/MCSO2, Spd, 0.814; Ser, Pro, Spd, 0.814; Gly, C it, Spd, 0.814; Gln, Cystathionine, Spd, 0.814; Gln, Kyn, S pd, 0.814; Ala, Cystathionine, Spd, 0.814; aABA, ADMA, Spd, 0.814; Ile, Phe, Spd, 0.814; Cystathionine, Hypro, Spd, 0. 814; Hypro, Kyn, Spd, 0.814; Hypro, N6-AcLys, Spd, 0.814; K yn, Kyn/Trp, Spd, 0.814; Kyn, MCSO2/MeCys, Spd, 0.814; Kyn, SDMA, Spd, 0.814; Arg, Spd, Thioproline, 0.813; aABA, bAiB A, Spd, 0.813; MCSO2, PEA, Put, 0.813; Ser, GABA, Spd, 0.81 3; Gly, SDMA, Spd, 0.813; Gln, Trp, Spd, 0.813; Ala, SDMA, Spd, 0.813; Cit, MCSO2/MeCys, Spd, 0.813; Pro, Kyn, Spd, 0. 813; aABA, SDMA, Spd, 0.813; Tyr, Val, Spd, 0.813; Tyr, Hyp ro, Spd, 0.813; Val, Trp, Spd, 0.813; Trp, Hypro, Spd, 0.81 3; 3-MeHis, N8-AcSpd, Spd, 0.813; Kyn, MCSO2, Put, 0.813; K yn/Trp, MCSO2/MeCys, Spd, 0.813; Tyr, aAAA, Spd, 0.813; aAA A, Kyn/Trp, Spd, 0.813; bAiBA, Kyn/BCAA, Spd, 0.813; aABA, bABA, Spd, 0.813; bAiBA, Spd, Thioproline, 0.813; Ser, Gln, Spd, 0.813; Gln, His, Spd, 0.813; Gln, Val, Spd, 0.813; Gl n, 3-MeHis, Spd, 0.813; His, MeCys, Spd, 0.813; His, N8-AcS pd, Spd, 0.813; 3-MeHis, Cystathionine, Spd, 0.813; MCSO2, N6-AcLys, Put, 0.813; bAiBA, SDMA, Spd, 0.813; Gln, Tyr, Sp d, 0.813; His, MCSO2/MeCys, Spd, 0.813; Tyr, Cystathionine, Spd, 0.813; Met, MCSO2/MeCys, Spd, 0.813; Orn, Kyn/BCAA, S pd, 0.813; Leu, MCSO2, Put, 0.813; Trp, Cystathionine, Spd, 0.813; Hypro, MCSO2, Put, 0.813; Phe, aAAA, Spd, 0.813; Ta urine, MCSO2/MeCys, Spm, 0.813; Gln, bAiBA, Spd, 0.813; bAB A, Spd, Thioproline, 0.813; Ser, Kyn, Spd, 0.813; Gln, ADMA, Spd, 0.813; Arg, Kyn/BCAA, Spd, 0.813; Pro, N6-AcLys, Spd, 0.813; Tyr, Phe, Spd, 0.813; Ile, N6-AcLys, Spd, 0.813; Ph e, GABA, Spd, 0.813; GABA, Kyn/Trp, Spd, 0.813; Hypotaurine, MCSO1/MCSO2, Spd, 0.813; Met, Pipecolic acid, Spd, 0.812; Ser, Trp, Spd, 0.812; Asn, Kyn, Spd, 0.812; Thr, Kyn, Spd, 0.812; Ile, Trp, Spd, 0.812; Leu, Cystathionine, Spd, 0.81 2; Leu, MeCys, Spd, 0.812; Phe, ADMA, Spd, 0.812; ADMA, Cys tathionine, Spd, 0.812; GABA, MCSO2, Put, 0.812; Thr, Spd, Thioproline, 0.812; Gln, GABA, Spd, 0.812; Gln, SDMA, Spd, 0.812; Thr, SDMA, Spd, 0.812; Val, 3-MeHis, Spd, 0.812; Val, Cystathionine, Spd, 0.812; Val, GABA, Spd, 0.812; Pro, bAB A, Spd, 0.812; Asn, Kyn/BCAA, Spd, 0.812; Gly, GABA, Spd, 0. 812; Gln, MCSO2, Put, 0.812; aABA, Tyr, Spd, 0.812; Tyr, Tr p, Spd, 0.812; Met, Kyn, Spd, 0.812; Orn, MCSO2, Put, 0.81 2; Lys, MeCys, Spd, 0.812; Ile, MCSO2, Put, 0.812; Hypotaur ine, MCSO2, Put, 0.812; bABA, Pipecolic acid, Spd, 0.812; A sn, aAAA, Spd, 0.812; Gln, MeCys, Spd, 0.812; His, aABA, Sp d, 0.812; Pro, Kyn/Trp, Spd, 0.812; Pro, MCSO2, Put, 0.812; Val, Ile, Spd, 0.812; Trp, Kyn, Spd, 0.812; MCSO2, MCSO2/M eCys, Put, 0.812; 1-MeHis, MCSO2/MeCys, Put, 0.812; ADMA, G ABA, Spd, 0.812; Val, bAiBA, Spd, 0.812; bAiBA, Cystathioni ne, Spd, 0.812; bAiBA, Hypro, Spd, 0.812; MCSO2, Put, Thiop roline, 0.812; aAAA, bABA, Spd, 0.811; Asn, Pro, Spd, 0.81 1; Gly, Kyn/BCAA, Spd, 0.811; Gln, N6-AcLys, Spd, 0.811; Ty r, 3-MeHis, Spd, 0.811; Ile, MCSO2/MeCys, Spd, 0.811; Phe, Cystathionine, Spd, 0.811; Phe, MCSO2, Put, 0.811; 3-MeHis, GABA, Spd, 0.811; ADMA, Hypro, Spd, 0.811; Cystathionine, SDMA, Spd, 0.811; hArg, Kyn/BCAA, Spd, 0.811; Pro, bAiBA, S pd, 0.811; Ile, bAiBA, Spd, 0.811; bABA, Kyn/BCAA, Spd, 0.8 11; bABA, MCSO2/MeCys, Spd, 0.811; Gly, MCSO1/MCSO2, Spd, 0. 811; Ala, aABA, Spd, 0.811; Tyr, N6-AcLys, Spd, 0.811; Val, Phe, Spd, 0.811; Lys, N8-AcSpd, Spd, 0.811; Ile, Kyn/Trp, Spd, 0.811; Leu, ADMA, Spd, 0.811; ADMA, Kyn/Trp, Spd, 0.81 1; ADMA, MCSO2, Put, 0.811; MCSO1,N8-AcSpd, Spd, 0.811; Hi s, aAAA, Spd, 0.811; N6-AcLys, Pipecolic acid, Spd, 0.811; bAiBA, GABA, Spd, 0.811; bABA, GABA, Spd, 0.811; bABA, Kyn, Spd, 0.811; Ser, Ile, Spd, 0.811; Ser, Leu, Spd, 0.811; Gl n, Phe, Spd, 0.811; His, Pro, Spd, 0.811; Thr, MCSO1/MCSO2, Spd, 0.811; aABA, Met, Spd, 0.811; aABA, MCSO2/MeCys, Spd, 0.811; Tyr, Leu, Spd, 0.811; ADMA, N6-AcLys, Spd, 0.811; H ypotaurine, Kyn/BCAA, Spd, 0.811; MCSO2/MeCys, N6-AcLys, Sp d, 0.811; Put, SDMA, Spd, 0.811; Lys, Spd, Thioproline, 0.8 11; Gln, bABA, Spd, 0.811; Ser, SDMA, Spd, 0.811; Thr, Cyst athionine, Spd, 0.811; Ala, Pro, Spd, 0.811; Arg, Pro, Spd, 0.811; Pro, hArg, Spd, 0.811; Pro, Hypotaurine, Spd, 0.81 1; Tyr, MCSO2/MeCys, Spd, 0.811; Val, ADMA, Spd, 0.811; Met, Kyn/BCAA, Spd, 0.811; Leu, 3-MeHis, Spd, 0.811; Phe, 3-MeH is, Spd, 0.811; 3-MeHis, MCSO2, Put, 0.811; Cystathionine, N6-AcLys, Spd, 0.811; MeCys, Pipecolic acid, Spd, 0.811; Hi s, Spd, Thioproline, 0.811; bABA, Hypro, Spd, 0.81; Ser, As n, Spd, 0.81; Cit, Lys, Spd, 0.81; Pro, Phe, Spd, 0.81; Val, Leu, Spd, 0.81; Met, Leu, Spd, 0.81; Leu, GABA, Spd, 0.81; Phe, Trp, Spd, 0.81; Trp, 3-MeHis, Spd, 0.81; Trp, Kyn/Trp, Spd, 0.81; Trp, SDMA, Spd, 0.81; Hypro, MCSO2/MeCys, Spd, 0.81; MCSO1,MCSO2/MeCys, Spd, 0.81; MeCys, N6-AcLys, Spd, 0.81; N6-AcLys, SDMA, Spd, 0.81; Gly, Spd, Thioproline, 0.8 1; Tyr, bAiBA, Spd, 0.81; Trp, bAiBA, Spd, 0.81; Ile, bABA, Spd, 0.81; Glu, N8-AcSpd, Spd, 0.81; His, Phe, Spd, 0.81; Val, MeCys, Spd, 0.81; Phe, Hypro, Spd, 0.81; Phe, SDMA, Sp d, 0.81; 3-MeHis, Kyn, Spd, 0.81; MCSO2, MeCys, Put, 0.81; Sar, SDMA, Spd, 0.81; Ser, Cystathionine, Spd, 0.81; Ser, H ypro, Spd, 0.81; Ala, MCSO2/MeCys, Spd, 0.81; Arg, aABA, Sp d, 0.81; Val, N6-AcLys, Spd, 0.81; Met, SDMA, Spd, 0.81; Tr p, GABA, Spd, 0.81; ADMA, SDMA, Spd, 0.81; Cystathionine, G ABA, Spd, 0.81; bABA, MCSO2, Spm, 0.81; Phe, bAiBA, Spd, 0. 81; 3-MeHis, bAiBA, Spd, 0.81; Glu, MCSO2, Put, 0.81; Ser, Tyr, Spd, 0.81; Ser, MCSO2, Put, 0.81; Ser, MCSO2/MeCys, Sp d, 0.81; Gly, aABA, Spd, 0.81; Gly, Kyn, Spd, 0.81; His, Cy stathionine, Spd, 0.81; Thr, MCSO2, Put, 0.81; Thr, N8-AcSp d, Spd, 0.81; Ala, Kyn, Spd, 0.81; Val, Kyn/Trp, Spd, 0.81; Val, MCSO2, Put, 0.81; Hypro, Kyn/Trp, Spd, 0.81; MCSO1,M eCys, Spd, 0.81; Cysteic acid, MCSO2, Put, 0.81; Orn, Pipec olic acid, Spd, 0.809; Glu, MCSO1/MCSO2, Spd, 0.809; Asn, V al, Spd, 0.809; Gly, Pro, Spd, 0.809; Cit, N6-AcLys, Spd, 0. 809; Phe, MCSO2/MeCys, Spd, 0.809; Hypro, SDMA, Spd, 0.809; Ser, bAiBA, Spd, 0.809; Put, Spd, Thioproline, 0.809; ADMA, Pipecolic acid, Spd, 0.809; Ser, Val, Spd, 0.809; Ser, ADM A, Spd, 0.809; Tyr, SDMA, Spd, 0.809; Orn, Kyn/Trp, Spd, 0. 809; 1-MeHis, Cysteic acid, Spd, 0.809; Ala, aAAA, Spd, 0.8 09; Val, bABA, Spd, 0.809; Thr, Pipecolic acid, Spd, 0.809; Ser, 3-MeHis, Spd, 0.809; Asn, Hypro, Spd, 0.809; Ala, Kyn /BCAA, Spd, 0.809; aABA, GABA, Spd, 0.809; Met, Hypro, Spd, 0.809; Orn, Phe, Spd, 0.809; Ile, ADMA, Spd, 0.809; ADMA, Kyn, Spd, 0.809; Kyn/Trp, SDMA, Spd, 0.809; Asn, His, Spd, 0.809; Asn, aABA, Spd, 0.809; Asn, MCSO2, Put, 0.809; Thr, MeCys, Spd, 0.809; Ala, Hypro, Spd, 0.809; Cit, hArg, MCSO2, 0.809; aABA, N6-AcLys, Spd, 0.809; Tyr, ADMA, Spd, 0.809; Tyr, GABA, Spd, 0.809; Met, MCSO2, Put, 0.809; Phe, MeCys, Spd, 0.809; Trp, ADMA, Spd, 0.809; Trp, MCSO2/MeCys, Spd, 0. 809; Cystathionine, MCSO2/MeCys, Spd, 0.809; Hypotaurine, N 8-AcSpd, Spd, 0.809; Met, aAAA, Spd, 0.809; Trp, bABA, Spd, 0.808; bABA, N6-AcLys, Spd, 0.808; His, GABA, Spd, 0.808; Met, Kyn/Trp, Spd, 0.808; Ile, MeCys, Spd, 0.808; Hypro, Me Cys, Spd, 0.808; His, Pipecolic acid, Spd, 0.808; 1-MeHis, Kyn/BCAA, PEA, 0.808; Ser, Kyn/Trp, Spd, 0.808; Asn, Leu, S pd, 0.808; Gln, Kyn/Trp, Spd, 0.808; Gln, MCSO2/MeCys, Spd, 0.808; His, Kyn/BCAA, Spd, 0.808; Val, MCSO2/MeCys, Spd, 0. 808; 3-MeHis, ADMA, Spd, 0.808; Cystathionine, MeCys, Spd, 0.808; hArg, Pipecolic acid, Spd, 0.808; MCSO2, Pipecolic a cid, Put, 0.808; Thr, MCSO2, Spm, 0.808; bAiBA, N6-AcLys, S pd, 0.808; His, hArg, Spd, 0.808; Ala, Trp, Spd, 0.808; aAB A, Kyn/Trp, Spd, 0.808; Tyr, Kyn/Trp, Spd, 0.808; bABA, SDM A, Spd, 0.808; Gly, Pipecolic acid, Spd, 0.808; Ser, Phe, S pd, 0.808; Asn, Thr, Spd, 0.808; Asn, Trp, Spd, 0.808; Gly, Val, Spd, 0.808; Pro, Met, Spd, 0.808; Pro, Orn, Spd, 0.80 8; Leu, bAiBA, Spd, 0.808; Asn, Met, Spd, 0.807; Asn, Ile, Spd, 0.807; His, Kyn, Spd, 0.807; Thr, Kyn/Trp, Spd, 0.807; Ala, Ile, Spd, 0.807; Ala, ADMA, Spd, 0.807; Pro, hArg, MC SO2, 0.807; Lys, SDMA, Spd, 0.807; ADMA, MCSO2/MeCys, Spd, 0.807; hArg, Hypro, Spd, 0.807; Kyn/BCAA, N6-AcLys, Spd, 0. 807; MCSO1/MCSO2, MCSO2/MeCys, Put, 0.807; Gly, aAAA, Spd, 0.807; MCSO2, PEA, Spm, 0.807; MCSO1,Spd, Thioproline, 0.8 07; His, Hypro, Spd, 0.807; Lys, MCSO2/MeCys, Put, 0.807; L eu, N6-AcLys, Spd, 0.807; Trp, MeCys, Spd, 0.807; Trp, N6-A cLys, Spd, 0.807; MCSO1, MCSO1/MCSO2, Put, 0.807; Asn, MeCy s, Spd, 0.807; Gly, hArg, MCSO2, 0.807; Gly, Kyn/Trp, Spd, 0.807; Gln, Lys, Spd, 0.807; Gln, hArg, Spd, 0.807; Thr, Ky n/BCAA, Spd, 0.807; Ala, Leu, Spd, 0.807; Arg, Val, Spd, 0. 807; Arg, Leu, Spd, 0.807; Tyr, MeCys, Spd, 0.807; ADMA, Me Cys, Spd, 0.807; Cystathionine, Kyn/Trp, Spd, 0.807; GABA, MeCys, Spd, 0.807; MCSO1,SDMA, Spd, 0.807; bABA, Cystathio nine, Spd, 0.807; Asn, Phe, Spd, 0.807; Asn, 3-MeHis, Spd, 0.807; Asn, ADMA, Spd, 0.807; Gly, Ile, Spd, 0.807; Ala, 3-MeHis, Spd, 0.807; Ala, Kyn/Trp, Spd, 0.807; Ile, Leu, Spd, 0.807; Phe, Kyn/Trp, Spd, 0.807; 1-MeHis, MCSO2, PEA, 0.80 7; Lys, Pipecolic acid, Spd, 0.807; Arg, aAAA, Spd, 0.807; Asn, Cit, Spd, 0.807; Asn, MCSO2/MeCys, Spd, 0.807; Gly, Ph e, Spd, 0.807; His, 3-MeHis, Spd, 0.807; Cit, Hypotaurine, Spd, 0.807; Hypotaurine, Pipecolic acid, Spd, 0.806; bAiBA, Kyn/Trp, Spd, 0.806; Ser, N6-AcLys, Spd, 0.806; Asn, GABA, Spd, 0.806; Gly, Leu, Spd, 0.806; Gly, Hypro, Spd, 0.806; Ala, Tyr, Spd, 0.806; Ala, Val, Spd, 0.806; Ala, hArg, Spd, 0.806; Met, MeCys, Spd, 0.806; 3-MeHis, MCSO2/MeCys, Spd, 0.806; hArg, Kyn, Spd, 0.806; Kyn/Trp, MCSO2, N6-AcLys, 0.8 06; bABA, MeCys, Spd, 0.806; Glu, Kyn/BCAA, Spd, 0.806; Gly, MeCys, Spd, 0.806; Gln, Hypotaurine, Spd, 0.806; His, Arg, Spd, 0.806; His, Trp, Spd, 0.806; His, ADMA, Spd, 0.806; T hr, MCSO2/MeCys, Put, 0.806; Met, GABA, Spd, 0.806; Kyn/BCA A, Put, Spd, 0.806; Glu, Spd, Thioproline, 0.806; Ser, Ala, Spd, 0.806; Asn, Tyr, Spd, 0.806; Ala, Phe, Spd, 0.806; Ar g, GABA, Spd, 0.806; aABA, Orn, Spd, 0.806; aABA, hArg, Spd, 0.806; Lys, Phe, Spd, 0.806; 3-MeHis, MeCys, Spd, 0.806; T yr, bABA, Spd, 0.806; GABA, N6-AcLys, Spd, 0.806; bABA, bAi BA, Spd, 0.806; MCSO2, Sar, Spd, 0.806; Ser, Gly, Spd, 0.80 6; Gly, MCSO2/MeCys, Spd, 0.806; Gln, Met, Spd, 0.806; His, Val, Spd, 0.806; GABA, hArg, Spd, 0.806; Leu, bABA, Spd, 0. 805; ADMA, bAiBA, Spd, 0.805; Glu, MCSO2/MeCys, Spd, 0.805; Ser, His, Spd, 0.805; Gly, Cystathionine, Spd, 0.805; Arg, 3-MeHis, Spd, 0.805; Leu, hArg, Spd, 0.805; Leu, Kyn/Trp, Spd, 0.805; ADMA, hArg, Spd, 0.805; Asn, bAiBA, Spd, 0.805; Ser, Met, Spd, 0.805; Asn, Gln, Spd, 0.805; His, Ile, Spd, 0.805; Ala, MeCys, Spd, 0.805; Arg, Hypro, Spd, 0.805; Val, Met, Spd, 0.805; hArg, MCSO2, MCSO1/MCSO2, 0.805; Phe, bAB A, Spd, 0.805; 3-MeHis, bABA, Spd, 0.805; aAAA, MeCys, Spd, 0.805; MCSO2, Sar, Spm, 0.805; Met, 3-MeHis, Spd, 0.805; O rn, Cystathionine, Spd, 0.805; hArg, Kyn/Trp, Spd, 0.805; S er, bABA, Spd, 0.805; Asn, Cystathionine, Spd, 0.805; Asn, N6-AcLys, Spd, 0.805; Gly, Trp, Spd, 0.805; Gly, 3-MeHis, S pd, 0.805; His, Leu, Spd, 0.805; Arg, Cystathionine, Spd, 0. 805; aABA, Hypotaurine, Spd, 0.805; aABA, MCSO1,Spd, 0.80 5; Val, hArg, Spd, 0.805; Orn, ADMA, Spd, 0.805; Trp, hArg, Spd, 0.805; hArg, N6-AcLys, Spd, 0.805; MeCys, N6-AcLys, S DMA, 0.805; Ala, bAiBA, Spd, 0.804; Hypotaurine, Spd, Thiop roline, 0.804; Gly, ADMA, Spd, 0.804; Cit, Arg, Spd, 0.804; Orn, 3-MeHis, Spd, 0.804; Orn, MeCys, Spd, 0.804; Lys, Kyn /BCAA, Spd, 0.804; GABA, Hypro, Spd, 0.804; Kyn/BCAA, MCSO1, Spd, 0.804; Asn, bABA, Spd, 0.804; bABA, Kyn/Trp, Spd, 0.8 04; bAiBA, MCSO2/MeCys, Spd, 0.804; Asn, Kyn/Trp, Spd, 0.80 4; Asn, SDMA, Spd, 0.804; Gly, Gln, Spd, 0.804; His, Met, S pd, 0.804; Ala, Met, Spd, 0.804; Arg, Tyr, Spd, 0.804; Arg, Met, Spd, 0.804; Arg, hArg, Spd, 0.804; Pro, MeCys, Spd, 0. 804; Met, Cystathionine, Spd, 0.804; Met, N6-AcLys, Spd, 0. 804; Phe, N6-AcLys, Spd, 0.804; Kyn/Trp, Put, Spd, 0.804; A rg, bAiBA, Spd, 0.804; Ser, Arg, Spd, 0.804; His, N6-AcLys, Spd, 0.804; Thr, Hypro, Spd, 0.804; Arg, N6-AcLys, Spd, 0. 804; Orn, GABA, Spd, 0.804; Lys, hArg, MCSO2, 0.804; Ile, H ypotaurine, Spd, 0.804; 3-MeHis, Hypotaurine, Spd, 0.804; L ys, 1-MeHis, MCSO2, 0.804; 1-MeHis, PEA, Spd, 0.804; Gly, T yr, Spd, 0.804; His, Tyr, Spd, 0.804; Arg, Ile, Spd, 0.804; Arg, MeCys, Spd, 0.804; Arg, SDMA, Spd, 0.804; Tyr, Hypota urine, Spd, 0.804; Lys, 3-MeHis, Spd, 0.804; Phe, hArg, Spd, 0.804; Cystathionine, hArg, Spd, 0.804; Taurine, MeCys, Sp m, 0.803; aAAA, MCSO1,Spd, 0.803; Asn, Arg, Spd, 0.803; Th r, Arg, Spd, 0.803; Arg, ADMA, Spd, 0.803; Tyr, Orn, Spd, 0. 803; Val, Hypotaurine, Spd, 0.803; Met, ADMA, Spd, 0.803; K yn, N6-AcLys, Spd, 0.803; Ala, bABA, Spd, 0.803; Ser, Orn, Spd, 0.803; Gln, Orn, Spd, 0.803; Cit, Lys, MCSO2, 0.803; O rn, Ile, Spd, 0.803; Orn, Trp, Spd, 0.803; 3-MeHis, N6-AcLy s, Spd, 0.803; hArg, SDMA, Spd, 0.803; Kyn/Trp, MeCys, Put, 0.803; MCSO2/MeCys, Put, SDMA, 0.803; His, bABA, Spd, 0.80 3; ADMA, bABA, Spd, 0.803; Arg, Pipecolic acid, Spd, 0.803; aAAA, MCSO2/MeCys, Spd, 0.803; Gly, N6-AcLys, Spd, 0.803; Thr, GABA, Spd, 0.803; Tyr, hArg, Spd, 0.803; Val, Orn, Spd, 0.803; Ile, hArg, Spd, 0.803; 3-MeHis, hArg, Spd, 0.803; h Arg, MCSO2/MeCys, Put, 0.803; bAiBA, Hypotaurine, Spd, 0.80 3; MCSO1,Pipecolic acid, Spd, 0.803; Thr, aAAA, Spd, 0.80 3; Ser, hArg, Spd, 0.803; Gln, Thr, Spd, 0.803; His, Hypota urine, Spd, 0.803; Thr, 3-MeHis, Spd, 0.803; Orn, Hypro, Sp d, 0.803; MCSO1/MCSO2, MeCys, Put, 0.803; Gly, bAiBA, Spd, 0.803; Glu, Kyn, Spd, 0.802; Ser, Lys, Spd, 0.802; Asn, hAr g, Spd, 0.802; Thr, Met, Spd, 0.802; aABA, Lys, Spd, 0.802; Met, Trp, Spd, 0.802; Orn, N6-AcLys, Spd, 0.802; Lys, GABA, Spd, 0.802; Hypotaurine, Kyn, Spd, 0.802; Kyn/Trp, MCSO1, Spd, 0.802; aAAA, hArg, MCSO2, 0.802; Thr, Leu, Spd, 0.802; Arg, Lys, Spd, 0.802; Leu, MCSO1,Spd, 0.802; Cystathionin e, Hypotaurine, Spd, 0.802; hArg, Hypotaurine, Spd, 0.802; hArg, MCSO2, SDMA, 0.802; Hypotaurine, Hypro, Spd, 0.802; M et, bAiBA, Spd, 0.802; 1-MeHis, MCSO2/MeCys, PEA, 0.802; Or n, aAAA, Spd, 0.802; Arg, Kyn/Trp, Spd, 0.802; Met, Orn, Sp d, 0.802; Met, hArg, Spd, 0.802; Orn, hArg, MCSO2, 0.802; L ys, Hypro, Spd, 0.802; Leu, Hypotaurine, Spd, 0.802; His, b AiBA, Spd, 0.802; bABA, Hypotaurine, Spd, 0.802; Thr, aABA, Spd, 0.802; Orn, bAiBA, Spd, 0.802; Asn, Lys, Spd, 0.801; Cit, Put, Spd, 0.801; Arg, Phe, Spd, 0.801; 3-MeHis, MCSO2, SDMA, 0.801; Kyn/Trp, N6-AcLys, Spd, 0.801; bAiBA, MeCys, Spd, 0.801; Met, bABA, Spd, 0.801; bABA, hArg, Spd, 0.801; aAAA, MCSO2, Put, 0.801; Asn, Gly, Spd, 0.801; Asn, Orn, Sp d, 0.801; Gly, Met, Spd, 0.801; His, Lys, Spd, 0.801; Arg, Orn, Spd, 0.801; Met, Phe, Spd, 0.801; Lys, Ile, Spd, 0.80 1; Kyn, Put, Spd, 0.801; bAiBA, hArg, Spd, 0.801; Glu, Kyn/ Trp, Spd, 0.801; Thr, Pro, Spd, 0.801; Cit, MCSO1,Spd, 0.8 01; Val, Lys, Spd, 0.801; MCSO2, PEA, Spd, 0.801; Thr, Val, Spd, 0.801; Thr, ADMA, Spd, 0.801; Met, Lys, Spd, 0.801; L ys, Leu, Spd, 0.801; bABA, hArg, MCSO2, 0.8; 1-MeHis, Sar, Spd, 0.8; Gly, Arg, Spd, 0.8; Gly, Orn, Spd, 0.8; Gln, Arg, Spd, 0.8; Ala, Arg, Spd, 0.8; Pro, Put, Spd, 0.8; Trp, Hyp otaurine, Spd, 0.8; ADMA, Hypotaurine, Spd, 0.8; hArg, Kyn, MCSO2, 0.8; hArg, MCSO2, N6-AcLys, 0.8; hArg, MCSO2, PEA, 0.8; Ser, Hypotaurine, Spd, 0.8; Asn, Ala, Spd, 0.8; Gly, H is, Spd, 0.8; His, SDMA, Spd, 0.8; Orn, Leu, Spd, 0.8; Orn, hArg, Spd, 0.8; Lys, Trp, Spd, 0.8; 3-MeHis, Kyn/Trp, Spd, 0.8; hArg, MCSO1,Spd, 0.8; hArg, Put, Spd, 0.8; MCSO2, MC SO1, Spm, 0.8; Ser, Thr, Spd, 0.8; Thr, hArg, Spd, 0.8; Arg, Trp, Spd, 0.8; Met, Ile, Spd, 0.8; Lys, Cystathionine, Spd, 0.8; Lys, MCSO2, MCSO1/MCSO2, 0.8; Kyn, MCSO1,Spd, 0.8; G ly, Ala, Spd, 0.8; Cit, MCSO2/MeCys, Put, 0.8; Arg, bABA, S pd, 0.8; Glu, hArg, MCSO2, 0.799; Ser, hArg, MCSO2, 0.799; Thr, Ile, Spd, 0.799; aABA, Put, Spd, 0.799; Lys, ADMA, Spd, 0.799; His, Kyn/Trp, Spd, 0.799; His, Put, Spd, 0.799; Thr, Phe, Spd, 0.799; Ala, Hypotaurine, Spd, 0.799; hArg, MCSO2 /MeCys, MeCys, 0.799; Glu, MeCys, Spd, 0.799; His, Ala, Spd, 0.799; Thr, Tyr, Spd, 0.799; Ala, hArg, MCSO2, 0.799; Lys, MCSO2, MCSO1,0.799; hArg, Kyn/BCAA, MCSO2, 0.799; Gly, Th r, Spd, 0.799; Thr, Ala, Spd, 0.799; Lys, hArg, Spd, 0.799; Lys, N6-AcLys, Spd, 0.799; Cystathionine, Put, Spd, 0.799; GABA, MCSO1,Spd, 0.799; hArg, MCSO2, MCSO1,0.799; Hypota urine, MCSO2/MeCys, Spd, 0.799; His, Thr, Spd, 0.798; Lys, Kyn/Trp, Spd, 0.798; Gln, Put, Spd, 0.798; Thr, Trp, Spd, 0. 798; Tyr, Lys, Spd, 0.798; His, MCSO1,Spd, 0.798; Thr, MCS O1, Spd, 0.798; Ile, MCSO1,Spd, 0.798; Phe, Hypotaurine, S pd, 0.798; Hypotaurine, N6-AcLys, Spd, 0.798; Kyn/Trp, MCSO 2/MeCys, Put, 0.798; His, Orn, Spd, 0.798; Thr, Put, Spd, 0. 798; Ala, Put, Spd, 0.798; hArg, Hypotaurine, MCSO2, 0.798; Ser, Lys, MCSO2, 0.797; Lys, MCSO2, SDMA, 0.797; Lys, MeCy s, Put, 0.797; Hypotaurine, MeCys, Spd, 0.797; Pro, MCSO1, Spd, 0.797; Tyr, Met, Spd, 0.797; Orn, Lys, Spd, 0.797; Phe, Put, Spd, 0.797; Ala, N6-AcLys, Spd, 0.797; Phe, MCSO1,Sp d, 0.797; GABA, Put, Spd, 0.797; hArg, Kyn/Trp, MCSO2, 0.79 7; Hypotaurine, SDMA, Spd, 0.797; Ala, Orn, Spd, 0.797; Pro, Lys, Spd, 0.797; Met, hArg, MCSO2, 0.797; Cystathionine, M CSO1, Spd, 0.797; Gln, MCSO1,Spd, 0.796; Ala, Lys, Spd, 0. 796; Val, MCSO1,Spd, 0.796; 3-MeHis, Put, Spd, 0.796; Ala, MCSO2/MeCys, Put, 0.796; Tyr, MCSO1,Spd, 0.796; GABA, Hyp otaurine, Spd, 0.796; Glu, SDMA, Spd, 0.796; Gly, Lys, Spd, 0.796; Gly, MCSO1,Spd, 0.796; Thr, Lys, Spd, 0.796; Ala, MCSO1,Spd, 0.796; His, hArg, MCSO2, 0.796; Ala, GABA, Spd, 0.796; Cit, MCSO2, N6-AcLys, 0.796; Lys, Kyn, MCSO2, 0.79 6; Ser, MCSO1,Spd, 0.795; Gln, hArg, MCSO2, 0.795; Thr, Or n, Spd, 0.795; Lys, MCSO1,Spd, 0.795; Cystathionine, hArg, MCSO2, 0.795; Asn, hArg, MCSO2, 0.795; Trp, MCSO1,Spd, 0. 795; His, MeCys, Put, 0.795; Tyr, hArg, MCSO2, 0.795; Val, Put, Spd, 0.795; Leu, Put, Spd, 0.795; Ser, Met, MCSO2, 0.7 95; Pro, MCSO2, SDMA, 0.795; GABA, hArg, MCSO2, 0.795; Ser, hArg, MeCys, 0.794; Pro, MCSO2/MeCys, Put, 0.794; 3-MeHis, MCSO1,Spd, 0.794; hArg, MCSO2, MCSO2/MeCys, 0.794; hArg, MCSO1/MCSO2, MeCys, 0.794; Leu, hArg, MCSO2, 0.794; Trp, hA rg, MCSO2, 0.794; Hypotaurine, Kyn/Trp, Spd, 0.794; Thr, hA rg, MCSO2, 0.794; Cit, Pro, MCSO2, 0.794; aABA, hArg, MCSO2, 0.794; 3-MeHis, hArg, MCSO2, 0.794; ADMA, hArg, MCSO2, 0.7 94; Kyn/BCAA, MCSO2/MeCys, Put, 0.794; MCSO1,Put, Spd, 0.7 94; Gly, MCSO2/MeCys, Put, 0.794; Val, hArg, MCSO2, 0.794; Met, MCSO1,Spd, 0.794; Lys, Put, Spd, 0.794; N6-AcLys, Put, Spd, 0.794; ADMA, Put, Spd, 0.793; Glu, GABA, Spd, 0.793; Ser, Put, Spd, 0.793; Asn, MCSO1,Spd, 0.793; Asn, Put, Spd, 0.793; Arg, Hypotaurine, Spd, 0.793; Lys, MCSO1/MCSO2, MCS O2/MeCys, 0.793; Lys, MCSO2/MeCys, MeCys, 0.793; Ile, hArg, MCSO2, 0.793; hArg, MCSO2, MeCys, 0.793; Glu, aABA, Spd, 0. 793; Asn, Hypotaurine, Spd, 0.793; Gly, hArg, MeCys, 0.793; Orn, MCSO1,Spd, 0.793; Phe, hArg, MCSO2, 0.793; hArg, MeC ys, N6-AcLys, 0.793; Cit, Met, MCSO2, 0.793; Cit, Orn, MCSO 2, 0.793; Arg, hArg, MCSO2, 0.793; Glu, Pro, Spd, 0.793; Ci t, hArg, MeCys, 0.793; Lys, Kyn/Trp, MCSO2, 0.793; Glu, Cit, Spd, 0.792; Gly, Hypotaurine, Spd, 0.792; Gln, Lys, MCSO2, 0.792; Ser, Thr, MCSO2, 0.792; Thr, N6-AcLys, Spd, 0.792; Cit, Arg, MCSO2, 0.792; Arg, MCSO1,Spd, 0.792; Arg, MeCys, Put, 0.792; Lys, MCSO2, MCSO2/MeCys, 0.792; Ile, Put, Spd, 0.792; Kyn/BCAA, MeCys, Put, 0.792; Glu, Val, Spd, 0.792; Glu, Phe, Spd, 0.792; Arg, MCSO2/MeCys, Put, 0.792; Tyr, Pu t, Spd, 0.792; Ala, hArg, MeCys, 0.792; Met, Hypotaurine, S pd, 0.792; Trp, Put, Spd, 0.792; GABA, MCSO2/MeCys, Put, 0. 791; Gly, hArg, Spd, 0.791; His, Lys, MCSO2, 0.791; Met, hA rg, MeCys, 0.791; Orn, Put, Spd, 0.791; Lys, GABA, MCSO2, 0. 791; Glu, Ile, Spd, 0.791; Gly, Put, Spd, 0.791; ADMA, MCSO 1, Spd, 0.791; Cystathionine, MCSO2/MeCys, Put, 0.791; Glu, Orn, Spd, 0.791; Trp, MCSO2/MeCys, Put, 0.791; Glu, Cystat hionine, Spd, 0.791; Met, Put, Spd, 0.791; hArg, MeCys, SDM A, 0.791; Tyr, Lys, MCSO2, 0.79; Val, Lys, MCSO2, 0.79; Met, MeCys, Put, 0.79; Glu, Put, Spd, 0.79; aABA, MeCys, Put, 0. 79; Met, MCSO2/MeCys, Put, 0.79; Orn, Lys, MCSO2, 0.79; Kyn, MCSO2/MeCys, Put, 0.79; Glu, Gly, Spd, 0.79; Ala, Lys, MCS O2, 0.79; Orn, MCSO2/MeCys, Put, 0.79; Lys, hArg, MeCys, 0. 79; Leu, MCSO2/MeCys, Put, 0.79; Kyn/Trp, MeCys, N6-AcLys, 0.79; aABA, Lys, MCSO2, 0.79; Lys, Hypotaurine, MCSO2, 0.7 9; Arg, Lys, MCSO2, 0.789; Lys, Cystathionine, MCSO2, 0.78 9; Glu, 3-MeHis, Spd, 0.789; Lys, ADMA, MCSO2, 0.789; Lys, Kyn/BCAA, MCSO2, 0.789; Asn, Lys, MCSO2, 0.789; His, Cit, M CSO2, 0.789; His, hArg, MeCys, 0.789; Cit, MeCys, Put, 0.78 9; Lys, Ile, MCSO2, 0.789; Hypotaurine, Put, Spd, 0.789; Th r, Lys, MCSO2, 0.789; Arg, Put, Spd, 0.789; Pro, Kyn/Trp, M CSO2, 0.789; Lys, Hypotaurine, Spd, 0.789; Lys, MCSO2, MeCy s, 0.789; Lys, MCSO2, N6-AcLys, 0.789; His, MCSO2/MeCys, Pu t, 0.788; Tyr, MCSO2/MeCys, Put, 0.788; Glu, Trp, Spd, 0.78 8; Gly, MeCys, Put, 0.788; Pro, hArg, MeCys, 0.788; hArg, M CSO1/MCSO2, MCSO2/MeCys, 0.788; Thr, Cit, MCSO2, 0.788; Met, MCSO2, MCSO1,0.788; hArg, Kyn/Trp, MeCys, 0.788; Glu, Lys, MCSO2, 0.788; Pro, Lys, MCSO2, 0.788; Lys, Leu, MCSO2, 0.7 88; Lys, Phe, MCSO2, 0.788; Glu, hArg, MeCys, 0.787; Tyr, M eCys, Put, 0.787; Met, MCSO2, MCSO1/MCSO2, 0.787; Lys, 3-Me His, MCSO2, 0.787; hArg, Kyn/BCAA, MeCys, 0.787; Lys, Trp, MCSO2, 0.787; ADMA, MCSO2/MeCys, Put, 0.787; Kyn, MeCys, Pu t, 0.787; MCSO1,N6-AcLys, Spd, 0.787; Glu, Arg, Spd, 0.78 7; Gly, Lys, MCSO2, 0.787; Tyr, MCSO2, N6-AcLys, 0.787; Phe, MCSO2/MeCys, Put, 0.787; Glu, Gln, Spd, 0.786; Glu, Lys, S pd, 0.786; Thr, Hypotaurine, Spd, 0.786; Pro, MCSO2, MCSO1, 0.786; Met, Lys, MCSO2, 0.786; Hypotaurine, MCSO2/MeCys, P ut, 0.786; Kyn/BCAA, MCSO2, N6-AcLys, 0.786; Gly, hArg, MCS O2/MeCys, 0.786; Ala, MCSO2, MCSO1/MCSO2, 0.786; Pro, MCSO2, MCSO1/MCSO2, 0.786; Glu, Met, Spd, 0.786; Thr, MeCys, Put, 0.786; Met, MCSO2, SDMA, 0.786; Glu, Ser, Spd, 0.785; hArg, Kyn, MeCys, 0.785; Orn, Hypotaurine, Spd, 0.785; MCSO2/MeC ys, N6-AcLys, SDMA, 0.785; Ala, MCSO2, MCSO1,0.785; Met, G ABA, MCSO2, 0.785; Cystathionine, MeCys, Put, 0.785; Glu, A la, Spd, 0.785; Glu, Tyr, Spd, 0.785; Asn, MCSO2/MeCys, Put, 0.785; Thr, MCSO2, N6-AcLys, 0.785; Cit, Lys, MCSO2/MeCys, 0.785; Tyr, hArg, MeCys, 0.785; MCSO1,MeCys, Put, 0.785; Glu, His, Spd, 0.784; Gln, MCSO2/MeCys, Put, 0.784; Trp, Me Cys, Put, 0.784; His, Pro, MCSO2, 0.784; Thr, MCSO2, MCSO1/ MCSO2, 0.784; MCSO2/MeCys, N6-AcLys, Put, 0.784; Glu, N6-Ac Lys, Spd, 0.784; Met, MCSO2, N6-AcLys, 0.784; Leu, MeCys, P ut, 0.784; MCSO2, MCSO1/MCSO2, N6-AcLys, 0.784; MCSO1,MCSO 2/MeCys, Put, 0.784; aABA, MCSO2/MeCys, Put, 0.784; Orn, Ky n/Trp, MCSO2, 0.784; Leu, MCSO2, MCSO1/MCSO2, 0.784; Phe, h Arg, MeCys, 0.784; Glu, ADMA, Spd, 0.784; Arg, hArg, MeCys, 0.784; Val, MCSO2/MeCys, Put, 0.784; Orn, hArg, MeCys, 0.7 84; Ile, MCSO2/MeCys, Put, 0.784; hArg, Hypotaurine, MeCys, 0.784; Glu, MCSO2/MeCys, Put, 0.783; 3-MeHis, MCSO2/MeCys, Put, 0.783; Glu, MeCys, Put, 0.783; Thr, Cit, MCSO2/MeCys, 0.783; 3-MeHis, MeCys, Put, 0.783; hArg, MCSO1,MeCys, 0.7 83; Glu, Thr, Spd, 0.783; His, MCSO2, MCSO1/MCSO2, 0.783; T hr, MCSO1/MCSO2, MCSO2/MeCys, 0.783; Pro, MeCys, Put, 0.78 3; Met, Orn, MCSO2, 0.783; Met, Kyn/BCAA, MCSO2, 0.783; Orn, Kyn/BCAA, MCSO2, 0.783; Kyn, MCSO2, N6-AcLys, 0.783; MeCys, N6-AcLys, Put, 0.783; Glu, Leu, Spd, 0.782; Gly, Cit, MCSO 2, 0.782; Thr, hArg, MeCys, 0.782; Orn, MeCys, Put, 0.782; Ser, MCSO2/MeCys, Put, 0.782; Gln, MeCys, Put, 0.782; Pro, hArg, MCSO2/MeCys, 0.782; Pro, Kyn/BCAA, MCSO2, 0.782; Met, Kyn/Trp, MCSO2, 0.782; Cystathionine, hArg, MeCys, 0.782; Cit, MCSO2, MCSO1,0.782; Met, Kyn, MCSO2, 0.782; Orn, MCSO 2, SDMA, 0.782; Lys, Kyn, MCSO2/MeCys, 0.782; Glu, MCSO1,S pd, 0.782; Gln, hArg, MeCys, 0.782; Thr, Pro, MCSO2, 0.782; Ala, MeCys, Put, 0.782; Leu, hArg, MeCys, 0.782; Phe, MeCy s, Put, 0.782; 3-MeHis, Kyn/BCAA, MCSO2, 0.782; Asn, hArg, MeCys, 0.781; Gln, Met, MCSO2, 0.781; Ala, MCSO1/MCSO2, MCS O2/MeCys, 0.781; MCSO1,MCSO2/MeCys, MeCys, 0.781; Asn, MeC ys, Put, 0.781; Thr, MCSO2, MCSO1, 0.781; Tyr, MCSO2, MCSO1, 0.781; Met, Ile, MCSO2, 0.781; 3-MeHis, Kyn/Trp, MCSO2, 0. 781; MCSO2, MCSO1, N6-AcLys, 0.781; Glu, hArg, Spd, 0.781; Asn, Cit, MCSO2, 0.781; Gly, Met, MCSO2, 0.781; His, Ala, M CSO2, 0.781; His, MCSO2, SDMA, 0.781; Met, Phe, MCSO2, 0.78 1; Lys, hArg, MCSO2/MeCys, 0.781; Lys, MCSO2/MeCys, SDMA, 0. 781; Ile, MeCys, Put, 0.781; Glu, Asn, Spd, 0.78; Ser, Thr, MCSO2/MeCys, 0.78; Ala, MCSO2, N6-AcLys, 0.78; Pro, MCSO2, N6-AcLys, 0.78; aABA, MCSO2, MCSO1/MCSO2, 0.78; Tyr, MCSO2, MCSO1/MCSO2, 0.78; Val, hArg, MeCys, 0.78; Val, MeCys, Put, 0.78; ADMA, MeCys, Put, 0.78; aABA, hArg, MeCys, 0.78; Val, Met, MCSO2, 0.78; Lys, Kyn/Trp, MCSO2/MeCys, 0.78; Ile, Le u, MCSO2, 0.78; hArg, MCSO1, MCSO1/MCSO2, 0.78; Ala, Orn, M CSO2, 0.78; Leu, MCSO2, MCSO1, 0.78; Trp, MCSO2, N6-AcLys, 0.78; Hypotaurine, MeCys, Put, 0.78; MCSO1/MCSO2, MCSO2/MeC ys, MeCys, 0.78; Gly, Kyn/Trp, MCSO2, 0.78; His, Kyn/Trp, M CSO2, 0.78; Thr, hArg, MCSO2/MeCys, 0.78; Gly, Pro, MCSO2, 0.779; Gly, MCSO2, MCSO1, 0.779; Gly, MCSO2, N6-AcLys, 0.77 9; Arg, Met, MCSO2, 0.779; 3-MeHis, hArg, MeCys, 0.779; Ser, Arg, MCSO2, 0.779; Gly, MCSO2, MCSO1/MCSO2, 0.779; Arg, Pr o, MCSO2, 0.779; GABA, MeCys, Put, 0.779; Ala, Kyn/BCAA, MC SO2, 0.779; Cit, hArg, MCSO2/MeCys, 0.779; Gly, Trp, MCSO2, 0.779; Pro, MCSO2/MeCys, SDMA, 0.779; Met, Cystathionine, MCSO2, 0.779; Ser, Asn, MCSO2, 0.778; Ser, MeCys, Put, 0.77 8; Met, MCSO2, MeCys, 0.778; Ile, hArg, MeCys, 0.778; Trp, hArg, MeCys, 0.778; Ser, Pro, MCSO2, 0.778; His, Orn, MCSO2, 0.778; Thr, Orn, MCSO2, 0.778; Pro, Lys, MCSO2/MeCys, 0.77 8; Met, MCSO1/MCSO2, MCSO2/MeCys, 0.778; Leu, MCSO2, SDMA, 0.778; GABA, hArg, MeCys, 0.778; Asn, MCSO2, MCSO1/MCSO2, 0. 778; His, MCSO2, MCSO1, 0.778; Cit, 3-MeHis, MCSO2, 0.778; Cit, GABA, MCSO2, 0.778; Met, Hypotaurine, MCSO2, 0.778; Me t, MCSO2, MCSO2/MeCys, 0.778; 3-MeHis, MeCys, SDMA, 0.778; Gly, MCSO2, SDMA, 0.778; Arg, MCSO2, MCSO1, 0.778; aABA, MC SO2, N6-AcLys, 0.778; Tyr, Met, MCSO2, 0.778; Val, Leu, MCS O2, 0.778; Orn, MCSO2, MCSO1, 0.778; Ser, Tyr, MCSO2, 0.77 7; Pro, MCSO2/MeCys, MeCys, 0.777; ADMA, hArg, MeCys, 0.77 7; Asn, Met, MCSO2, 0.777; Ala, MCSO2, SDMA, 0.777; Met, AD MA, MCSO2, 0.777; Glu, Met, MCSO2, 0.777; Ala, Met, MCSO2, 0.777; Cit, Trp, MCSO2, 0.777; Cit, Kyn/Trp, MCSO2, 0.777; Arg, Kyn/BCAA, MCSO2, 0.777; Pro, Tyr, MCSO2, 0.777; Pro, M et, MCSO2, 0.777; aABA, Orn, MCSO2, 0.777; aABA, MCSO2, MCS O1, 0.777; Ser, Ala, MCSO2, 0.777; His, Put, SDMA, 0.777; C it, MeCys, N6-AcLys, 0.777; Pro, aABA, MCSO2, 0.777; Met, L eu, MCSO2, 0.777; Met, MCSO2/MeCys, MeCys, 0.777; Ser, MCSO 2, MCSO1, 0.776; Gln, Arg, MCSO2, 0.776; His, Kyn/BCAA, MCS O2, 0.776; Thr, Kyn/Trp, MCSO2, 0.776; Ala, Pro, MCSO2, 0.7 76; Cit, MCSO2, SDMA, 0.776; Arg, MCSO2, N6-AcLys, 0.776; T rp, MCSO2, MCSO1, 0.776; Trp, MCSO2, MCSO1/MCSO2, 0.776; GA BA, MCSO2, MCSO1, 0.776; Kyn/BCAA, MCSO2, MCSO1/MCSO2, 0.77 6; Glu, hArg, MCSO2/MeCys, 0.776; Ala, Arg, MCSO2, 0.776; A rg, MCSO2, SDMA, 0.776; Lys, GABA, MCSO2/MeCys, 0.776; Lys, MCSO1/MCSO2, MeCys, 0.776; MCSO2, MCSO1, SDMA, 0.776; Gln, Orn, MCSO2, 0.776; Thr, MCSO2/MeCys, MeCys, 0.776; Cit, Le u, MCSO2, 0.776; Pro, Kyn, MCSO2, 0.776; Val, Lys, MCSO2/Me Cys, 0.776; Orn, MCSO2, MCSO1/MCSO2, 0.776; Cystathionine, MCSO2, N6-AcLys, 0.776; hArg, MCSO2/MeCys, SDMA, 0.776; Ala, Leu, MCSO2, 0.776; Ala, Kyn/Trp, MCSO2, 0.776; Cit, MCSO2, MeCys, 0.776; Arg, MCSO2, MCSO1/MCSO2, 0.776; Tyr, Orn, MC SO2, 0.776; Orn, hArg, MCSO2/MeCys, 0.776; Glu, Lys, MCSO2/ MeCys, 0.775; Asn, MCSO2, MCSO1, 0.775; Gly, Orn, MCSO2, 0. 775; Thr, Lys, MCSO2/MeCys, 0.775; Thr, 3-MeHis, MCSO2, 0.7 75; Thr, Kyn/BCAA, MCSO2, 0.775; Ala, aABA, MCSO2, 0.775; C it, MCSO2, MCSO1/MCSO2, 0.775; 3-MeHis, MCSO2, MCSO1/MCSO2, 0.775; ADMA, MCSO2, MCSO1, 0.775; Glu, MCSO2, MCSO1, 0.77 5; Ser, aABA, MCSO2, 0.775; Gln, Thr, MCSO2, 0.775; Cit, AD MA, MCSO2, 0.775; Cit, Kyn/BCAA, MCSO2, 0.775; Pro, GABA, M CSO2, 0.775; Met, 3-MeHis, MCSO2, 0.775; Lys, 3-MeHis, MCSO 2/MeCys, 0.775; Ile, MCSO2, MCSO1/MCSO2, 0.775; Phe, MCSO2, MCSO1, 0.775; hArg, Hypotaurine, MCSO2/MeCys, 0.775; hArg, Put, SDMA, 0.775; MCSO2, MCSO1/MCSO2, MCSO2/MeCys, 0.775; N6-AcLys, Put, SDMA, 0.775; Asn, MCSO2, N6-AcLys, 0.775; As n, MCSO2, SDMA, 0.775; Thr, MCSO2, MCSO2/MeCys, 0.775; Ala, Kyn, MCSO2, 0.775; Pro, MCSO2, MCSO2/MeCys, 0.775; Met, Tr p, MCSO2, 0.775; Lys, Put, SDMA, 0.775; ADMA, MCSO2, SDMA, 0.775; Cystathionine, MCSO2, MCSO1, 0.775; Hypotaurine, MCS O1, Spd, 0.775; Kyn, MCSO2, MCSO1, 0.775; Kyn/BCAA, MCSO2, MCSO1, 0.775; MCSO2, MCSO1/MCSO2, SDMA, 0.775; Gly, Leu, MC SO2, 0.775; His, Met, MCSO2, 0.775; Thr, Tyr, MCSO2, 0.775; Thr, MCSO2, SDMA, 0.775; Ala, ADMA, MCSO2, 0.775; Ala, hAr g, MCSO2/MeCys, 0.775; Arg, Leu, MCSO2, 0.775; Arg, Trp, MC SO2, 0.775; Val, MCSO2, MCSO1/MCSO2, 0.775; Orn, Leu, MCSO2, 0.775; Gln, MCSO2, N6-AcLys, 0.774; Arg, MCSO2/MeCys, MeCy s, 0.774; Orn, Kyn, MCSO2, 0.774; Leu, Kyn, MCSO2, 0.774; G ABA, MCSO2, N6-AcLys, 0.774; Ser, Leu, MCSO2, 0.774; Gly, H is, MCSO2, 0.774; Gln, His, MCSO2, 0.774; Gln, Cit, MCSO2, 0.774; Gln, Pro, MCSO2, 0.774; His, Tyr, MCSO2, 0.774; Thr, Ala, MCSO2, 0.774; Thr, Met, MCSO2, 0.774; Thr, ADMA, MCSO 2, 0.774; Cit, aABA, MCSO2, 0.774; Cit, MCSO2, MCSO2/MeCys, 0.774; Arg, Kyn/Trp, MCSO2, 0.774; Orn, ADMA, MCSO2, 0.77 4; Ser, Lys, MeCys, 0.774; Gly, MCSO2, MCSO2/MeCys, 0.774; His, Thr, MCSO2, 0.774; His, MCSO2, N6-AcLys, 0.774; Thr, K yn, MCSO2, 0.774; Cit, Hypotaurine, MCSO2, 0.774; Cit, Kyn, MCSO2, 0.774; Pro, Orn, MCSO2, 0.774; Orn, MCSO2/MeCys, Me Cys, 0.774; Lys, MCSO1, MCSO1/MCSO2, 0.774; Cit, Tyr, MCSO2, 0.774; Pro, Trp, MCSO2, 0.774; Orn, Ile, MCSO2, 0.774; Lys, Phe, MCSO2/MeCys, 0.774; 3-MeHis, MCSO2, MCSO1, 0.774; Hyp otaurine, MCSO2, N6-AcLys, 0.774; Kyn/Trp, MCSO2, MCSO1, 0. 774; Glu, Cit, MCSO2, 0.773; Gly, Lys, MCSO2/MeCys, 0.773; Thr, Leu, MCSO2, 0.773; Ile, MCSO2, MCSO1, 0.773; Leu, Kyn/ Trp, MCSO2, 0.773; GABA, MCSO2, MCSO1/MCSO2, 0.773; MCSO2, MCSO1, MCSO2/MeCys, 0.773; Ser, MCSO2, MCSO1/MCSO2, 0.773; Gly, Thr, MCSO2, 0.773; Gln, MCSO2, MCSO1, 0.773; Thr, Hypo taurine, MCSO2, 0.773; Cit, Phe, MCSO2, 0.773; Arg, MCSO2, MCSO2/MeCys, 0.773; aABA, Leu, MCSO2, 0.773; Tyr, MCSO2, SD MA, 0.773; Orn, GABA, MCSO2, 0.773; Orn, MCSO2, MCSO2/MeCys, 0.773; Ser, His, MCSO2, 0.773; Ser, MCSO2, N6-AcLys, 0.77 3; Asn, Kyn/BCAA, MCSO2, 0.773; Gly, GABA, MCSO2, 0.773; Gl y, hArg, Put, 0.773; Ala, Cit, MCSO2, 0.773; Ala, Cystathio nine, MCSO2, 0.773; Arg, Tyr, MCSO2, 0.773; Arg, GABA, MCSO 2, 0.773; Pro, Kyn/Trp, MCSO2/MeCys, 0.773; Orn, MCSO2, N6-AcLys, 0.773; Lys, Cystathionine, MCSO2/MeCys, 0.773; Leu, MCSO2, N6-AcLys, 0.773; Asn, Pro, MCSO2, 0.773; Arg, Orn, M CSO2, 0.773; Pro, Leu, MCSO2, 0.773; Val, MCSO2, N6-AcLys, 0.773; Orn, Cystathionine, MCSO2, 0.773; 3-MeHis, MCSO2, N6 -AcLys, 0.773; ADMA, MCSO2, N6-AcLys, 0.773; MCSO2, MCSO1, MeCys, 0.773; Glu, MCSO2, N6-AcLys, 0.773; Asn, Ala, MCSO2, 0.773; His, Trp, MCSO2, 0.773; Thr, Trp, MCSO2, 0.773; Cit, Val, MCSO2, 0.773; Tyr, 3-MeHis, MCSO2, 0.773; Orn, Trp, M CSO2, 0.773; Orn, 3-MeHis, MCSO2, 0.773; ADMA, MCSO2, MCSO1 /MCSO2, 0.773; Gln, Lys, MCSO2/MeCys, 0.772; Thr, GABA, MCS O2, 0.772; Thr, Put, SDMA, 0.772; Ala, MCSO2, MCSO2/MeCys, 0.772; Pro, Ile, MCSO2, 0.772; Pro, ADMA, MCSO2, 0.772; Lys, Ile, MCSO2/MeCys, 0.772; 3-MeHis, Kyn, MCSO2, 0.772; Glu, Orn, MCSO2, 0.772; Ser, Gly, MCSO2, 0.772; Gly, Kyn/BCAA, M CSO2, 0.772; His, MCSO2/MeCys, MeCys, 0.772; Ala, GABA, MCS O2, 0.772; Ala, MCSO2/MeCys, MeCys, 0.772; Lys, Kyn/BCAA, M CSO2/MeCys, 0.772; Ile, MCSO2, N6-AcLys, 0.772; Leu, MCSO1/ MCSO2, MCSO2/MeCys, 0.772; hArg, Kyn/Trp, MCSO2/MeCys, 0.77 2; Gly, Gln, MCSO2, 0.772; His, Arg, MCSO2, 0.772; His, ADM A, MCSO2, 0.772; Thr, Cystathionine, MCSO2, 0.772; Pro, MCS O2, MeCys, 0.772; Tyr, ADMA, MCSO2, 0.772; Orn, Phe, MCSO2, 0.772; GABA, MCSO2, SDMA, 0.772; Gly, Ala, MCSO2, 0.772; G ly, Arg, MCSO2, 0.772; His, Kyn, MCSO2, 0.772; Ala, Tyr, MC SO2, 0.772; Cit, Orn, MCSO2/MeCys, 0.772; Cit, Ile, MCSO2, 0.772; Arg, Ile, MCSO2, 0.772; Pro, 3-MeHis, MCSO2, 0.772; Pro, Cystathionine, MCSO2, 0.772; aABA, 3-MeHis, MCSO2, 0.7 72; Tyr, Leu, MCSO2, 0.772; Tyr, Kyn/BCAA, MCSO2, 0.772; Va l, Orn, MCSO2, 0.772; Trp, Kyn/BCAA, MCSO2, 0.772; Hypotaur ine, MCSO2, MCSO1, 0.772; MCSO2, MeCys, N6-AcLys, 0.772; MC SO2/MeCys, MeCys, N6-AcLys, 0.772; Asn, Arg, MCSO2, 0.771; Thr, Ile, MCSO2, 0.771; Arg, Lys, MCSO2/MeCys, 0.771; Pro, MCSO1/MCSO2, MCSO2/MeCys, 0.771; aABA, Kyn/BCAA, MCSO2, 0.7 71; Lys, MCSO1, MCSO2/MeCys, 0.771; ADMA, Kyn/BCAA, MCSO2, 0.771; His, 3-MeHis, MCSO2, 0.771; Thr, Val, MCSO2, 0.771; Ala, Phe, MCSO2, 0.771; Cit, Pro, MCSO2/MeCys, 0.771; Cit, Cystathionine, MCSO2, 0.771; Orn, Lys, MCSO2/MeCys, 0.771; Orn, MCSO2, MeCys, 0.771; Orn, MCSO1/MCSO2, MCSO2/MeCys, 0. 771; Trp, MCSO2, SDMA, 0.771; ADMA, Kyn/Trp, MCSO2, 0.771; Kyn, Kyn/BCAA, MCSO2, 0.771; Glu, Gly, MCSO2, 0.771; Ser, C it, MCSO2, 0.771; Ser, Lys, MCSO2/MeCys, 0.771; Asn, His, M CSO2, 0.771; Asn, Thr, MCSO2, 0.771; Thr, Arg, MCSO2, 0.77 1; Ala, Lys, MCSO2/MeCys, 0.771; Ala, Trp, MCSO2, 0.771; Al a, Hypotaurine, MCSO2, 0.771; Arg, Phe, MCSO2, 0.771; Pro, Val, MCSO2, 0.771; aABA, Met, MCSO2, 0.771; Tyr, MCSO2, MCS O2/MeCys, 0.771; Orn, Hypotaurine, MCSO2, 0.771; Cystathion ine, MCSO2, MCSO1/MCSO2, 0.771; hArg, Kyn/Trp, Put, 0.771; Glu, Hypotaurine, Spd, 0.771; Glu, MCSO2, MCSO1/MCSO2, 0.77 1; Ser, Orn, MCSO2, 0.771; Asn, Orn, MCSO2, 0.771; Gly, Val, MCSO2, 0.771; Gly, 3-MeHis, MCSO2, 0.771; Ala, Ile, MCSO2, 0.771; Kyn, MCSO2, SDMA, 0.771; Kyn/Trp, MCSO2/MeCys, N6-A cLys, 0.771; MCSO2, MCSO2/MeCys, N6-AcLys, 0.771; Gly, aABA, MCSO2, 0.77; Gln, Tyr, MCSO2, 0.77; His, Phe, MCSO2, 0.77; Ala, Val, MCSO2, 0.77; Val, MCSO2, MCSO1, 0.77; Lys, MeCys, SDMA, 0.77; Ile, Kyn/BCAA, MCSO2, 0.77; Ile, MCSO2, SDMA, 0.77; Phe, MCSO2, MCSO1/MCSO2, 0.77; Gly, Ile, MCSO2, 0.77; Gly, Cystathionine, MCSO2, 0.77; His, aABA, MCSO2, 0.77; H is, GABA, MCSO2, 0.77; Thr, Pro, MCSO2/MeCys, 0.77; Thr, Ph e, MCSO2, 0.77; Thr, Kyn/Trp, MCSO2/MeCys, 0.77; Thr, MCSO2, MeCys, 0.77; Ala, MCSO2, MeCys, 0.77; Pro, Hypotaurine, MC SO2, 0.77; Leu, Kyn/BCAA, MCSO2, 0.77; 3-MeHis, GABA, MCSO2, 0.77; MCSO2, MCSO1/MCSO2, MeCys, 0.77; Glu, Thr, MCSO2, 0. 77; His, Leu, MCSO2, 0.77; Thr, Cit, Put, 0.77; Ala, 3-MeHi s, MCSO2, 0.77; Arg, Cystathionine, MCSO2, 0.77; Arg, Kyn, MCSO2, 0.77; Tyr, GABA, MCSO2, 0.77; Lys, Hypotaurine, MCSO 2/MeCys, 0.77; Leu, Hypotaurine, MCSO2, 0.77; Trp, ADMA, MC SO2, 0.77; Trp, GABA, MCSO2, 0.77; GABA, hArg, MCSO2/MeCys, 0.77; GABA, Kyn/Trp, MCSO2, 0.77; Ser, Kyn/BCAA, MCSO2, 0. 77; Asn, Kyn/Trp, MCSO2, 0.77; His, MCSO2, MCSO2/MeCys, 0.7 7; Arg, 3-MeHis, MCSO2, 0.77; Val, MCSO2, SDMA, 0.77; Met, hArg, MCSO2/MeCys, 0.77; Leu, MCSO2/MeCys, MeCys, 0.77; hAr g, Kyn/BCAA, MCSO2/MeCys, 0.77; Glu, Arg, MCSO2, 0.769; Glu, Leu, MCSO2, 0.769; Asn, Gln, MCSO2, 0.769; Gly, Phe, MCSO2, 0.769; His, Lys, MCSO2/MeCys, 0.769; Arg, Val, MCSO2, 0.76 9; aABA, Tyr, MCSO2, 0.769; aABA, ADMA, MCSO2, 0.769; Tyr, Lys, MCSO2/MeCys, 0.769; Orn, Kyn/Trp, MCSO2/MeCys, 0.769; Lys, Leu, MCSO2/MeCys, 0.769; Phe, MCSO2, N6-AcLys, 0.769; Kyn/BCAA, MCSO2, MCSO2/MeCys, 0.769; Ser, GABA, MCSO2, 0.76 9; Gly, ADMA, MCSO2, 0.769; His, Hypotaurine, MCSO2, 0.769; Cit, MCSO2/MeCys, N6-AcLys, 0.769; Pro, Phe, MCSO2, 0.769; Tyr, Kyn, MCSO2, 0.769; Tyr, Kyn/Trp, MCSO2, 0.769; Val, K yn/BCAA, MCSO2, 0.769; Trp, 3-MeHis, MCSO2, 0.769; Kyn/Trp, MCSO2, MCSO1/MCSO2, 0.769; Kyn/Trp, MCSO2, MCSO2/MeCys, 0. 769; MCSO2/MeCys, MeCys, SDMA, 0.769; Glu, His, MCSO2, 0.76 9; Asn, Tyr, MCSO2, 0.769; Asn, Lys, MCSO2/MeCys, 0.769; Gl n, MCSO2, MCSO1/MCSO2, 0.769; Thr, aABA, MCSO2, 0.769; aABA, Lys, MCSO2/MeCys, 0.769; Lys, MCSO2/MeCys, N6-AcLys, 0.76 9; Phe, Kyn/BCAA, MCSO2, 0.769; Phe, MCSO2, SDMA, 0.769; Hy potaurine, MCSO2, MCSO1/MCSO2, 0.769; Kyn, Kyn/Trp, MCSO2, 0.769; Kyn, MeCys, N6-AcLys, 0.769; Glu, Ala, MCSO2, 0.769; Ser, Kyn/Trp, MCSO2, 0.769; Cit, Arg, MCSO2/MeCys, 0.769; Arg, ADMA, MCSO2, 0.769; aABA, MCSO2, SDMA, 0.769; Lys, Trp, MCSO2/MeCys, 0.769; Ile, Kyn/Trp, MCSO2, 0.769; Glu, Pro, MCSO2, 0.768; Gly, Hypotaurine, MCSO2, 0.768; Gly, Kyn, MCS O2, 0.768; His, Ile, MCSO2, 0.768; Cit, Lys, MeCys, 0.768; Arg, hArg, MCSO2/MeCys, 0.768; aABA, Ile, MCSO2, 0.768; Tyr, Phe, MCSO2, 0.768; Tyr, Trp, MCSO2, 0.768; Trp, Kyn/Trp, M CSO2, 0.768; 3-MeHis, ADMA, MCSO2, 0.768; GABA, Kyn/BCAA, M CSO2, 0.768; Glu, Tyr, MCSO2, 0.768; Ser, MCSO2, SDMA, 0.76 8; His, Val, MCSO2, 0.768; His, Cystathionine, MCSO2, 0.76 8; Cit, Arg, MeCys, 0.768; aABA, Trp, MCSO2, 0.768; Leu, AD MA, MCSO2, 0.768; Leu, Cystathionine, MCSO2, 0.768; Trp, Ky n, MCSO2, 0.768; Trp, MCSO2/MeCys, MeCys, 0.768; Tyr, Val, MCSO2, 0.768; Tyr, Ile, MCSO2, 0.768; Phe, hArg, MCSO2/MeCy s, 0.768; MCSO1/MCSO2, MeCys, N6-AcLys, 0.768; Asn, Leu, MC SO2, 0.768; Gln, Ala, MCSO2, 0.768; His, hArg, MCSO2/MeCys, 0.768; Cit, Arg, Put, 0.768; Cit, MCSO2/MeCys, MeCys, 0.76 8; Tyr, MCSO2/MeCys, MeCys, 0.768; Met, Lys, MCSO2/MeCys, 0. 768; Hypotaurine, Kyn/BCAA, MCSO2, 0.768; Kyn/BCAA, MCSO1/M CSO2, MCSO2/MeCys, 0.768; Kyn/BCAA, MeCys, N6-AcLys, 0.768; MCSO2, MCSO2/MeCys, SDMA, 0.768; Gln, Trp, MCSO2, 0.767; G ln, Kyn/BCAA, MCSO2, 0.767; His, MCSO2, MeCys, 0.767; His, MeCys, SDMA, 0.767; Arg, Hypotaurine, MCSO2, 0.767; Tyr, Cy stathionine, MCSO2, 0.767; Val, MCSO1/MCSO2, MCSO2/MeCys, 0. 767; Trp, MCSO2, MCSO2/MeCys, 0.767; 3-MeHis, Cystathionine, MCSO2, 0.767; MCSO1/MCSO2, MCSO2/MeCys, N6-AcLys, 0.767; A rg, Put, SDMA, 0.767; Leu, hArg, MCSO2/MeCys, 0.767; GABA, Hypotaurine, MCSO2, 0.767; hArg, Kyn, MCSO2/MeCys, 0.767; H ypotaurine, MCSO2, SDMA, 0.767; Kyn, MCSO2, MCSO1/MCSO2, 0. 767; MCSO1, MCSO1/MCSO2, MCSO2/MeCys, 0.767; Glu, Kyn/BCAA, MCSO2, 0.767; Asn, Trp, MCSO2, 0.767; Arg, MCSO2, MeCys, 0. 767; Val, hArg, MCSO2/MeCys, 0.767; Lys, Kyn, MeCys, 0.767; ADMA, GABA, MCSO2, 0.767; Kyn/Trp, MCSO2, SDMA, 0.767; Kyn /BCAA, MCSO2/MeCys, MeCys, 0.767; Asn, GABA, MCSO2, 0.767; Thr, Kyn/BCAA, MCSO2/MeCys, 0.767; aABA, GABA, MCSO2, 0.76 7; aABA, Kyn/Trp, MCSO2, 0.767; Val, ADMA, MCSO2, 0.767; Ky n/BCAA, MCSO2, SDMA, 0.767; MCSO2, MCSO1, MCSO1/MCSO2, 0.76 7; Glu, ADMA, MCSO2, 0.766; Thr, MCSO2/MeCys, SDMA, 0.766; Pro, MeCys, SDMA, 0.766; Val, MCSO2/MeCys, MeCys, 0.766; Le u, MCSO2, MeCys, 0.766; Phe, Kyn/Trp, MCSO2, 0.766; Trp, Cy stathionine, MCSO2, 0.766; Trp, Hypotaurine, MCSO2, 0.766; 3-MeHis, MCSO2, MeCys, 0.766; GABA, MCSO1/MCSO2, MCSO2/MeCy s, 0.766; Kyn/Trp, Kyn/BCAA, MCSO2, 0.766; Asn, aABA, MCSO2, 0.766; Gly, Tyr, MCSO2, 0.766; Gln, aABA, MCSO2, 0.766; Gl n, hArg, MCSO2/MeCys, 0.766; Gln, MCSO2, SDMA, 0.766; His, Lys, MeCys, 0.766; Thr, GABA, MCSO2/MeCys, 0.766; aABA, Phe, MCSO2, 0.766; aABA, MCSO2, MCSO2/MeCys, 0.766; Tyr, Hypota urine, MCSO2, 0.766; Met, MCSO1/MCSO2, MeCys, 0.766; Lys, A DMA, MCSO2/MeCys, 0.766; Lys, Kyn/Trp, MeCys, 0.766; Ile, M CSO2/MeCys, MeCys, 0.766; Leu, Phe, MCSO2, 0.766; Leu, GABA, MCSO2, 0.766; Trp, hArg, MCSO2/MeCys, 0.766; 3-MeHis, Hypo taurine, MCSO2, 0.766; 3-MeHis, MCSO2, MCSO2/MeCys, 0.766; ADMA, hArg, MCSO2/MeCys, 0.766; Cystathionine, hArg, MCSO2/ MeCys, 0.766; hArg, Hypotaurine, Put, 0.766; hArg, MCSO1, P ut, 0.766; Kyn/Trp, MCSO2/MeCys, MeCys, 0.766; Glu, Trp, MC SO2, 0.766; Glu, Kyn/Trp, MCSO2, 0.766; Ser, Ile, MCSO2, 0. 766; Ser, Trp, MCSO2, 0.766; Ser, ADMA, MCSO2, 0.766; Gln, Lys, MeCys, 0.766; Gln, Leu, MCSO2, 0.766; Gln, Kyn/Trp, MC SO2, 0.766; aABA, Cystathionine, MCSO2, 0.766; Val, Kyn/Trp, MCSO2, 0.766; Ile, Kyn, MCSO2, 0.766; Leu, Trp, MCSO2, 0.7 66; 3-MeHis, MCSO2/MeCys, MeCys, 0.766; ADMA, MCSO2/MeCys, MeCys, 0.766; GABA, MCSO2, MCSO2/MeCys, 0.766; Ser, 3-MeHis, MCSO2, 0.766; Cystathionine, MCSO2, SDMA, 0.766; hArg, MCS O1, MCSO2/MeCys, 0.766; Kyn/BCAA, MCSO1/MCSO2, Put, 0.766; Ser, Gln, MCSO2, 0.765; Thr, MCSO1/MCSO2, MeCys, 0.765; aAB A, Kyn, MCSO2, 0.765; Met, Put, SDMA, 0.765; Leu, MCSO2, MC SO2/MeCys, 0.765; Phe, GABA, MCSO2, 0.765; ADMA, MCSO2, MCS O2/MeCys, 0.765; Cystathionine, Kyn/BCAA, MCSO2, 0.765; GAB A, Kyn, MCSO2, 0.765; Hypotaurine, Kyn/Trp, MCSO2, 0.765; M CSO2, MeCys, SDMA, 0.765; Asn, MCSO2, MCSO2/MeCys, 0.765; A rg, aABA, MCSO2, 0.765; Pro, Put, SDMA, 0.765; Tyr, MCSO2, MeCys, 0.765; Val, Kyn, MCSO2, 0.765; Met, MCSO2/MeCys, SDM A, 0.765; ADMA, Cystathionine, MCSO2, 0.765; ADMA, Hypotaur ine, MCSO2, 0.765; GABA, MCSO2/MeCys, MeCys, 0.765; Ser, hA rg, MCSO2/MeCys, 0.765; Gly, MCSO2, MeCys, 0.765; Gln, 3-Me His, MCSO2, 0.765; Gln, ADMA, MCSO2, 0.765; Val, Trp, MCSO2, 0.765; Val, 3-MeHis, MCSO2, 0.765; Val, GABA, MCSO2, 0.76 5; Cystathionine, GABA, MCSO2, 0.765; Glu, GABA, MCSO2, 0.7 65; Glu, MCSO2, SDMA, 0.765; Ser, MCSO2, MeCys, 0.765; Asn, 3-MeHis, MCSO2, 0.765; Asn, ADMA, MCSO2, 0.765; Leu, 3-MeH is, MCSO2, 0.765; Gln, GABA, MCSO2, 0.764; aABA, MCSO2, MeC ys, 0.764; Tyr, MCSO1/MCSO2, MCSO2/MeCys, 0.764; Lys, MeCys, N6-AcLys, 0.764; Ile, Trp, MCSO2, 0.764; Ile, 3-MeHis, MCS O2, 0.764; Phe, Trp, MCSO2, 0.764; Kyn/Trp, MCSO2, MeCys, 0. 764; Ala, Put, SDMA, 0.764; Cit, hArg, Put, 0.764; aABA, Hy potaurine, MCSO2, 0.764; Ile, MCSO1/MCSO2, MCSO2/MeCys, 0.7 64; ADMA, Kyn, MCSO2, 0.764; Kyn/BCAA, MCSO2, MeCys, 0.764; Ser, Val, MCSO2, 0.764; Asn, hArg, MCSO2/MeCys, 0.764; His, MCSO1, MCSO1/MCSO2, 0.764; His, MCSO1/MCSO2, MeCys, 0.764; Arg, MCSO1/MCSO2, MCSO2/MeCys, 0.764; Phe, 3-MeHis, MCSO2, 0.764; Phe, ADMA, MCSO2, 0.764; Trp, MCSO2, MeCys, 0.764; 3-MeHis, hArg, MCSO2/MeCys, 0.764; ADMA, Put, SDMA, 0.764; Cystathionine, Kyn/Trp, MCSO2, 0.764; Glu, aABA, MCSO2, 0.7 64; Ser, Phe, MCSO2, 0.764; Met, Kyn/Trp, MCSO2/MeCys, 0.76 4; Ile, Cystathionine, MCSO2, 0.764; Ile, hArg, MCSO2/MeCys, 0.764; Ile, Hypotaurine, MCSO2, 0.764; Phe, Cystathionine, MCSO2, 0.764; Phe, MCSO2, MCSO2/MeCys, 0.764; Trp, MCSO1/M CSO2, MCSO2/MeCys, 0.764; Asn, MCSO2/MeCys, MeCys, 0.763; H is, Cit, MCSO2/MeCys, 0.763; Thr, Orn, MCSO2/MeCys, 0.763; Phe, Hypotaurine, MCSO2, 0.763; ADMA, MCSO2, MeCys, 0.763; GABA, MCSO2, MeCys, 0.763; Ser, Kyn, MCSO2, 0.763; Asn, Hyp otaurine, MCSO2, 0.763; Gly, MCSO1/MCSO2, MCSO2/MeCys, 0.76 3; Gly, MCSO2/MeCys, MeCys, 0.763; His, hArg, Put, 0.763; A la, MCSO1/MCSO2, MeCys, 0.763; Val, Hypotaurine, MCSO2, 0.7 63; Met, Kyn, MCSO2/MeCys, 0.763; Lys, MCSO1, MeCys, 0.763; Ile, Phe, MCSO2, 0.763; Ile, ADMA, MCSO2, 0.763; hArg, MCS O2/MeCys, N6-AcLys, 0.763; MCSO1/MCSO2, Put, SDMA, 0.763; G lu, Ser, MCSO2, 0.763; Gln, Phe, MCSO2, 0.763; Pro, MCSO1/M CSO2, MeCys, 0.763; aABA, Val, MCSO2, 0.763; aABA, MCSO2/Me Cys, MeCys, 0.763; Ile, MCSO2, MeCys, 0.763; Phe, MCSO2, Me Cys, 0.763; Hypotaurine, MCSO2, MCSO2/MeCys, 0.763; Ser, Hy potaurine, MCSO2, 0.763; aABA, hArg, MCSO2/MeCys, 0.763; Or n, Kyn/BCAA, MCSO2/MeCys, 0.763; Cystathionine, MCSO2, MCSO 2/MeCys, 0.763; Cystathionine, MCSO2, MeCys, 0.763; Kyn, MC SO2, MCSO2/MeCys, 0.763; Glu, Phe, MCSO2, 0.762; Ser, MCSO2, MCSO2/MeCys, 0.762; Ser, MCSO2/MeCys, MeCys, 0.762; Asn, M CSO1/MCSO2, MCSO2/MeCys, 0.762; Thr, Lys, MeCys, 0.762; Val, Ile, MCSO2, 0.762; Kyn/Trp, MCSO1/MCSO2, MCSO2/MeCys, 0.76 2; Ser, Cystathionine, MCSO2, 0.762; Gln, Hypotaurine, MCSO 2, 0.762; Cit, Met, MCSO2/MeCys, 0.762; Cit, Met, MeCys, 0. 762; aABA, Lys, MeCys, 0.762; Asn, Gly, MCSO2, 0.762; Asn, MCSO2, MeCys, 0.762; Gln, Kyn, MCSO2, 0.762; Gln, MCSO2, Me Cys, 0.762; Thr, Phe, MCSO2/MeCys, 0.762; Met, GABA, MCSO2/ MeCys, 0.762; Phe, Kyn, MCSO2, 0.762; Cystathionine, Hypota urine, MCSO2, 0.762; Cystathionine, Kyn, MCSO2, 0.762; hArg, Kyn, Put, 0.762; MCSO1, MeCys, N6-AcLys, 0.762; Glu, 3-MeH is, MCSO2, 0.762; Gly, Thr, MCSO2/MeCys, 0.762; His, Pro, M CSO2/MeCys, 0.762; Thr, MCSO2/MeCys, N6-AcLys, 0.762; Val, Cystathionine, MCSO2, 0.762; Lys, Cystathionine, MeCys, 0.7 62; Ile, MCSO2, MCSO2/MeCys, 0.762; Kyn, MCSO2/MeCys, MeCys, 0.762; Kyn/Trp, MCSO1/MCSO2, Put, 0.762; Glu, MCSO2, MCSO2 /MeCys, 0.761; Ser, Thr, MeCys, 0.761; Asn, Cystathionine, MCSO2, 0.761; Gly, Lys, MeCys, 0.761; Ala, Lys, MeCys, 0.76 1; Ile, GABA, MCSO2, 0.761; Glu, Kyn, MCSO2, 0.761; Asn, Il e, MCSO2, 0.761; Asn, Kyn, MCSO2, 0.761; Thr, Kyn, MCSO2/Me Cys, 0.761; Cit, Orn, MeCys, 0.761; Leu, MCSO1/MCSO2, MeCys, 0.761; Gln, Ile, MCSO2, 0.761; Gln, MCSO2, MCSO2/MeCys, 0. 761; Gln, MCSO2/MeCys, MeCys, 0.761; Val, Lys, MeCys, 0.76 1; Lys, Phe, MeCys, 0.761; Kyn, MCSO2, MeCys, 0.761; Glu, G ln, MCSO2, 0.761; Thr, Cit, MeCys, 0.761; Cit, MCSO1/MCSO2, MCSO2/MeCys, 0.761; Pro, Kyn, MCSO2/MeCys, 0.761; Pro, Kyn /BCAA, MCSO2/MeCys, 0.761; Val, Phe, MCSO2, 0.761; Val, MCS O2, MeCys, 0.761; Met, MeCys, N6-AcLys, 0.761; Phe, MCSO2/M eCys, MeCys, 0.761; Glu, Hypotaurine, MCSO2, 0.761; Gln, Va l, MCSO2, 0.761; Gln, Cystathionine, MCSO2, 0.761; Tyr, hAr g, MCSO2/MeCys, 0.761; hArg, MCSO1/MCSO2, Put, 0.761; MCSO1 /MCSO2, MCSO2/MeCys, SDMA, 0.761; Glu, Cystathionine, MCSO2, 0.76; Val, MCSO2, MCSO2/MeCys, 0.76; Met, MeCys, SDMA, 0.7 6; Orn, Put, SDMA, 0.76; Glu, Asn, MCSO2, 0.76; Glu, MCSO2, MeCys, 0.76; Asn, Lys, MeCys, 0.76; His, MCSO1/MCSO2, MCSO 2/MeCys, 0.76; Met, Lys, MeCys, 0.76; Met, Kyn/BCAA, MCSO2/ MeCys, 0.76; Orn, Lys, MeCys, 0.76; Lys, GABA, MeCys, 0.76; Glu, MCSO2/MeCys, MeCys, 0.76; Thr, Cystathionine, MCSO2/M eCys, 0.76; Lys, ADMA, MeCys, 0.76; Thr, MCSO1, MCSO2/MeCys, 0.76; Lys, Hypotaurine, MeCys, 0.76; Lys, Kyn/BCAA, MeCys, 0.76; Leu, Put, SDMA, 0.76; Cystathionine, MCSO2/MeCys, Me Cys, 0.76; Hypotaurine, MCSO1/MCSO2, MCSO2/MeCys, 0.76; Glu, Val, MCSO2, 0.759; Glu, Ile, MCSO2, 0.759; Ser, MCSO1/MCSO 2, MeCys, 0.759; Cit, MCSO1/MCSO2, MeCys, 0.759; Val, MCSO1 /MCSO2, MeCys, 0.759; Hypotaurine, Kyn, MCSO2, 0.759; Asn, Phe, MCSO2, 0.759; Thr, Leu, MCSO2/MeCys, 0.759; Pro, Lys, MeCys, 0.759; Pro, GABA, MCSO2/MeCys, 0.759; Orn, MCSO2/MeC ys, SDMA, 0.759; Lys, 3-MeHis, MeCys, 0.759; Arg, Lys, MeCy s, 0.759; Arg, MeCys, SDMA, 0.759; Hypotaurine, MCSO2/MeCys, MeCys, 0.759; Asn, Val, MCSO2, 0.759; Gly, Put, SDMA, 0.75 9; Gln, MCSO1/MCSO2, MCSO2/MeCys, 0.759; His, Thr, MCSO2/Me Cys, 0.759; His, Cit, MeCys, 0.759; Met, Cystathionine, MCS O2/MeCys, 0.759; Lys, Leu, MeCys, 0.759; Glu, Lys, MeCys, 0. 758; Ser, Met, MeCys, 0.758; Asn, MCSO1/MCSO2, MeCys, 0.75 8; Met, Phe, MCSO2/MeCys, 0.758; Lys, Ile, MeCys, 0.758; Hy potaurine, MCSO2, MeCys, 0.758; Ala, Kyn/Trp, MCSO2/MeCys, 0.758; aABA, MCSO1/MCSO2, MCSO2/MeCys, 0.758; Lys, Trp, MeC ys, 0.758; Ile, MCSO1/MCSO2, MeCys, 0.758; Hypotaurine, MCS O1/MCSO2, MeCys, 0.758; Thr, Trp, MCSO2/MeCys, 0.758; Pro, Cystathionine, MCSO2/MeCys, 0.758; Gly, MCSO1/MCSO2, MeCys, 0.758; Arg, Pro, MCSO2/MeCys, 0.758; aABA, MeCys, N6-AcLys, 0.758; Cystathionine, hArg, Put, 0.758; Asn, Put, SDMA, 0. 757; Cit, Lys, Put, 0.757; Glu, Thr, MCSO2/MeCys, 0.757; Se r, MCSO1/MCSO2, MCSO2/MeCys, 0.757; His, MeCys, N6-AcLys, 0. 757; Pro, Met, MCSO2/MeCys, 0.757; aABA, MCSO1/MCSO2, Put, 0.757; Met, MCSO1, MeCys, 0.757; Ser, His, MeCys, 0.757; Th r, Ala, MCSO2/MeCys, 0.757; Tyr, Lys, MeCys, 0.757; Orn, MC SO1/MCSO2, MeCys, 0.757; Cystathionine, MCSO1/MCSO2, MCSO2/ MeCys, 0.757; MCSO1/MCSO2, MeCys, SDMA, 0.757; His, Ala, Me Cys, 0.757; Arg, MCSO1/MCSO2, MeCys, 0.757; Pro, Kyn/Trp, M eCys, 0.757; Tyr, MCSO1/MCSO2, MeCys, 0.757; Phe, MCSO1/MCS O2, MCSO2/MeCys, 0.757; ADMA, MCSO1/MCSO2, MCSO2/MeCys, 0.7 57; Kyn, MCSO1/MCSO2, MCSO2/MeCys, 0.757; Glu, MCSO1/MCSO2, MCSO2/MeCys, 0.756; Gln, His, MeCys, 0.756; Cit, Met, Put, 0.756; 3-MeHis, MCSO1/MCSO2, MeCys, 0.756; 3-MeHis, Put, S DMA, 0.756; Asn, Thr, MCSO2/MeCys, 0.756; Gly, Pro, MCSO2/M eCys, 0.756; Gln, Thr, MCSO2/MeCys, 0.756; Thr, Hypotaurine, MCSO2/MeCys, 0.756; Lys, Kyn, Put, 0.756; Phe, MCSO1/MCSO2, MeCys, 0.756; ADMA, MCSO1/MCSO2, MeCys, 0.756; hArg, Kyn/B CAA, Put, 0.756; His, GABA, MCSO2/MeCys, 0.756; Thr, aABA, MCSO2/MeCys, 0.756; Met, Orn, MCSO2/MeCys, 0.756; Orn, hArg, Put, 0.756; Trp, MCSO1/MCSO2, MeCys, 0.756; Thr, Tyr, MCSO 2/MeCys, 0.756; Thr, ADMA, MCSO2/MeCys, 0.756; Tyr, MeCys, N6-AcLys, 0.756; Cystathionine, MCSO1/MCSO2, MeCys, 0.756; Cystathionine, MeCys, N6-AcLys, 0.756; GABA, MCSO1/MCSO2, M eCys, 0.756; Gln, MCSO1/MCSO2, MeCys, 0.755; Thr, Ile, MCSO 2/MeCys, 0.755; Ala, Orn, MCSO2/MeCys, 0.755; Arg, Kyn/Trp, MCSO2/MeCys, 0.755; Pro, Orn, MCSO2/MeCys, 0.755; Gly, MCS O1, MeCys, 0.755; His, MCSO1/MCSO2, Put, 0.755; Thr, Kyn, P ut, 0.755; ADMA, Kyn/Trp, MCSO2/MeCys, 0.755; Asn, Cit, MCS O2/MeCys, 0.755; His, Kyn/Trp, MeCys, 0.755; GABA, Kyn/Trp, MCSO2/MeCys, 0.755; Kyn/BCAA, MCSO1/MCSO2, MeCys, 0.755; A DMA, Kyn/BCAA, MCSO2/MeCys, 0.755; Glu, MCSO1/MCSO2, MeCys, 0.754; Asn, hArg, Put, 0.754; Thr, Met, MCSO2/MeCys, 0.75 4; Thr, 3-MeHis, MCSO2/MeCys, 0.754; Thr, MeCys, N6-AcLys, 0.754; Ala, MCSO1/MCSO2, Put, 0.754; Arg, Kyn/BCAA, MCSO2/M eCys, 0.754; Lys, Kyn/Trp, Put, 0.754; Asn, MCSO2/MeCys, SD MA, 0.754; His, MCSO2/MeCys, SDMA, 0.754; Cit, MCSO1, MeCys, 0.754; aABA, hArg, Put, 0.754; Leu, MeCys, N6-AcLys, 0.75 4; Leu, MeCys, SDMA, 0.754; 3-MeHis, hArg, Put, 0.754; ADMA, Kyn/Trp, Put, 0.754; Asn, Pro, MCSO2/MeCys, 0.754; Gln, Ar g, MeCys, 0.754; Trp, MeCys, N6-AcLys, 0.754; 3-MeHis, MCSO 1/MCSO2, MCSO2/MeCys, 0.754; His, Orn, MCSO2/MeCys, 0.754; Ala, MeCys, N6-AcLys, 0.754; aABA, Put, SDMA, 0.754; Lys, h Arg, Put, 0.754; Kyn/Trp, N6-AcLys, Put, 0.754; His, MCSO1, MeCys, 0.753; Thr, Kyn/Trp, Put, 0.753; Arg, GABA, MCSO2/M eCys, 0.753; Orn, GABA, MCSO2/MeCys, 0.753; MCSO1, MCSO1/MC SO2, MeCys, 0.753; Thr, MCSO1, MCSO1/MCSO2, 0.753; Thr, MCS O1/MCSO2, Put, 0.753; Ala, hArg, Put, 0.753; Pro, hArg, Put, 0.753; Leu, MCSO2/MeCys, SDMA, 0.753; ADMA, MCSO2/MeCys, S DMA, 0.753; His, Pro, MeCys, 0.753; His, Kyn/Trp, MCSO2/MeC ys, 0.753; Cit, MCSO1/MCSO2, Put, 0.753; Arg, MCSO2/MeCys, SDMA, 0.753; Pro, aABA, MCSO2/MeCys, 0.753; His, Kyn/Trp, P ut, 0.753; His, Kyn/BCAA, MCSO2/MeCys, 0.753; Thr, Arg, MCS O2/MeCys, 0.753; Thr, Val, MCSO2/MeCys, 0.753; Ala, Kyn/BCA A, MCSO2/MeCys, 0.753; Ala, MCSO1, MeCys, 0.753; Pro, Hypot aurine, MCSO2/MeCys, 0.753; Arg, hArg, Put, 0.752; Pro, Leu, MCSO2/MeCys, 0.752; GABA, hArg, Put, 0.752; MCSO1, Put, SD MA, 0.752; Ser, MeCys, N6-AcLys, 0.752; His, aABA, MeCys, 0. 752; His, Kyn, MCSO2/MeCys, 0.752; Ala, MCSO2/MeCys, SDMA, 0.752; Lys, 3-MeHis, Put, 0.752; Phe, hArg, Put, 0.752; 3-M eHis, MeCys, N6-AcLys, 0.752; Gln, Met, MeCys, 0.752; Orn, MCSO1, MCSO1/MCSO2, 0.752; ADMA, MeCys, SDMA, 0.752; Hypota urine, Put, SDMA, 0.752; Asn, Kyn/Trp, MCSO2/MeCys, 0.752; His, Ala, MCSO2/MeCys, 0.752; Thr, MCSO1, MeCys, 0.752; Arg, Kyn/Trp, Put, 0.752; Arg, MeCys, N6-AcLys, 0.752; GABA, MC SO1, MCSO1/MCSO2, 0.752; Kyn, MCSO1/MCSO2, Put, 0.752; Pro, Trp, MCSO2/MeCys, 0.751; Orn, Trp, MCSO2/MeCys, 0.751; Gly, Kyn/BCAA, MCSO2/MeCys, 0.751; Gly, MeCys, SDMA, 0.751; His, Met, MeCys, 0.751; Thr, MeCys, SDMA, 0.751; Cit, Orn, Put, 0.751; Orn, Kyn, MCSO2/MeCys, 0.751; Glu, Pro, MCSO2/MeCys, 0.751; Ser, Arg, MeCys, 0.751; Gln, hArg, Put, 0.751; His, Kyn, MeCys, 0.751; Cit, ADMA, MeCys, 0.751; Pro, MCSO2/MeC ys, N6-AcLys, 0.751; aABA, MCSO1/MCSO2, MeCys, 0.751; Met, Kyn/BCAA, MeCys, 0.751; Met, MCSO1, MCSO2/MeCys, 0.751; Orn, MCSO1, MeCys, 0.751; Gly, Kyn/Trp, MCSO2/MeCys, 0.751; His, Kyn/BCAA, MeCys, 0.751; Ala, MeCys, SDMA, 0.751; Pro, MCSO 1, MCSO2/MeCys, 0.751; Met, Kyn, MeCys, 0.751; Orn, Kyn/Trp, MeCys, 0.751; Cit, Pro, MeCys, 0.75; Tyr, MeCys, SDMA, 0.7 5; Met, hArg, Put, 0.75; Met, Kyn/Trp, Put, 0.75; Leu, MCSO 1, MeCys, 0.75; His, Arg, MeCys, 0.75; Ala, Kyn, MCSO2/MeCy s, 0.75; Met, Orn, MeCys, 0.75; Leu, Kyn, MCSO2/MeCys, 0.7 5; Gln, Thr, MeCys, 0.75; Pro, Phe, MCSO2/MeCys, 0.75; Ile, Leu, MeCys, 0.75; Leu, Kyn/Trp, MCSO2/MeCys, 0.75; Kyn/BCA A, MCSO2/MeCys, N6-AcLys, 0.75; Glu, MeCys, N6-AcLys, 0.75; His, Kyn, Put, 0.75; Pro, ADMA, MCSO2/MeCys, 0.75; Val, hA rg, Put, 0.75; Leu, hArg, Put, 0.75; hArg, N6-AcLys, Put, 0. 75; Pro, Val, MCSO2/MeCys, 0.75; Tyr, hArg, Put, 0.75; Trp, MeCys, SDMA, 0.75; 3-MeHis, MCSO2/MeCys, SDMA, 0.75

## Claims

1. An evaluating method comprising:
an evaluating step of evaluating a state of mild cognitive impairment for a subject to be evaluated, using (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of the subject, or (ii) a value of a formula including an explanatory variable to be substituted with the at least one value, calculated using the at least one value and the formula.

2. The evaluating method according to claim 1, wherein the evaluating step is executed by a control unit of an information processing apparatus.

3. A calculating method comprising:
a calculating step of using (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of a subject to be evaluated, and (ii) a formula for evaluating a state of mild cognitive impairment, including an explanatory variable to be substituted with the at least one value, to calculate a value of the formula.

4. The calculating method according to claim 3, wherein the calculating step is executed by a control unit of an information processing apparatus.

5. An evaluating apparatus comprising a control unit, wherein the control unit includes:
an evaluating unit that evaluates a state of mild cognitive impairment for a subject to be evaluated, using (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of the subject, or (ii) a value of a formula including an explanatory variable to be substituted with the at least one value, calculated using the at least one value and the formula.

6. The evaluating apparatus according to claim 5, being communicatively connected via a network to a terminal apparatus that provides blood data on the at least one value, or the value of the formula,
wherein the control unit further includes:
a data-receiving unit that receives the blood data of the subject or the value of the formula transmitted from the terminal apparatus; and
a result-sending unit that transmits an evaluation result obtained by the evaluating unit to the terminal apparatus,
wherein the evaluating unit uses the at least one value included in the blood data or the value of the formula received by the data-receiving unit.

7. A calculating apparatus comprising a control unit, wherein the control unit includes:
a calculating unit that uses (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of a subject to be evaluated, and (ii) a formula for evaluating a state of mild cognitive impairment, including an explanatory variable to be substituted with the at least one value, to calculate a value of the formula.

8. An evaluating program for causing a control unit of an information processing apparatus to execute:
an evaluating step of evaluating a state of mild cognitive impairment for a subject to be evaluated, using (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of the subject, or (ii) a value of a formula including an explanatory variable to be substituted with the at least one value, calculated using the at least one value and the formula.

9. A calculating program for causing a control unit of an information processing apparatus to execute:
a calculating step of using (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of a subject to be evaluated, and (ii) a formula for evaluating a state of mild cognitive impairment, including an explanatory variable to be substituted with the at least one value, to calculate a value of the formula.

10. A computer readable recording medium storing the evaluating program according to claim 8 or the calculating program according to claim 9.

11. An evaluating system comprising:
an evaluating apparatus including a control unit; and
a terminal apparatus including a control unit to provide (i) blood data on at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of a subject to be evaluated, or (ii) a value of a formula including an explanatory variable to be substituted with the at least one value, calculated using the at least one value and the formula,
wherein the evaluating apparatus and the terminal apparatus are connected to each other communicatively via a network,
the control unit of the terminal apparatus includes:
a data-sending unit that transmits the blood data of the subject, or the value of the formula, to the evaluating apparatus; and
a result-receiving unit that receives an evaluation result on a state of mild cognitive impairment for the subject, transmitted from the evaluating apparatus, and
the control unit of the evaluating apparatus includes:
a data-receiving unit that receives the blood data of the subject or the value of the formula transmitted from the terminal apparatus;
an evaluating unit that evaluates a state of mild cognitive impairment for the subject, using the at least one value included in the blood data of the subject or the value of the formula received by the data-receiving unit; and
a result-sending unit that transmits the evaluation result obtained by the evaluating unit to the terminal apparatus.

12. A terminal apparatus comprising a control unit,
wherein the control unit includes a result-obtaining unit that obtains an evaluation result on a state of mild cognitive impairment for a subject to be evaluated,
wherein the evaluation result is a result of evaluating a state of mild cognitive impairment for the subject, using (i) at least one value of concentration values of Put, α-ABA, Ala, Arg, Asn, Cit, Glu, Gln, Gly, His, Ile, Leu, Lys, Met, Orn, Phe, Pro, Ser, Thr, Trp, Tyr, Val, Taurine, 1-MeHis, 3-MeHis, aAAA, aAiBA, ADMA, Allyl Cys, bABA, bAiBA, Cystathionine, Cysteic acid, EtGly, GABA, hArg, Hypotaurine, Hypro, Kyn, Pyrazole-1-Ala, MCSO, MeCys, N6-AcLys, N8-AcSpd, Opthalmic acid, PEA, Pipecolic acid, Sar, SDMA, Spd, Spm, and Thioproline and test values of Alb, Folate, WBC, RBC, Hb, Ht, and PLT in blood of the subject, or (ii) a value of a formula including an explanatory variable to be substituted with the at least one value, calculated using the at least one value and the formula.

13. The terminal apparatus according to claim 12, being communicatively connected via a network to an evaluating apparatus that evaluates a state of mild cognitive impairment for the subject,
wherein the control unit includes a data-sending unit that transmits blood data on the at least one value in blood of the subject, or the value of the formula, to the evaluating apparatus,
the result-obtaining unit receives the evaluation result transmitted from the evaluating apparatus.
